(19) **Europäisches Patentamt European Patent Office Office européen des brevets**

(11) **EP 4 610 652 A1**

(12) **EUROPEAN PATENT APPLICATION**
published in accordance with Art. 153(4) EPC

(43) Date of publication:
**03.09.2025 Bulletin 2025/36**

(21) Application number: **23883103.6**

(22) Date of filing: **25.10.2023**

(51) International Patent Classification (IPC):
**G01N 33/487** (2006.01)     **C07K 14/00** (2006.01)

(52) Cooperative Patent Classification (CPC):
**C07K 14/00; G01N 33/487**

(86) International application number:
**PCT/KR2023/016681**

(87) International publication number:
**WO 2024/090998 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.10.2022 KR 20220138724**

(71) Applicant: **Korea Research Institute of Bioscience and Biotechnology Daejeon 34141 (KR)**

(72) Inventors:
- **CHI, Seung-Wook Daejeon 34141 (KR)**
- **JEONG, Ki Baek Daejeon 34141 (KR)**
- **KIM, Jin Sik Daejeon 34141 (KR)**

- **LEE, Mi-Kyung Daejeon 34141 (KR)**
- **RYU, Minju Daejeon 34141 (KR)**
- **OH, So Hee Daejeon 34141 (KR)**
- **CHUNG, Minji Daejeon 34141 (KR)**
- **JO, Junhyeok Daejeon 34141 (KR)**

(74) Representative: **Ipsilon Lyon Le Contemporain 50 Chemin de la Bruyère 69574 Dardilly Cedex (FR)**

Remarks:
The complete document including Reference Table(s) and the Sequence Listing(s) can be downloaded from the EPO website

(54) **YAXAB NANOPORE, NANOPORE SYSTEM COMPRISING SAME AND APPLICATIONS THEREOF**

(57)     The present invention relates to a YaxAB nanopore, a system comprising the nanopore and applications thereof. The YaxAB nanopore of the present invention has a funnel-shaped three-dimensional structure so that a change in current blockade according to movement of an analyte captured inside the nanopore can be maximized to enable very sensitive measurements of even the structure of the analyte and minute physical and chemical characteristics changes including dynamic change and the like, and can cause very strong electroosmosis with high cation selectivity such that various types of analytes can be captured and analyzed without limiting the types of overall charges.

Additionally, with respect to the nanopore of the present invention, the amino acid sequence of a mono-

mer constituting the nanopore can be adjusted to improve protein purification yield and control oligomerization mechanism, and thus a YaxAB nanopore having a pore size that cannot be formed by a wild-type monomer can be prepared. Furthermore, with respect to the nanopore of the present invention, YaxAB nanopores of various pore sizes can be ensured using one-time purification, and a nanopore of a desired pore size can be selectively ensured. Therefore, limitations of a conventional biological nanopore with a fixed pore size can be overcome such that, with respect to the YaxAB nanopore of the present invention, the size of a nanopore can be adjusted according to the size of an analyte. Additionally, the YaxAB nanopore of the present invention has analyte analysis spectrums of a very wide dynamic range of

**(Cont. next page)**

EP 4 610 652 A1

detection from a small analyte up to a large analyte, resulting from the funnel-shaped three-dimensional structure with a wide inlet and a narrow outlet. Moreover, the YaxAB nanopore of the present invention has the largest nanopore structure among biological nanopores up to now, and large-sized analytes that cannot be detected by conventional biological nanopores can undergo single molecule detection and analysis on the basis of a funnel structure with a large inlet.

In addition, the present invention has established an optimized composition of a membrane in which the YaxAB nanopore can exist stably, and, by using a sensor implemented on the basis thereof, various types of analytes having relatively various sizes and charges can be qualitatively and quantitatively analyzed at a single molecular level with very high resolution and sensitivity.

[FIG. 3]

## Description

## TECHNICAL FIELD

[CROSS-REFERENCE TO RELATED APPLICATIONS]

This application claims priority from Korean Patent Application No. 10-2022-0138724, filed on October 25, 2022, the disclosure of which is incorporated herein by reference in its entirety.

[TECHNICAL FIELD]

The present invention relates to a YaxAB nanopore and a nanopore system containing the same, and relates to a novel nanopore sensing platform capable of precisely analyzing analytes having various sizes and charges at a single molecule level, screening a drug binding thereto, analyzing an interaction with a ligand, or detecting and quantitatively analyzing a disease biomarker.

## BACKGROUND ART

In drug discovery, protein-protein interactions (PPIs) are targets for effective disease treatment; however, the methods for screening PPI inhibitors through high-throughput screening has not yet been commercialized. Studies on a structure-activity relationship (SAR) and mode of action (MOA) are essential to monitor the protein-drug interactions (PDIs) in vitro. Although there are various methods for direct drug screening such as nuclear magnetic resonance (NMR), surface plasma resonance (SPR), isothermal titration calorimetry (ITC), and fluorescence resonance energy transfer (FRET); however, there are urgent needs for low-cost and efficient drug screening for the protein-protein interactions due to expensive equipment, inaccuracy caused by labeling/-fixation, low sensitivity in detecting small molecule drugs, and limited solubility of target proteins and/or drugs.

Nanopore is a novel high-precision biosensor capable of detecting subtle morphological changes in biomolecules at a single molecule level. Nanopores generally serve as only conduits for allowing ionic currents to flow between two fluid reservoirs in which individual nanopore is embedded in a thin insulating membrane. Nanopore experiments that utilize the principle of Coulter counter are related to changes in ionic currents, When the ionic currents are electrophoretically induced through the nanopore in the presence of an external electric field, the translocation of analytes through the nanopore induces a temporary blockage of the ionic currents. This is measured in a current amplitude or the like, and the length, size, charge, structure, shape, and the like of biomolecules are determined based on these electrical signals. The nanopore biosensor has been used for genome sequencing, detection of various individual biomolecules, and detection of biomolecular interactions.

Cytolysin A (ClyA) is a pore-forming protein known to form dodecameric to tetradecameric nanopores. A ClyA nanopore has a constriction having a diameter of 3.3 nm or more, and has been studied to monitor various biomolecules, protein-ligand interactions, and the like. Biomolecular analysis technologies using nanopores have the advantages of single molecule level resolution, high sensitivity, label-free, and real-time measurement, and thus, biomolecular analysis technologies using nanopores have been actively developed. However, these nanopores are based on recognizing large structural changes induced by ligand binding, and have limitations for targets where drugs cannot be used in a conventional manner, such as undruggable targets. In addition, since proteins basically have various surface charges, the principle of electrophoretic force (EPF), which is basically used as a driving force for attracting analytes in nanopores, has limitations in capturing protein analytes.

As a result of intensive efforts to develop methods that may accurately and efficiently screen protein-protein interaction inhibitors even with an extremely small amount of sample, the present inventors have found that the novel biological nanopore that utilizes electroosmotic force (EOF) that occurs depending on pore internal surface charges, and electrical signals are measured by using the novel nanopore sensing platform including the same, such that single molecule proteins or nucleic acids may be detected according to changes in electrical signals, protein-drug interactions, a protein-protein interaction, and even a small molecule drug that inhibit such protein-protein interactions may be effectively screened, and furthermore, screening based on "drug fingerprinting" at a single molecule level, which may sensitively distinguish subtle differences between different drugs bound to the same protein, may be performed, thereby completing the present invention.

## DISCLOSURE OF THE INVENTION

## TECHNICAL PROBLEM

An aspect of the present invention is to provide a novel biological nanopore subunit.

Another aspect of the present invention is to provide a nanopore containing the nanopore subunit.

Still another aspect of the present invention is to provide a nanopore-containing membrane including a membrane layer; and a nanopore containing at least one nanopore subunit and inserted into the membrane layer.

Still another aspect of the present invention is to provide a nanopore system including a chamber; and the nanopore-containing membrane, wherein a space in the chamber is divided into two compartments by the nanopore-containing membrane.

Still another aspect of the present invention is to provide a single molecule analysis method for single analytes or a plurality of analytes using the nanopore system.

Still another aspect of the present invention is to provide a method for analyzing interactions between at least one analyte and at least one ligand or screening ligands for an analyte using the nanopore system.

Still another aspect of the present invention is to provide a method for analyzing or screening interaction inhibitors or promoters between interactable biomolecules using the nanopore system.

Still another aspect of the present invention is to provide a method for identifying and quantitatively analyzing analytes in a sample using the nanopore system.

Still another aspect of the present invention is to provide a method for providing information for diagnosing biomarker-associated diseases using the nanopore system.

## TECHNICAL SOLUTION

In order to achieve the objects of the present invention described above, the present invention provides a YaxAB nanopore including a first opening, a middle area, and a second opening, the YaxAB nanopore having a funnel shape, wherein an outer diameter of a lumen of the first opening is 5 nm or more, the second opening includes a constriction, a diameter of the constriction is 0.5 nm or more, an outer diameter of a lumen of the second opening is 1 nm or more, and a depth of the lumen is 5 nm or more.

In addition, the present invention provides a YaxAB nanopore containing at least one subunit containing a first monomer having an amino acid sequence having 70% or more homology with an amino acid sequence of any one of SEQ ID NO: 7, SEQ ID NO: 24, or SEQ ID NO: 34; and a second monomer having an amino acid sequence having 70% or more homology with an amino acid sequence of SEQ ID NO: 18 or SEQ ID NO: 25.

In addition, the present invention provides a nanopore-containing membrane including a membrane layer; and a YaxAB nanopore inserted into the membrane layer, wherein the membrane layer includes at least one selected from the group consisting of 1,2-diphytanoyl-sn-glycero-3-phosphocholine (DPhPC), 1,2-dipalmitoyl-sn-glycero-3-phosphocholine (DPPC), 1,2-dipalmitoyl-sn-glycero-3-phosphorylethanolamine (DPPE), 1,2-dipalmitoyl-sn-glycero-3-phosphorylglycerol (DPPG), 1,2-dipalmitoyl-sn-glycero-3-phospho-L-serine (DPPS), 1,2-dihexanoyl-sn-glycero-3-phosphocholine (DHPC), 1,2-dilauroyl-sn-glycero-3-phosphocholine (DLPC), 1,2-dimyristoyl-sn-glycero-3-phosphocholine (DMPC), 1,2-dimyristoyl-sn-glycero-3-phosphorylethanolamine (DMPE), 1,2-dimyristoyl-sn-glycero-3-phosphorylglycerol (DMPG), 1,2-dimyristoyl-sn-glycero-3-phospho-L-serine (DMPS), 1,2-dioleoyl-sn-glycero-3-phospho-L-serine (DOPS), 1,2-dioleoyl-sn-glycero-3-phosphoethanolamine (DOPE), 1,2-dioleoyl-sn-glycero-3-phosphoglycerol (DOPG), 1,2-distearoyl-sn-glycero-3-phosphocholine (DSPC), 1-palmitoyl-2-oleoyl-SN-glycero-3-phosphocholine (POPC), and mycolic acid, and at least one selected from the group consisting of campesterol, sitosterol, stigmasterol, cholesterol, ergosterol, cardiolipin, sphingomyelin, 1-palmitoyl-2-cholesterylhemisuccinoyl-sn-glycero-3-phosphocholine (PChemsPC), 1-oleoyl-2-cholesterylhemisuccinoyl-sn-glycero-3-phosphocholine (OChemsPC), 1-palmitoyl-2-cholesterylcarbonoyl-sn-glycero-3-phosphocholine (PChcPC), 1,2-dicholesterylhemisuccinoyl-sn-glycero-3-phosphocholine (DChemsPC), 10,12-pentacosadiynoic acid (PCDA), 10,12-tricosadiynoic acid (TCDA), 5,7-hexadecadiynoic acid (HDDA), and 9,12-octadecadiynoic acid (ODDA).

In addition, the present invention provides a nanopore system including a chamber; and a YaxAB nanopore-containing membrane, wherein a space in the chamber is divided into two compartments by the nanopore-containing membrane.

In addition, the present invention provides a single molecule analysis method for single analytes or a plurality of analytes, the single molecule analysis method including placing at least one analyte in one compartment of the nanopore system; and measuring changes in electrical signals in the two compartments before and after the placing of the analytes.

In addition, the present invention provides a method for analyzing interactions between an analyte and ligands or screening a ligand for an analyte, the method including: performing a treatment with at least one analyte and at least one ligand candidate substance for the analyte in one compartment or two compartments of the nanopore system; and measuring changes in electrical signals in the two compartments before and after the treatment with the candidate substance.

In addition, the present invention provides a method for analyzing or screening interaction inhibitors or promoters between biomolecules, the method including: reacting a plurality of biomolecules capable of interacting in one compartment of the nanopore system; performing a treatment with candidate substances of the interaction inhibitors or promoters in the one compartment or two compartments; and measuring changes in electrical signals in the two compartments before and after the treatment with the candidate substances.

In addition, the present invention provides a method for identifying and quantitatively analyzing analytes in a sample, the method including: placing a sample in one compartment of the nanopore system; and comparing changes in electrical signals caused by a plurality of analytes in the sample with a database of electrical signals for each substance.

In addition, the present invention provides a method for providing information for diagnosing a biomarker-associated disease, the method including: placing a sample in one compartment of the nanopore system; and measuring changes in characteristic electrical signals caused by a biomarker for a specific disease among changes in electrical signals induced by the sample.

## ADVANTAGEOUS EFFECTS

The YaxAB nanopore of the present invention may induce electroosmosis by cations that predominantly flow along a pore inner wall since the amino acids constituting the inner wall of the pore are negatively charged under a condition of pH 7.5; thus, analytes may be captured inside the nanopore regardless of the overall charge. In addition, the structure including the diameter of the YaxAB nanopore may be adjusted depending on the number of heterodimers constituted by two monomers YaxA and YaxB, and YaxAB nanopores with various sizes may be ensured by only one-time purification process, and therefore, there is an advantage in that analysis targets are not limited by the charge or size of the analytes. Moreover, since the nanopore has a funnel structure in which an inlet is wide but an outlet is relatively much narrower, even large analytes may be captured inside the nanopore one by one, and also, due to a high ion density in a narrow area, an analyte-derived current blockade changes significantly compared to conventional cylindrical nanopores, such that even subtle physicochemical property changes in the analyte may be detected much more sensitively, and also, analytes with various sizes may also be analyzed.

Based on these unique physical and chemical characteristics, in a case where the YaxAB nanopore of the present invention is used, a much wider range of analytes may be detected with high resolution than conventional nanopores, furthermore, protein-small molecule compound complexes may be directly detected, and further, protein-small molecule compound complexes in which different small molecule compounds are bound may be sensitively distinguished at a single molecule level. Therefore, compared to the conventional technology, the YaxAB nanopore may be efficiently utilized in various ways in terms of time and cost for interactions between an analyte and a ligand, analysis of structure and dynamics of biomolecules, new drug screening, disease diagnosis, protein identification, protein posttranslational modification (PTM) analysis, genome and proteome analysis, and sequencing of proteins and nucleic acids.

## BRIEF DESCRIPTION OF THE DRAWINGS

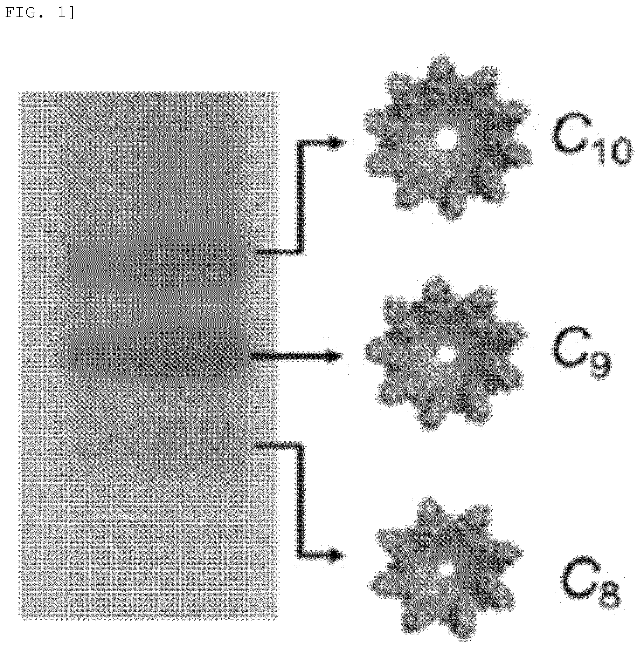

FIG. 1 illustrates bands of different oligomers obtained by electrophoresis of modified YaxAB_dN nanopores with a gradient blue native polyacrylamide gel. The modified YaxAB_dN nanopore is one of the modified YaxAB nanopores having amino acid sequences modified from a wild-type YaxAB, and refers to a YaxAB oligomer formed of first monomers consisting of SEQ ID NO: 1 (YaxA_(45-410)) and second monomers consisting of SEQ ID NO: 2 (YaxB_(12-343)). The presence of modified YaxAB_dN-$C_8$, -$C_9$, and -$C_{10}$ was confirmed through the three divided bands.

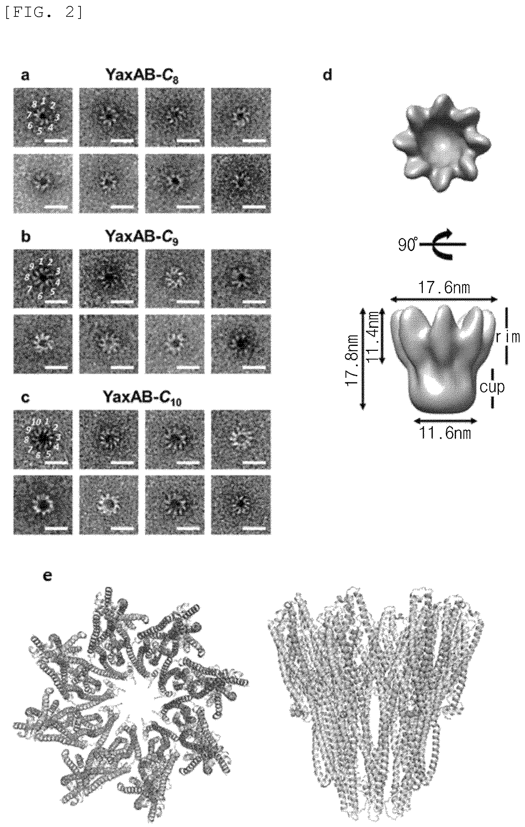

FIGS. 2a to 2c illustrate the results of observing the modified YaxAB_dN-$C_8$, -$C_9$, and -$C_{10}$ nanopores using Negative-stain EM (negative staining electron microscopy), FIG. 2d illustrates a three-dimensional reconstruction based on the results of the Negative-stain EM, and FIG. 2e illustrates a structure of the modified YaxAB_dN-$C_8$ nanopore based on the results of Cryo-EM (cryogenic electron microscopy).

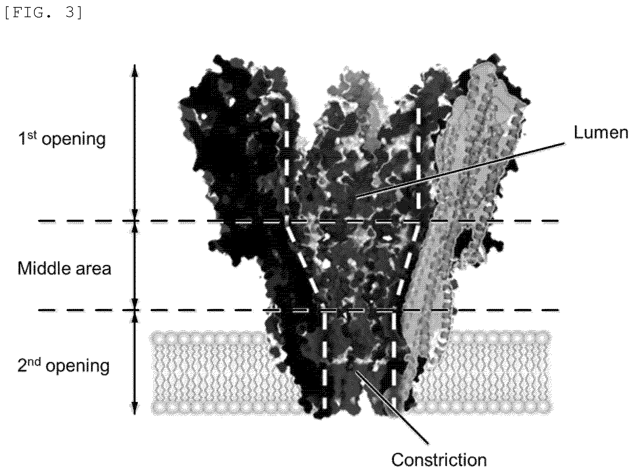

FIG. 3 is a cross-sectional view of the modified YaxAB_dN nanopore illustrating the structure of the nanopore.

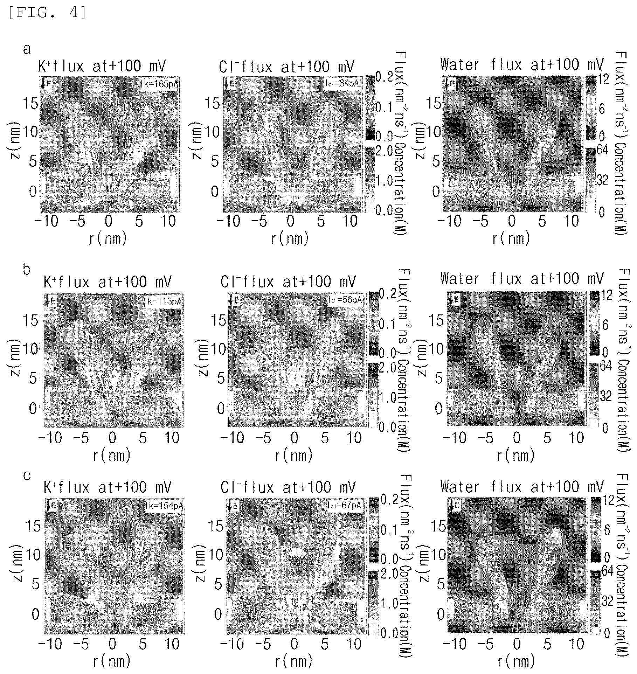

FIG. 4a illustrates the result of molecular dynamics (MD) simulation showing a flow of each of potassium ions, chloride ions, and water molecules in the modified YaxAB_dN nanopore when 100 mV is applied, and FIGS. 4b and 4c illustrate the results of molecular dynamics simulation when Bcl-xL protein is located at distances of 4 nm and 10 nm from a membrane, respectively.

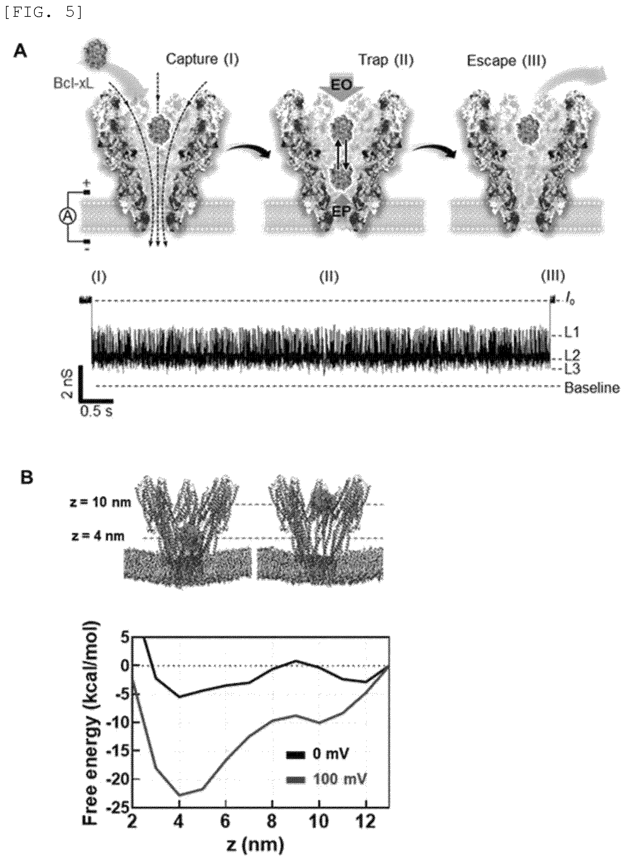

FIG. 5a is a schematic diagram of an analyte trapping event and illustrates a single molecule analyte signal when Bcl-xL protein is trapped in the modified YaxAB_dN nanopore, and FIG. 5b is a schematic diagram of MD simulation results when an analyte is present at distances of 4 nm and 10 nm from the membrane, and illustrates a free energy of the analyte according to a distance (z) from the membrane calculated by the MD simulation.

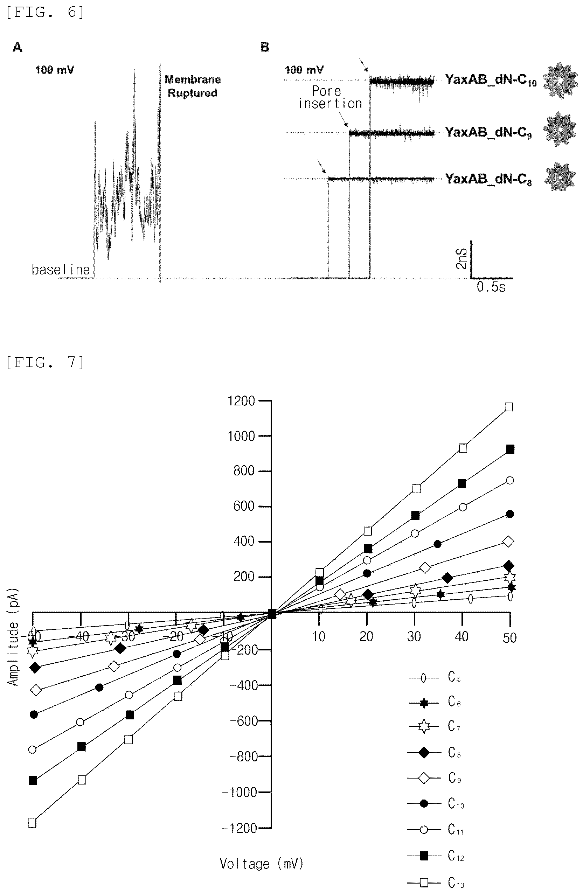

FIG. 6 illustrates the results showing that the modified YaxAB_dN nanopores achieve better stabilization of DPhPC-sterol composite membranes than membranes formed with only DPhPC. FIG. 6a illustrates the change in conductivity when the modified YaxAB_dN nanopore is inserted into a membrane formed of only DPhPC and the conductivity after insertion, showing that membrane rupturing occurs. FIG. 6b illustrates stable operations of the nanopores of the modified YaxAB_dN-$C_8$, -$C_9$, and -$C_{10}$ when inserted into the DPhPC-sterol composite membrane, where C represents a heterodimer subunit composed of a first monomer and a second monomer, and $C_8$ represents a YaxAB nanopore composed of eight pairs of heterodimer subunits (hereinafter, referred to as YaxAB-$C_8$).

FIG. 7 illustrates that, when a voltage is applied under conditions of the modified YaxAB_dN nanopores with various sizes, constant currents flow compared to the applied voltage.

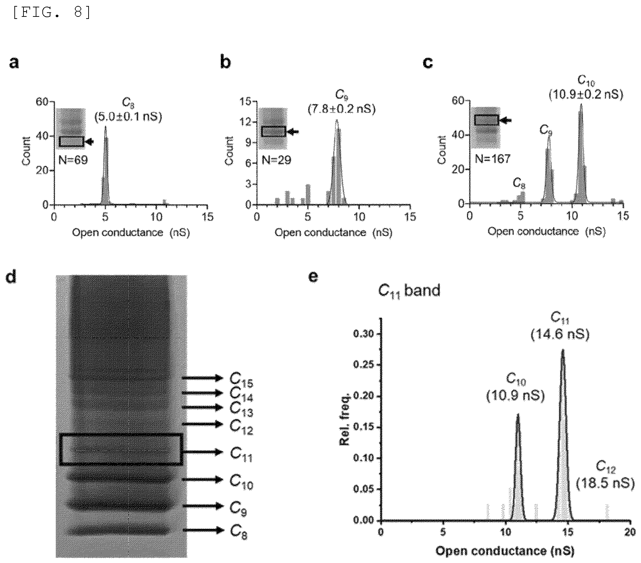

FIG. 8 illustrates formation tendencies of modified YaxAB_dN nanopores ($C_8$, $C_9$, $C_{10}$, $C_{11}$, $C_{12}$, $C_{13}$, $C_{14}$, and $C_{15}$) through the results of purified and electrophoresed protein bands and conductivity measurement of each nanopore.

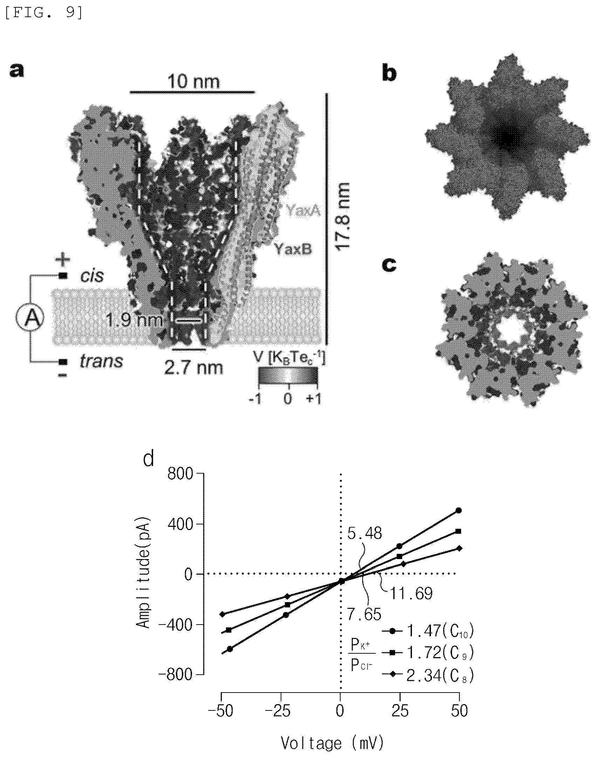

FIG. 9a illustrates a schematic diagram of electrical measurement of the modified YaxAB_dN-$C_8$ nanopore inserted into the DPhPC-sterol composite membrane, FIG. 9b illustrates a lumen structure connected by eight alpha helices, FIG. 9c is a cross section of the modified YaxAB_dN-$C_8$ nanopore and illustrates a surface charge that becomes more negative as it approaches a trans opening, and FIG. 9d illustrates a reversal potential and cation selectivity ($P_K^+/P_{Cl}^-$) of each of the modified YaxAB_dN nanopores ($C_8$, $C_9$, and $C_{10}$).

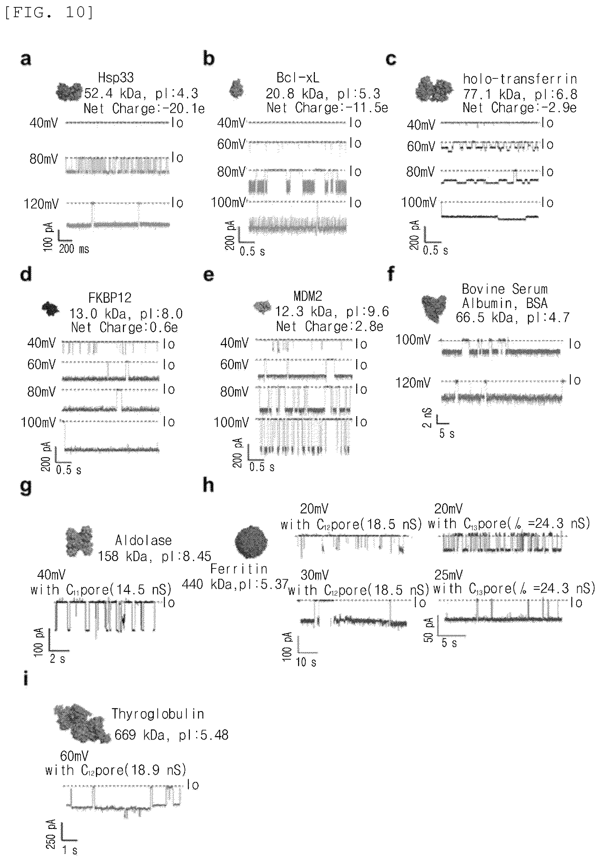

FIG. 10 illustrates the results of single molecule analysis of nine types of proteins such as unlabeled proteins Hsp33, Bcl-xL, Holo-transferrin, FKBP12, MDM2, BSA, Aldolase, and Ferritin, and Thyroglobulin using the modified YaxAB_dN nanopore.

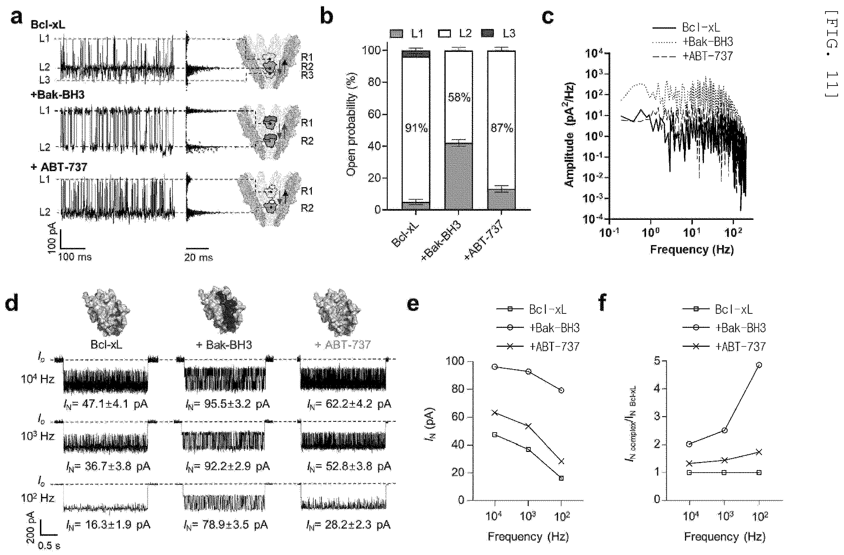

FIG. 11 illustrates current blockade and current noise analysis of a protein-ligand interaction using modified YaxAB_dN nanopores, FIG. 11a illustrates a schematic diagram of Bcl-xL, a Bcl-xL+Bak-BH3 complex, and a Bcl-xL+ABT-737 complex in the pores and the results of measuring current blockade signals, FIG. 11b illustrates a dwell time ratio of each levels of current blockade (L1-L3), FIG. 11c illustrates a power spectral density of the Bcl-xL, the Bcl-xL+Bak-BH3 complex, and the Bcl-xL+ABT-737 complex, and FIGS. 11d to 11f illustrate the results of the current noise ($I_N$)-based analysis thereof.

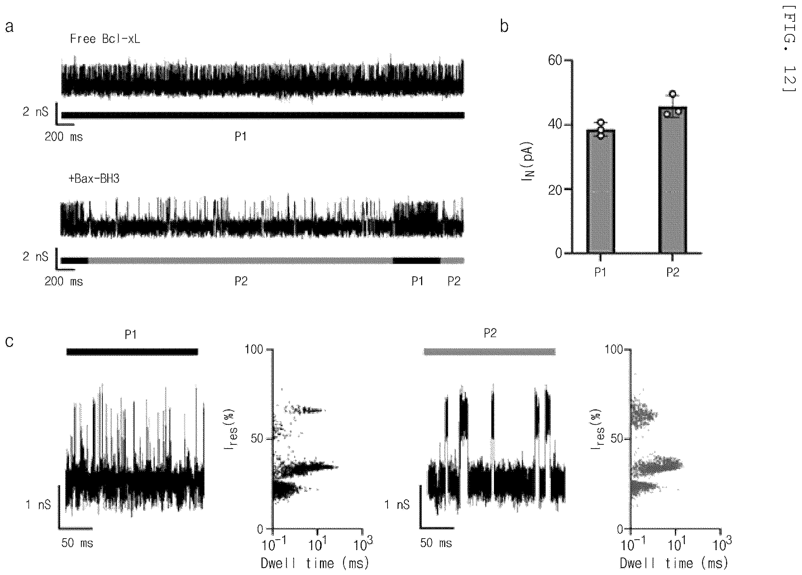

FIG. 12 illustrates the results of analyzing the binding of Bax-BH3 peptide ($K_D$ = 13 $\mu$M) having a low binding affinity for Bcl-xL protein using the modified YaxAB_dN nanopore, FIG. 12a illustrates changes in ionic current signal that occurs depending on whether Bax-BH3 peptide binds to Bcl-xL protein, FIG. 12b illustrates the average and standard deviation of current noise ($I_N$) that occurs depending on whether Bax-BH3 peptide binds to Bcl-xL protein, and FIG. 12c is scatter plot representing a change in pattern of ionic current trace that occurs depending on whether Bax-BH3 peptide binds to Bcl-xL protein.

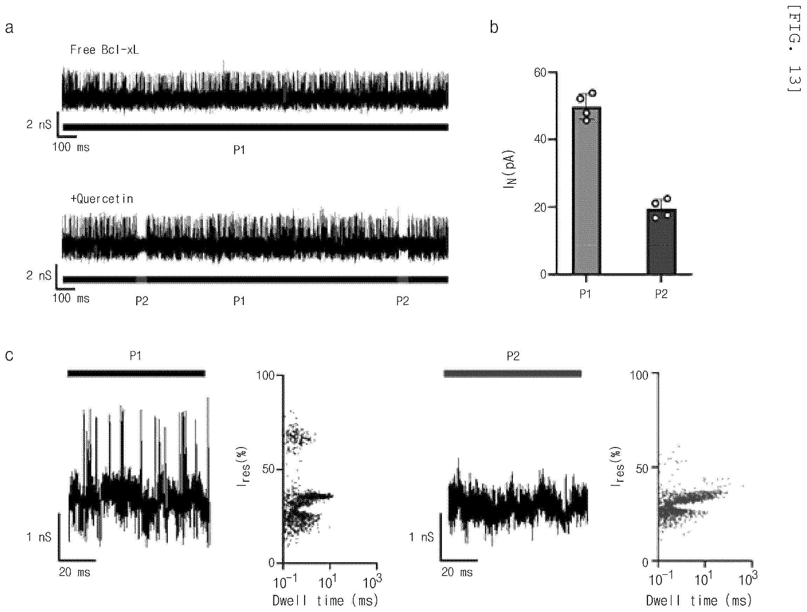

FIG. 13 illustrates the results of analyzing binding of a small molecule drug (Quercetin, $K_D$ = 1.1 $\mu$M) having a low binding affinity for Bcl-xL protein using the modified YaxAB_dN nanopore, FIG. 13a illustrates changes in ionic current signal that occurs depending on whether Quercetin binds to Bcl-xL protein, FIG. 13b illustrates the average and standard deviation of current noise ($I_N$) that occurs depending on whether Quercetin binds to Bcl-xL protein, and FIG. 13c is scatter plot showing changes in pattern of ionic current trace that occurs depending on whether Quercetin binds to Bcl-xL protein.

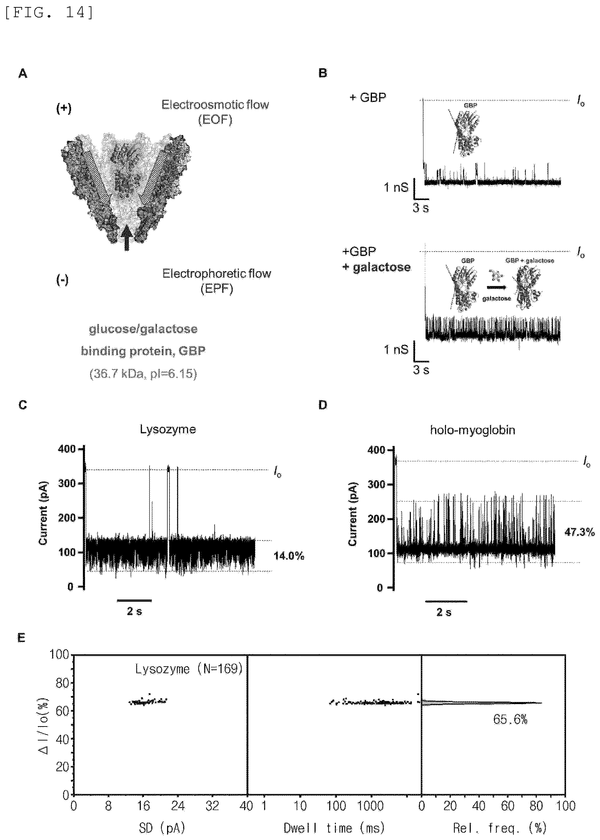

FIG. 14 illustrates the results of electroosmosis-based GBP protein (Glucose/Galactose binding protein) detection using the modified YaxAB_dN nanopore, FIG. 14a illustrates a schematic diagram of electroosmosis-based GBP protein detection in the modified YaxAB_dN nanopore, and FIG. 14b illustrates a current blockade signal for GBP protein and a current blockade signal caused by structural change of a GBP protein that occurs when GBP protein binds to galactose, which is an allosteric activator. FIG. 14c illustrates a current blockade signal for lysozyme protein. FIG. 14d illustrates a current blockade signal for holo-myoglobin protein. FIG. 14e illustrates the two-dimensional scatter plot showing the results of analyzing the current blockade signal, standard deviation, and dwell time factors for lysozyme protein using a two-dimensional scatter plot.

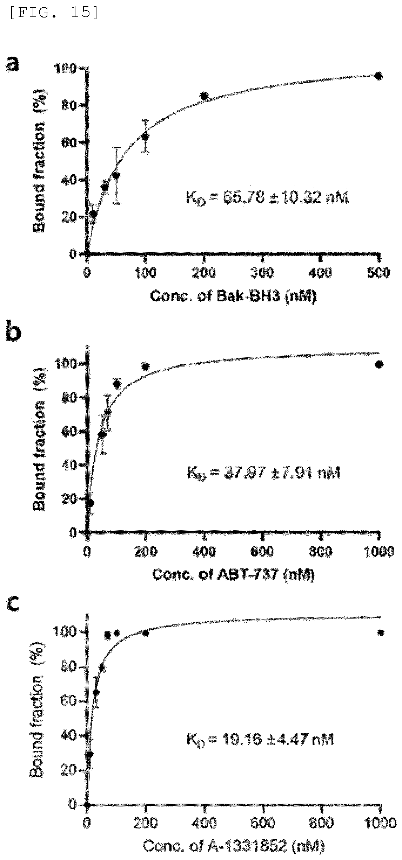

FIG. 15a illustrates a Hill diagram showing a bound fraction of the Bcl-xL+Bak-BH3 complex according to a concentration of Bak-BH3 peptide, FIG. 15b illustrates a Hill diagram showing a bound fraction of the Bcl-xL+ABT-737 complex according to a concentration of ABT-737, and FIG. 15c illustrates a Hill diagram showing a bound fraction of the Bcl-xL+A-1331852 complex according to a concentration of A-1331852.

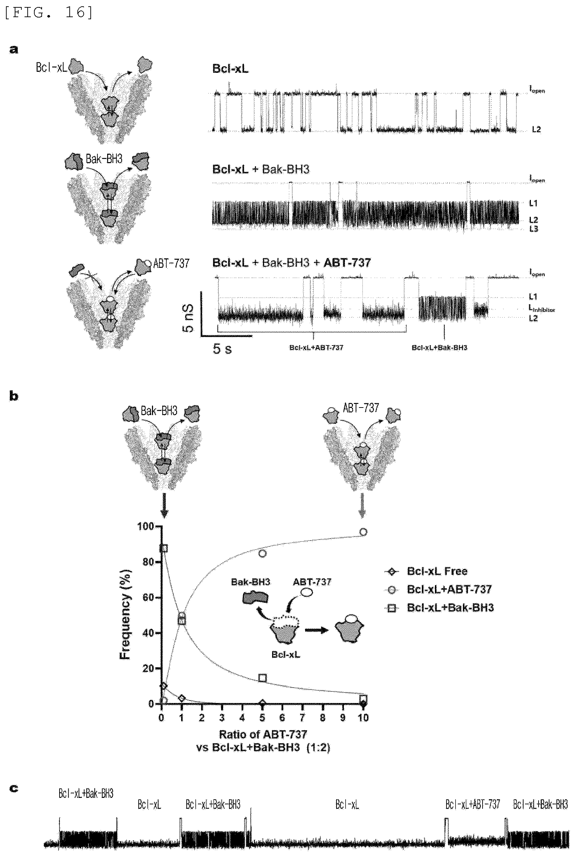

FIG. 16a illustrates a schematic diagram of analytes in the pores and electrical signal results when only free Bcl-xL is present, when Bak-BH3 is reacted with Bcl-xL, and when a small molecule compound (ABT-737) that inhibits the protein-protein interaction between Bcl-xL and Bak-BH3 is treated in a state where Bak-BH3 is pre-reacted with Bcl-xL using the modified YaxAB_dN nanopore, FIG. 16b illustrates the results of measuring signal frequency of three types of analytes (Bcl-xL, Bcl-xL+Bak-BH3, and Bcl-xL+ABT-737) when a small molecule compound (ABT-737) is treated at different concentrations in a state where Bak-BH3 is pre-reacted with Bcl-xL, and FIG. 16c illustrates a current trace showing the three types of analyte signals.

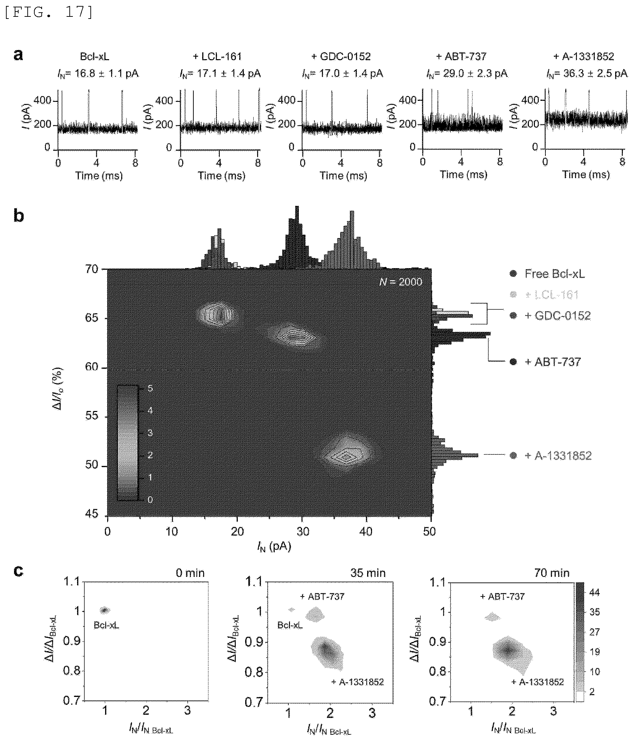

FIG. 17 illustrates the detection of the interaction between Bcl-xL protein and small molecule compounds using the modified YaxAB_dN nanopore, FIG. 17a illustrates ionic current traces when Bcl-xL protein is individually treated with non-binders (LCL-161 and GDC-0152) and binders (ABT-737 and A-1331852), and FIG. 17b illustrates a 2D density contour plot of current blockade ($\Delta I/I_o$)-current noise ($I_N$) when Bcl-xL protein is individually treated with small molecule compounds (LCL-161, GDC-0152, A-1331852, and ABT-737). FIG. 17c illustrates 2D density contour plots of current blockade ($\Delta I/I_o$)-current noise ($I_N$) and the results of real-time monitoring when Bcl-xL protein is treated with a plurality of small molecule compounds (LCL-161, GDC-0152, A-1331852, and ABT-737) at the same time.

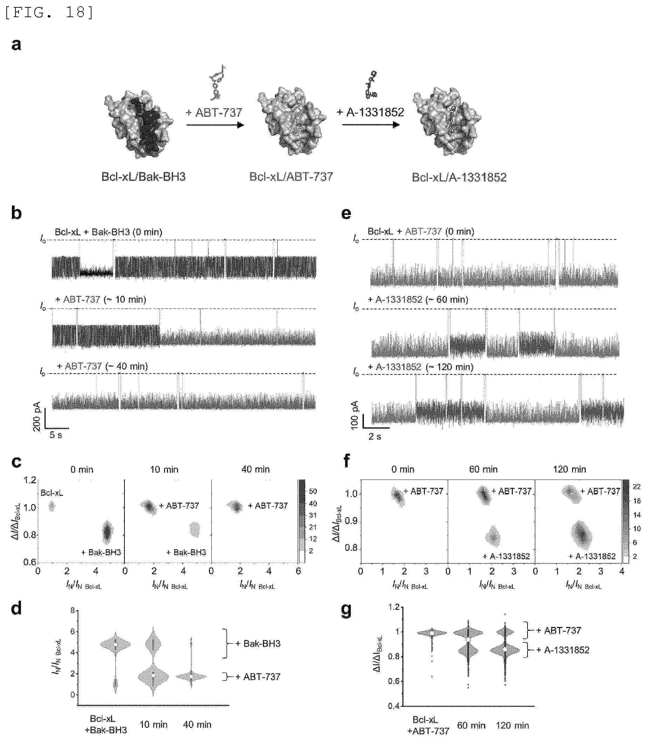

FIG. 18 illustrates the results of analyzing competitive binding of drugs to Bcl-xL protein using the modified YaxAB_dN nanopore, FIG. 18a is a schematic diagram of the competitive binding of the drugs to the Bcl-xL protein, FIG. 18b illustrates an ionic current trace when ABT-737 is titrated to the Bcl-xL/Bak-BH3 complex, and FIGS. 18c and 18d are a 2D density contour plot and a violin plot showing competitive binding of Bak-BH3 and ABT-737 to Bcl-xL protein in real time, respectively. FIG. 18e illustrates an ionic current trace when A-1331852 is titrated to the Bcl-xL+ABT-737 complex, and FIGS. 18f and 18g are a 2D density contour plot and a violin plot showing the competitive binding of ABT-737 and A-1331852 to Bcl-xL protein in real time, respectively.

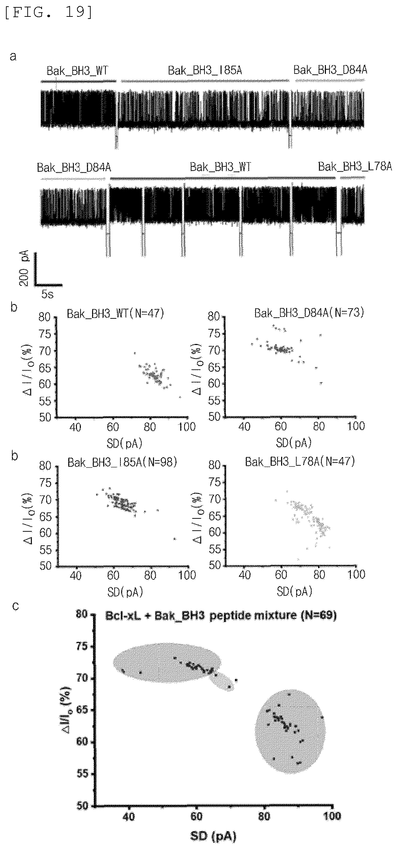

FIG. 19 illustrates the results of detecting peptides that bind to Bcl-xL protein using the modified Yax-AB_dN nanopore and the results of peptide drug screening, FIG. 19a illustrates current blockade signals derived from Bcl-xL protein and Bcl-xL+peptide drug complexes that appear after reacting Bcl-xL protein withwild-type or three types of Bak_BH3 mutant peptides (Bak_BH3_D84A, Bak_BH3_I85A, and Bak_BH3_L78A), FIG. 19b illustrates 2D scatter plots of current noise (SD)-current blockade ($\Delta I/I_o$) for the current blockade signals derived from Bcl-xL protein and Bcl-xL+peptide drug complexes, and FIG. 19c illustrates the scatter plots of analyzing the current blockade signals that appear after reacting Bcl-xL protein and a solution obtained by mixing three types of mutant peptides (Bak_BH3_D84A, Bak_BH3_I85A, and Bak_BH3_L78A) and wild-type Bak_BH3.

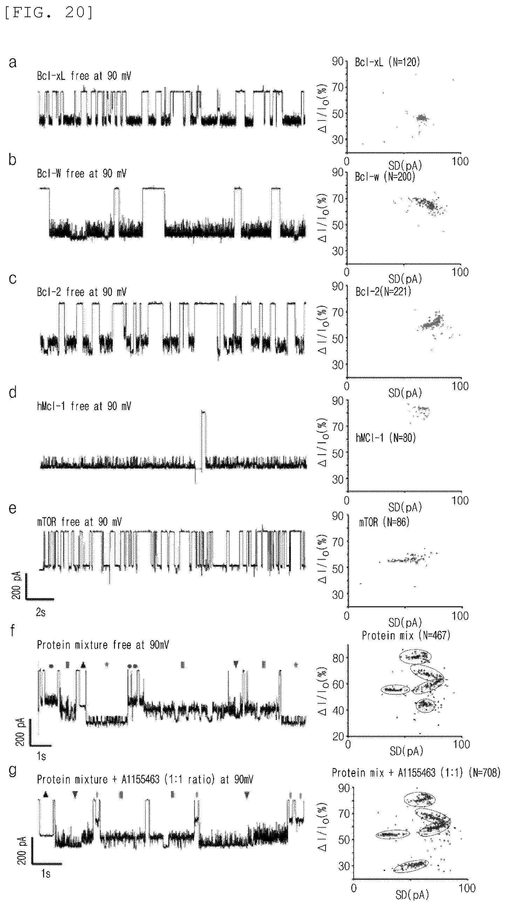

FIG. 20 illustrates the results of electroosmosis-based detection of Bcl-2 family proteins (Bcl-xL, Bcl-w, Bcl-2, and Mcl-1) using the modified Yax-AB_dN nanopore, FIGS. 20a to 20e illustrate current blockade signal patterns and 2D scatter plots of current noise (SD)-current blockade ($\Delta I/I_o$) for each protein, FIG. 20f illustrates the current blockade signals that appear after reacting a solution obtained by mixing equal amounts of Bcl-2 family proteins (Bcl-xL, Bcl-w, Bcl-2, and Mcl-1) and mTOR protein as a negative control, and the scatter plot results obtained by analyzing the same, and FIG. 20g illustrates current blockade signals that appear after further adding an equal ratio of A1155463, which is a small molecule drug targeting Bcl-xL, to the mixed solution at an equal ratio, and the scatter plot results obtained by analyzing the same.

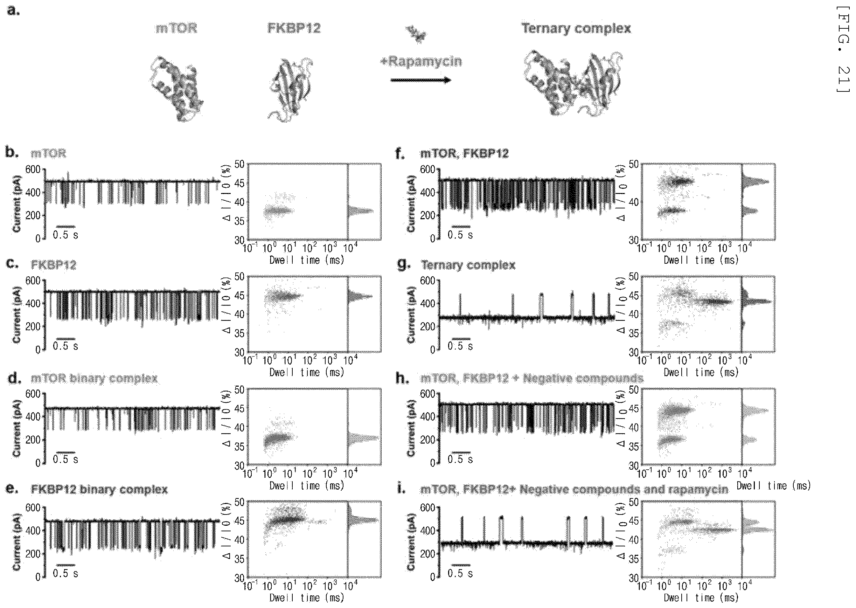

FIG. 21 illustrates the results of detecting a ternary complex (a protein complex containing three different molecules that are bound together) using the modified YaxAB_dN nanopore, FIG. 21a is a schematic diagram showing that mTOR-FRB domain and FKBP12 protein may form a protein-protein interaction via rapamycin, FIGS. 21b and 21c illustrate the current blockade signals for mTOR and FKBP12 proteins and the scatter plotsobtained by analyzing the same, respectively, FIGS. 21d and 21e illustrate the current blockade signals for a binary complex of mTOR and rapamycin and a binary complex of FKBP12 and rapamycin and the scatter plots obtained by analyzing the same, respectively, FIG. 21f illustrates the current blockade signals that appear when mTOR and FKBP12 are added together and the scatter plots obtained by analyzing the same, and FIG. 21g illustrates current blockade signals that appear when mTOR, FKBP12, and rapamycin are added together and the scatter plots obtained by analyzing the same. FIG. 21h illustrates current blockade signals that appear when mTOR and FKBP12 are added together with eight types of negative small molecule compounds (Astemizole, Mibefradil, Terfenadine, Pranlukast, Capecitabine, Dimantine, Sulfanilamide, and Monobenzone) that do not bind to mTOR-FRB domain and scatter plot results obtained by analyzing the same, and FIG. 21i illustrates current blockade signals that appear when mTOR and FKBP12 are added together with eight types of negative small molecule compounds and rapamycin and scatter plot results obtained by analyzing the same.

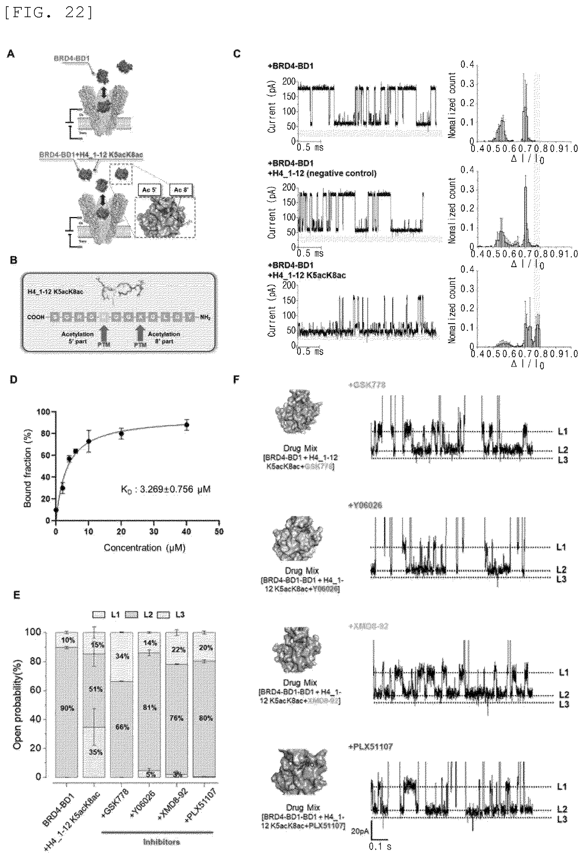

FIG. 22 illustrates the results of the interaction analysis between the BRD4-BD1 protein and the post-translational modification (PTM) histone peptide, as well as the analysis of interaction inhibitors using the modified YaxAB_dN nanopore, FIGS. 22a and 22b illustrate schematic diagrams of the H4 peptide (H4_1-12), which consists of 12 amino acids from the histone protein, and of the binding interaction between the acetylated peptide (H4_1-12 K5acK8-ac)-acetylated at the 5th and 8th lysine residues-and BRD4-BD1, and FIG. 22c illustrates current blockade signals measured after addition of the H4 peptide (H4_1-12) or the peptide acetylated at residues 5 and 8 (H4_1-12 K5acK8ac) to the target protein BRD4-BD1. FIG. 22d illustrates changes in nanopore current blockade signal caused by the interaction of the BRD4-BD1 protein and the acetylated peptide (H4_1-12 K5acK8ac) as a current noise ($I_N$) value including analysis of Power Spectrum Density (PSD) or standard deviation (SD) of ionic current, and a binding affinity ($K_D$) of the acetylated peptide (H4_1-12 K5acK8ac) for the BRD4-BD1 protein as measured by quantitative analysis therefor, FIG. 22e illustrates the results of measuring the nanopore current blockade signals after reacting each of four types of small molecule compounds (GSK778, Y06026, XMD8-92, and PLX51107) that can bind to the BRD4-BD1 protein with the BRD4-BD1 protein, and FIG. 22f illustrates the results of analyzing a ratio of dwell times at the respective levels of current blockade (L1-L3) when reacting the BRD4-BD1 target protein, the acetylated peptide (H4_1-12 K5acK8ac), and each small molecule compound, capturing the analyte in the nanopore, and then measuring subtle changes in ionic currents.

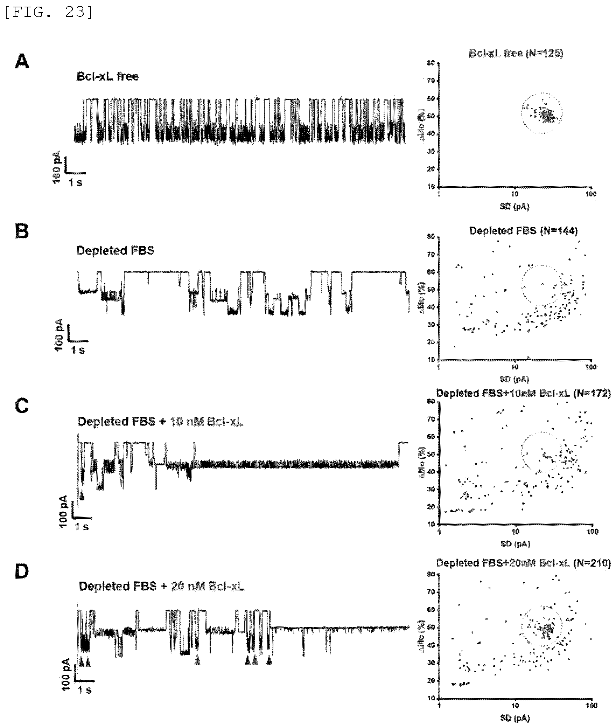

FIG. 23 illustrates the results of selective detection of cancer biomarkers in the presence of serum using the modified YaxAB_dN nanopore. FIG. 23a illustrates a two-dimensional scatter plot of the nanopore current blockade signal and current noise (SD)-current blockade ($\Delta I/I_o$) of the cancer biomarker Bcl-xL protein, and FIG. 23b illustrates a current blockade signal in a depleted fetal bovine serum (FBS) alone state. FIG. 23c illustrates the results of adding 10 nM Bcl-xL protein in the presence of depleted FBS, and FIG. 23d illustrates the results of measuring the nanopore current blockade signal by adding 20 nM Bcl-xL protein in the presence of depleted FBS.

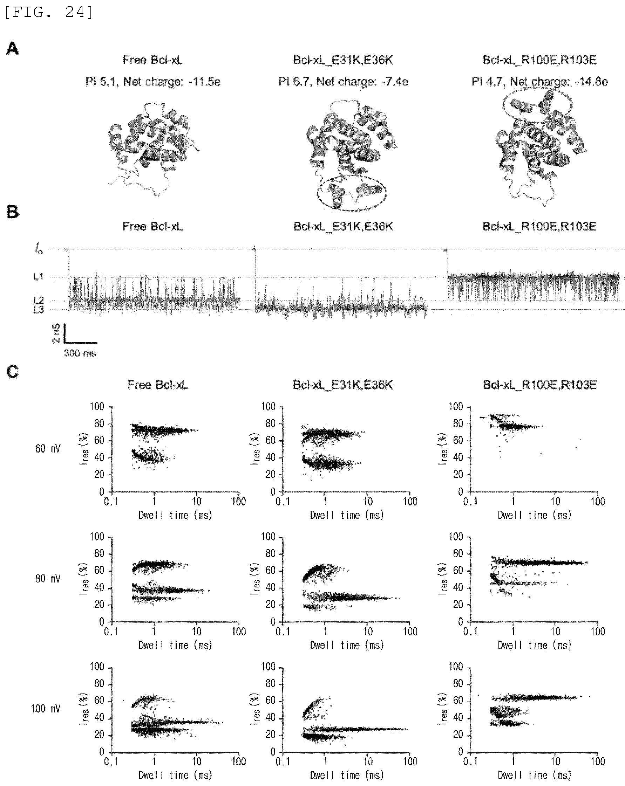

FIG. 24 illustrates the results of detecting and analyzing the wild-type Bcl-xL protein and the mutant proteins Bcl-xL (E31K, E36K) and Bcl-xL (R100E, R103E) using the modified YaxAB_dN nanopore, which the unique nanopore electrical signals of each three type of proteins distinct from each other. FIG. 24a illustrates structural features, FIG. 24b illustrates current signals, and FIG. 24c illustrates the scatter plot results obtained by analyzing the current blockade signals.

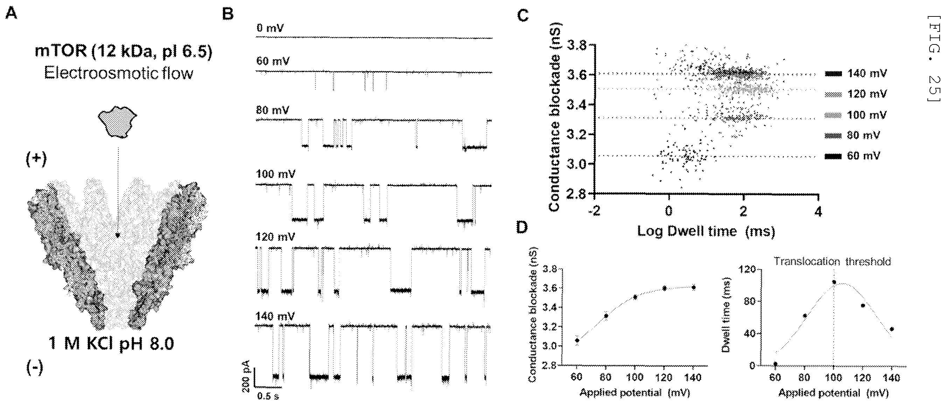

FIG. 25 illustrates the results of electroosmosis-based mTOR protein detection using the modified YaxAB_dN nanopore, which show the results of capture or translocation signals that occur depending on the applied voltage.

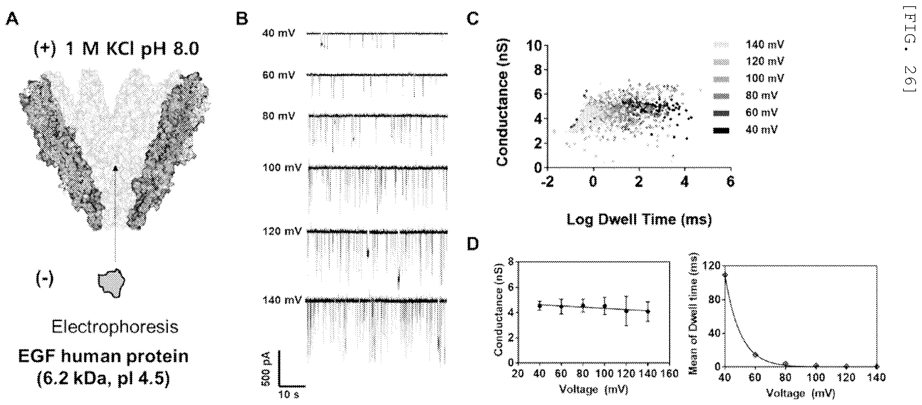

FIG. 26 illustrates the results of electrophoresis-based EGF protein detection using the modified YaxAB_dN nanopore.

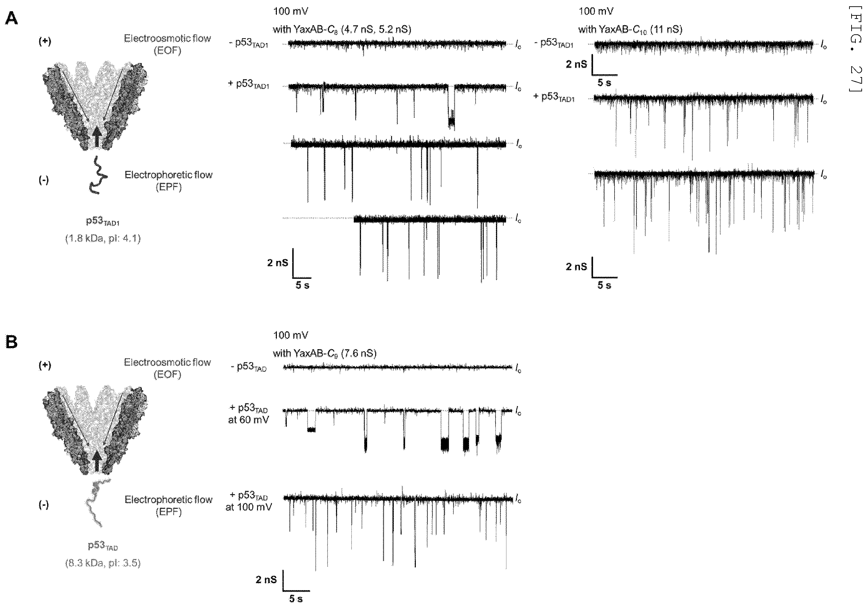

FIG. 27 illustrates the results of detecting p53TAD$_1$ peptide (1.8 kDa, residues 15 to 29) and p53TAD protein (8.3 kDa, residues 1 to 73), which are intrinsically disordered proteins (IDPs), by the principle of electrophoresis using the modified YaxAB_dN nanopore.

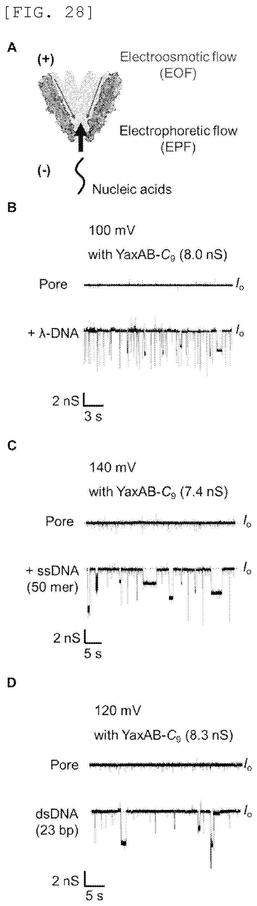

FIG. 28 illustrates the results of detecting double-stranded nucleic acids and single-stranded nucleic acids by the principle of electrophoresis using the modified YaxAB_dN nanopore.

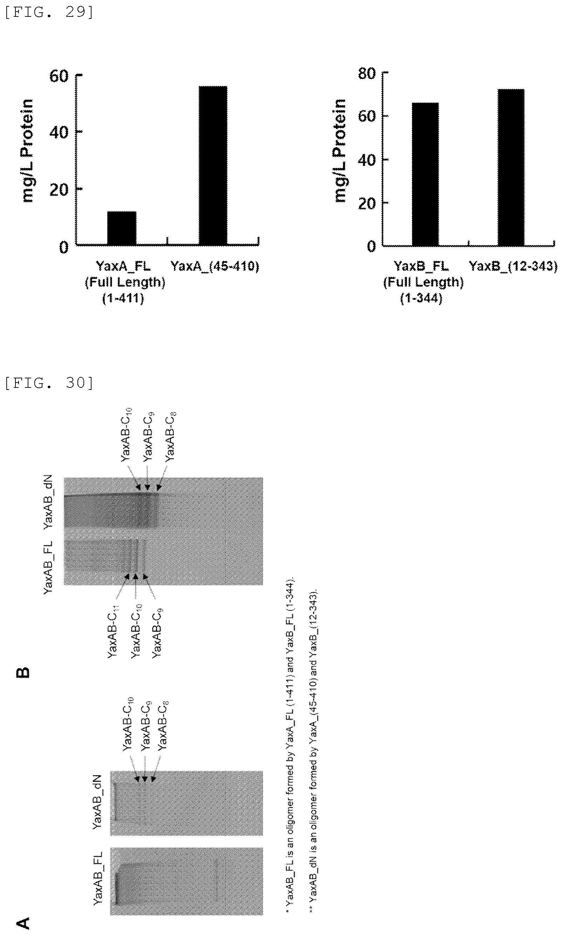

FIG. 29 illustrates the results of comparing the protein yields of wild-type YaxA_FL (SEQ ID NO: 3), wild-type YaxB_FL (SEQ ID NO: 4), YaxA_(45-410) (SEQ ID NO: 1), and YaxB_(12-343) (SEQ ID NO: 2) purified under the same conditions.

FIG. 30 illustrates bands of different oligomers obtained by electrophoresis of the modified YaxAB_dN nanopores on a gradient blue native polyacrylamide gel and illustrates that the oligomer formation tendencies of wild-type YaxAB (YaxAB_FL) and modified YaxAB_dN are different. In FIG. 30, the wild-type YaxAB refers to an oligomer formed of YaxA_FL (a first monomer consisting of SEQ ID NO: 3) and YaxB_FL (a second monomer consisting of SEQ ID NO: 4). FIG. 30a illustrates the results of forming nanopores by oligomerization under low protein concentration conditions, and FIG. 30b illustrates the result of forming nanopores by oligomerization under high protein concentration conditions.

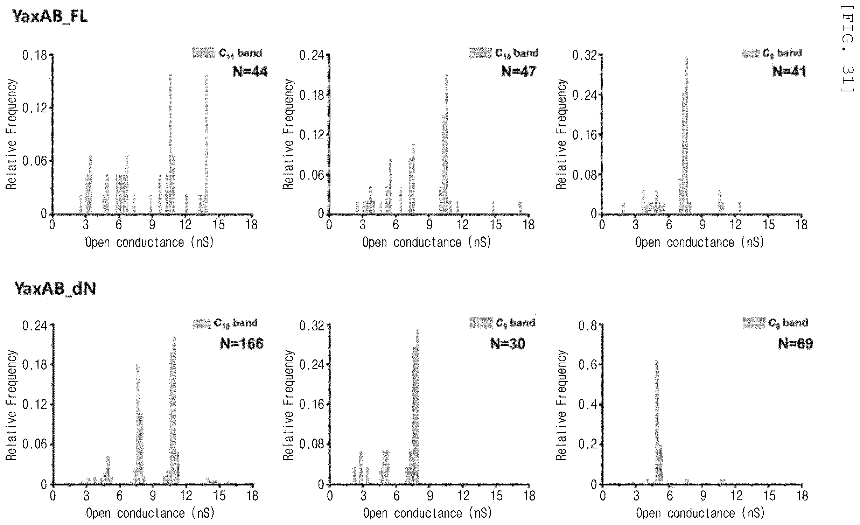

FIG. 31 illustrates the tendencies of the YaxAB nanopore formation from the respective protein bands obtained by electrophoresis of the wild-type nanopores and the modified YaxAB_dN nanopores and the results of comparing the conductivity measurement values of the respective nanopores.

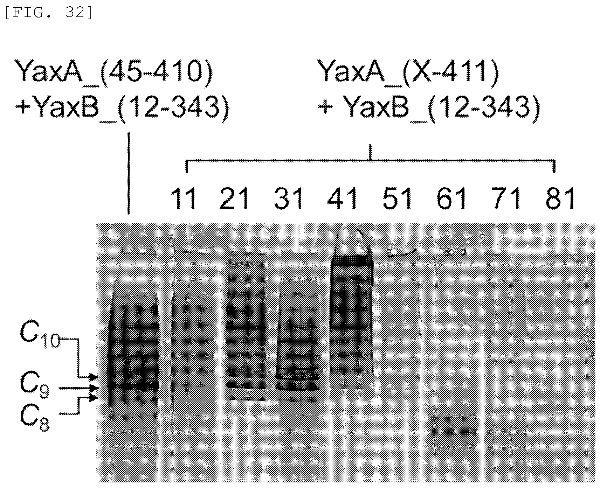

FIG. 32 shows bands of various YaxAB oligomers observed on a gradient blue native polyacrylamide gel, where the nanopores were formed using sequence-length-adjusted YaxA and YaxB _(12-343). The figure demonstrates that the pattern of oligomer formation differs depending on the sequence length of YaxA, by comparing reactions between YaxB_(12-343) (SEQ ID NO: 2) and either YaxA_(45-410) (SEQ ID NO: 1) or sequence-length-adjusted YaxA_(X-411).

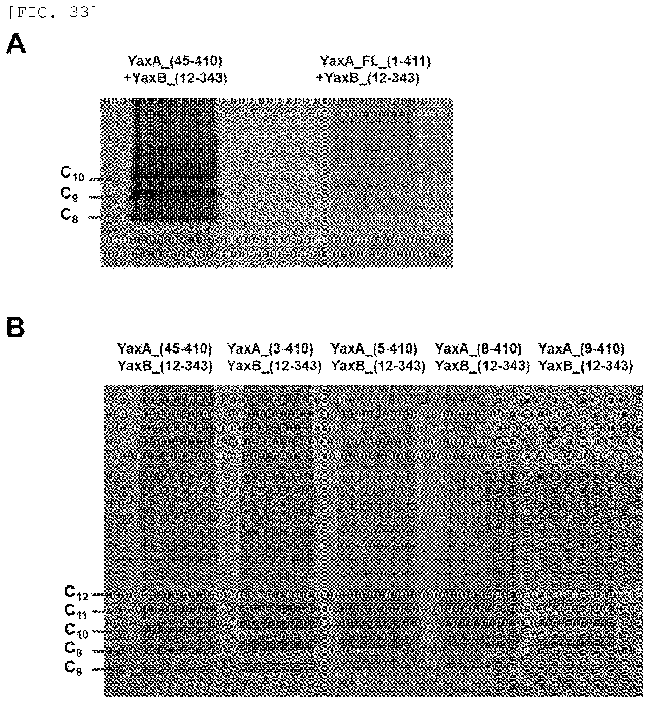

FIG. 33 illustrates bands of YaxAB oligomers obtained by electrophoresis of the YaxAB nanopores on a gradient blue native polyacrylamide gel, and FIG. 33a illustrates the results of the tendencies of the YaxAB oligomers formed after reacting the wild-type YaxA_FL or the first monomer YaxA (45-410) consisting of SEQ ID NO: 1 with the second monomer YaxB _ (12-343) consisting of SEQ ID NO: 2. FIG. 33b illustrates bands of YaxAB oligomers obtained by electrophoresis of the YaxAB nanopores on a gradient blue native polyacrylamide gel, and illustrates the results of tendencies of the YaxAB oligomers formed after individually reacting YaxA_(45-410), YaxA_(3-410), YaxA_ (5-410), YaxA_(8-410), or YaxA_(9-410) with the second monomer YaxB_(12-343) consisting of SEQ ID NO: 2.

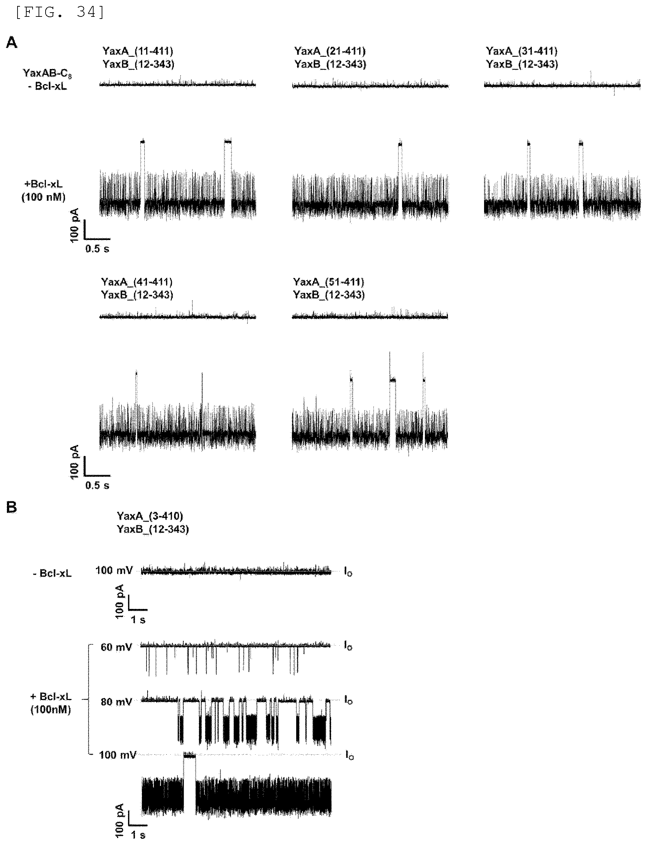

FIG. 34 illustrates the results of applying the YaxAB oligomer to a membrane and verifying the Bcl-xL detection ability of the nanopore system through an electrical measurement experiment, and FIG. 34a illustrates the results of verifying the detection capability of the YaxAB nanopore system for the Bcl-xL protein through electrical measurements, using YaxAB oligomers formed by complexation of YaxB_(12-343) (SEQ ID NO: 2) with various sequence-length variants of YaxA: YaxA_(11-411), YaxA_(21-411), YaxA_(31-411), YaxA_(41-411), and YaxA_(51-411). FIG. 34b illustrates the results of verifying the detection ability of the YaxAB nanopore system for Bcl-xL protein through an electrical measurement experiment using YaxAB oligomers formed by binding of YaxA_(3-410) and the second monomer YaxB_(12-343) consisting of an amino acid sequence of SEQ ID NO: 2.

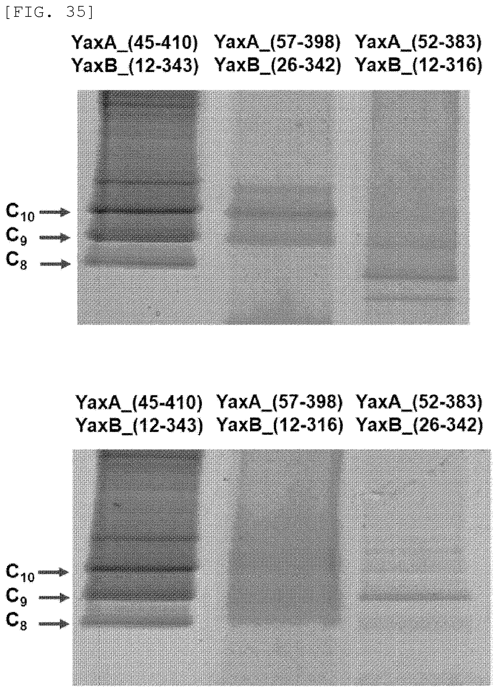

FIG. 35 illustrates the results of bands of different oligomers obtained by electrophoresis of four types of YaxAB oligomers composed of nanopore subunits formed by binding of YaxA_(57-398) or YaxA_(52-383) and YaxB_(26-342) or YaxB _(12-316), respectively, which are the minimum sequences capable of forming YaxAB oligomers, and a YaxAB_dN oligomer composed of nanopore subunits formed by binding of YaxA (45-410) and YaxB_(12-343) on a gradient blue native polyacrylamide gel.

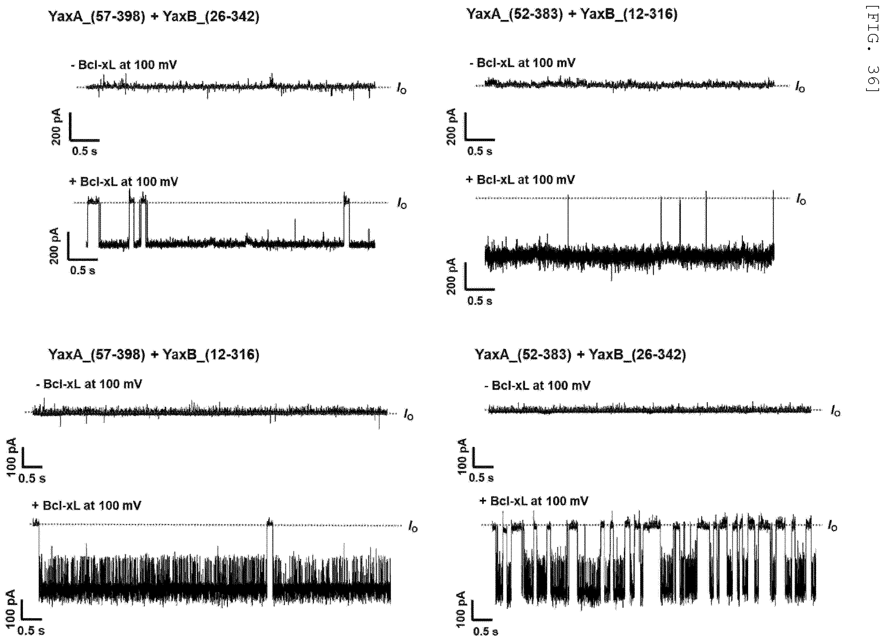

FIG. 36 illustrates YaxAB nanopores prepared with the minimum sequence capable of forming YaxAB oligomers, and illustrates the results of applying four types of YaxAB oligomers formed by binding of each of YaxA_(57-398) or YaxA_(52-383) and YaxB_(26-342) or YaxB_(12-316) to a membrane and verifying the Bcl-xL detection ability of the nanopore system through an electrical measurement experiment.

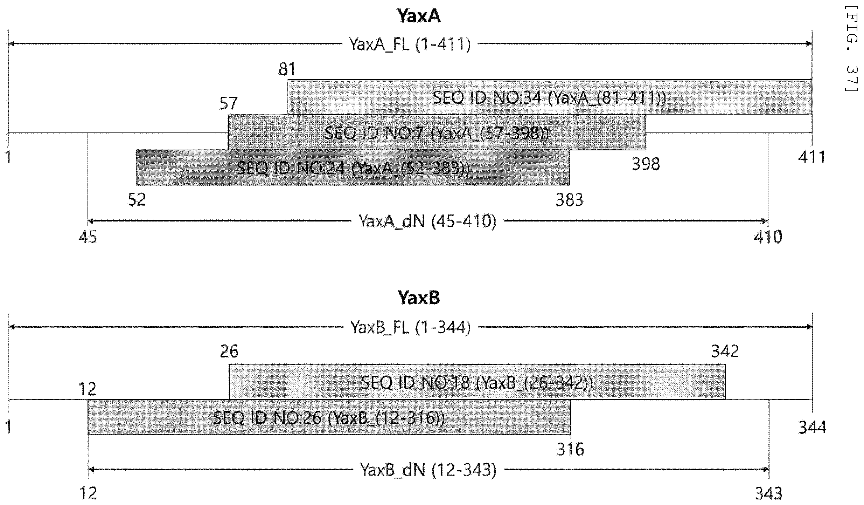

FIG. 37 illustrates the results showing YaxA minimum sequences and YaxB minimum sequences capable of forming YaxAB oligomers.

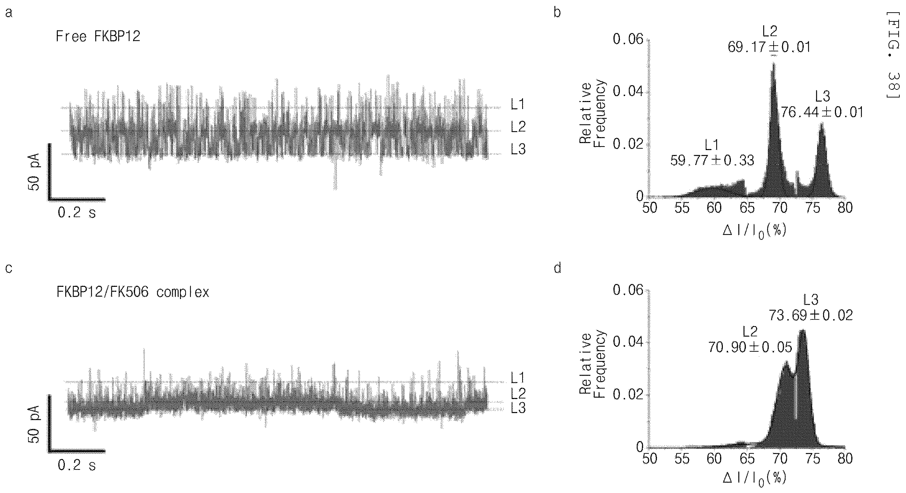

FIG. 38 is a histogram showing a relative event frequency according to a current trace change and a current blockade ratio ($\Delta I/I_o$) observed when electrically neutral FKBP12 and FKBP12-FK506 complexes are applied to the YaxAB nanopore system.

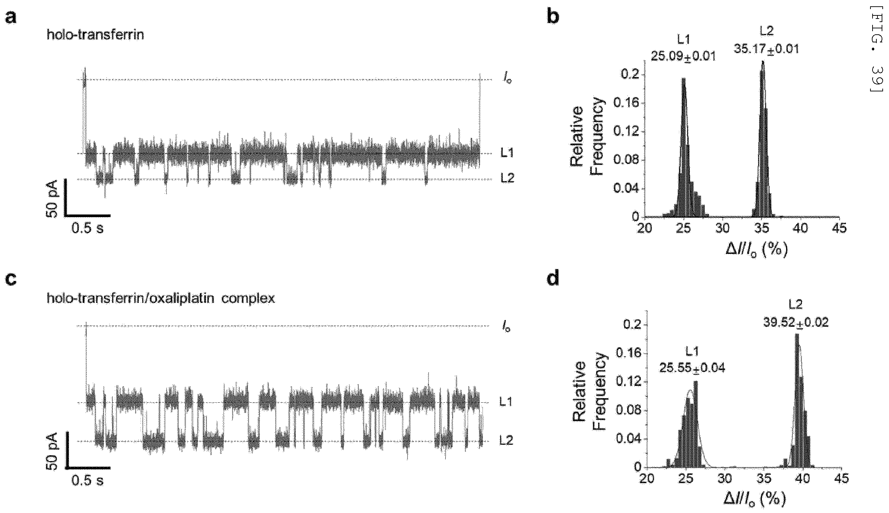

FIG. 39 is a histogram showing the relative event frequency, along with the current blockade ratio ($\Delta I/I_o$), based on changes in the current trace when oxaliplatin is applied to holo-transferrin alone and holo-transferrin-oxaliplatin complexes in the YaxAB nanopore system.

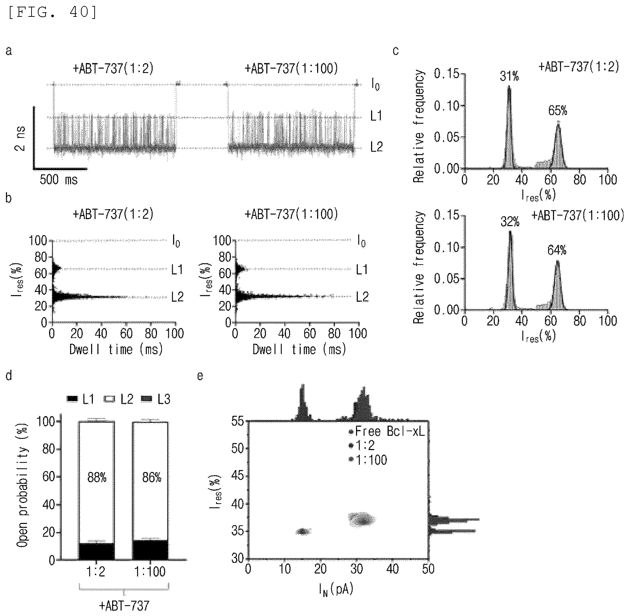

FIG. 40 illustrates the results of comparing the signals of the analyte-compound complex under a low concentration and a high concentration of ABT-737 compound in the YaxAB nanopore system.

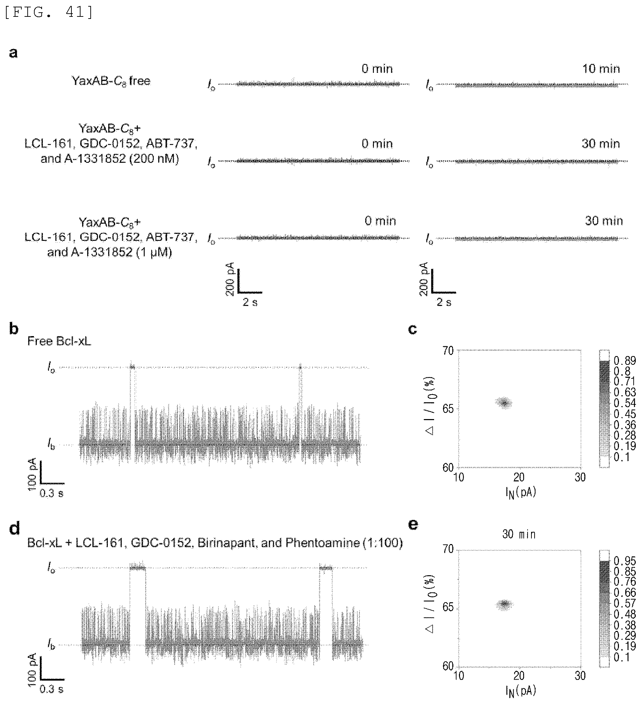

FIG. 41 illustrates the results showing whether non-specific signals are generated upon addition of a non-target compound depending on the presence or absence of an analyte in the YaxAB nanopore system.

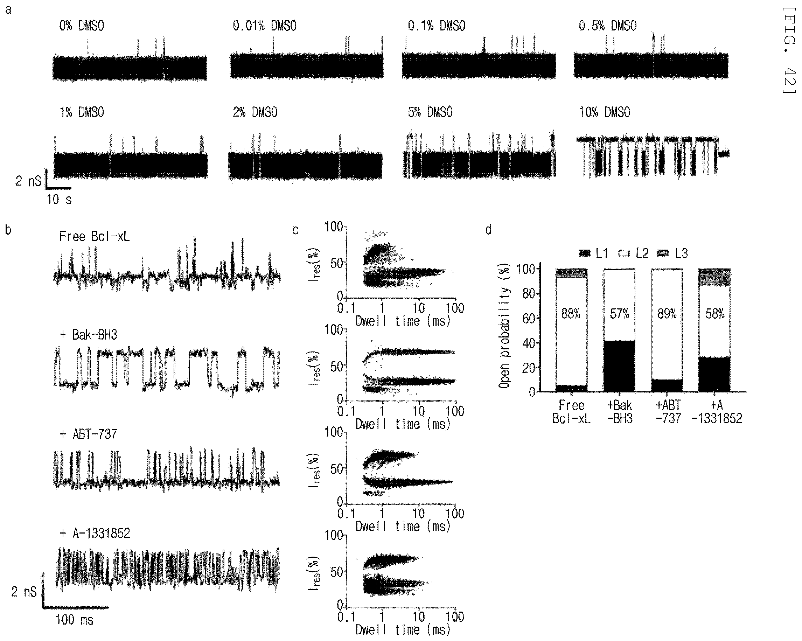

FIG. 42 illustrates the results of comparing the signals of the analyte-compound complex under DMSO conditions at various concentrations in the YaxAB nanopore system.

## BEST MODE FOR CARRYING OUT THE INVENTION

Hereinafter, the present invention will be described in detail.

### 1. <u>YaxAB nanopore subunit</u> and <u>YaxAB nanopore containing the same</u>

An aspect of the present invention provides a YaxAB nanopore subunit and a YaxAB nanopore.

YaxAB is a heterodimer in which YaxA protein and YaxB protein are bound and is one of the pore-forming toxins that allow *Yersinia enterocolitica,* which is an intestinal pathogen that causes a variety of diseases from mild diarrhea to severe systemic bacteremia, to bind to and invade host cells. In *Yersinia enterocolitica,* RovA regulates transcription of virulence genes, of which YE1984 and YE1985 are identified as genes upregulated by RovA and are named yaxA and yaxB, respectively, due to their homology with the cytotoxin genes xaxA and *xaxB* of *Xenorhabdus nematophila.* Both the YaxA protein and YaxB protein have a membrane-active moiety, but only the YaxA protein may bind to the membrane on its own. YaxB protein binds to membrane-embedded YaxA protein so as to induce the heterodimer to form a transmembrane helix, and YaxAB may form a YaxAB nanopore in a host cell membrane by additional oligomerization. A wild-type YaxAB nanopore mainly has a decamer structure composed of 10 YaxAB heterodimers, and in the YaxAB nanopore, the YaxB protein is arranged to face the lumen of the pore, and the YaxA protein forms a structure surrounding the YaxB protein. That is, YaxAB-mediated toxicity may induce osmotic lysis by forming pores in the membrane of the host cells.

In an aspect, the YaxAB nanopore of the present invention may be formed by containing at least one subunit containing a membrane-active moiety or a structure with transmembrane α-helices as part of the wild-type YaxAB, which is a pore-forming toxin, and in particular, the subunit constituting the YaxAB nanopore of the present invention may contain a first monomer and a second monomer. In this case, the first monomer may have an amino acid sequence having 70% or more homology with an amino acid sequence of any one of SEQ ID NO: 7, SEQ ID NO: 24, or SEQ ID NO: 34, and the second monomer may have an amino acid sequence having 70% or more homology with an amino acid sequence of SEQ ID NO: 18 or SEQ ID NO: 25.

In the present invention, the "YaxAB nanopore" includes all types of proteins that may be referred to as YaxAB proteins, and specifically, may include all isoforms of YaxAB proteins, and all homologs, orthologs, and paralog proteins. In addition, the YaxAB nanopore of the present invention may be non-natural proteins. The non-natural protein refers to the proteins that consists of amino acid sequences that do not exist in nature, is not normally expressed by a cell or organism, and is artificially produced.

For example, in the present invention, the first monomer contained in the YaxAB nanopore may be, but is not limited to, a protein having an amino acid sequence of any one of SEQ ID NO: 7, SEQ ID NO: 24, or SEQ ID NO: 34, or an amino acid sequence of NCBI accession NO. WP_005169901.1 (SEQ ID NO: 3), WP_011816273.1 (SEQ ID NO: 35), HDL8508204.1, HDL8476175.1, HDL7646675.1, PNM17258.1, WP_050336257.1, HDL8287337.1, HDL7822654.1, HDL7748605.1, HDL6737510.1, HEB4799346.1, WP_221871123.1, EKN3734298.1, EKN3569010.1, HDL7089514.1, WP_050160085.1, WP_221876226.1, EKN4747040.1, WP_050916836.1, HDM8435746.1, HDL8238855.1, WP_019079472.1, EKN3341278.1, WP_050163370.1, HDL7317648.1, HDM8081034.1, WP_050870897.1, EKN3737775.1, HDL7467110.1, HDL7471348.1, WP_046694975.1, WP_301211860.1, HDL7726911.1, WP_268215747.1, EKN5089609.1, WP_057620566.1, EKN6360258.1, WP_019083154.1, EKN5159302.1, WP_050131624.1, ELI8015017.1, EKN4802793.1, EKN6165952.1, ELI8293390.1, ELI8097018.1, WP_050157801.1, EKN4708697.1, EKN5102380.1, EKN5913011.1, WP_050164534.1, WP_242365000.1, HDL6759266.1, WP_050327956.1, EKN3969341.1, HDL8127360.1, EKN3724092.1, WP_050334096.1, HDL7761807.1, HDL6872781.1, EKN3573207.1, EKN3562735.1, EKN5949803.1, HDL6624424.1, WP_219649234.1, EKN3957447.1, EKN3611203.1, EKN5162868.1, WP_172654801.1, HDL6886351.1, WP_263797395.1, EKN3989601.1, HDL7855057.1, HDW8038128.1, EKN3870380.1, WP_057631219.1, EKN5074149.1, WP_219650267.1, HDL6522072.1, EKN4058214.1, EKN4068486.1, HDL6961480.1, WP_075338710.1, EKN5112876.1, HDL8368473.1, HDL7423034.1, HDM8447363.1, EKN6363360.1, EKN3564743.1, HDL7684594.1, HDL7735658.1, HDL7339639.1, HDL7966642.1, CNH41000.1, HDL7909665.1, WP_219648645.1, WP_219643575.1, CQD54886.1, CNG18579.1, EKN4119565.1, WP_145592082.1, WP_038639531.1, AJJ34365.1, WP_050114520.1, WP_271298628.1, WP_004390824.1, WP_050095452.1, WP_145562021.1, WP_159678334.1, WP_309477958.1, WP_145510697.1, WP_219646505.1, WP_145556850.1, WP_145537515.1, WP219655358.1, CNG40075.1, EKN3837153.1, EKN3315583.1, WP_301178346.1, HDL8028676.1, HDL8052082.1, HDL7585724.1, HDL8104119.1, WP_005164820.1, WP_050127938.1, WP_271306546.1, CBX71227.1, KGA73448.1, HDL8279144.1, HDL8024634.1, WP_115240285.1, WP_254461835.1, HDL8320198.1, WP_050124432.1, WP_050326508.1, HDL8083732.1, EKN3713082.1, HDL8750155.1, CNK45388.1, WP_221864008.1, SUP62773.1, or the like, or a variant thereof, and specifically, may be a protein consisting of the amino acid sequence or the variant thereof. A variant of the amino acid sequence may include a variant peptide having different sequences by deletion, insertion, or substitution of amino acids, or a combination thereof, within a range that does not affect the function of the amino acid sequence, or may be in the form of a protein fragment having the same function. Amino acid modifications at the protein and peptide level that do not change the activity of the amino acid sequences are known in the art, and in some cases, the amino acid sequence may be modified by phosphorylation, sulfation, acrylation, glycosylation, methylation, farnesylation, or the like. Therefore, a variant of the amino acid sequence may have an amino acid sequence that is "substantially" identical to the amino acid sequences described above. The peptide having the substantially identical amino acid sequence may be an amino acid sequence having 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, or 99.5% or more homology with each of the amino acid sequences, but is not limited thereto, and any protein having an amino acid sequence having 70% or more homology with each of the amino acid sequences and having an activity of binding to a lipid membrane is included within the scope of the present invention.

In addition, in the present invention, the second monomer contained in the YaxAB nanopore may be, but is not limited to, a protein having an amino acid sequence of SEQ ID NO: 18 or SEQ ID NO: 25 or an amino acid sequence of NCBI accession NO. WP_011816274.1 (SEQ ID NO: 4), WP_050336255.1 (SEQ ID NO: 36), WP_005169898.1, PNM17259.1, WP_050137918.1, HDL7748604.1, HDL6508072.1, WP_050137583.1, WP_050322893.1, HDL7822655.1, HDL7646676.1, WP_050321988.1, WP_221871120.1, HDL6961479.1, WP_050298175.1, CNG06681.1, EKN5919601.1, EKN5074150.1, WP_075338711.1, HDL7057906.1, WP_219656641.1, WP_050157802.1, ELI8015018.1, EKN4068485.1, HDL7533782.1, WP_057631218.1, WP_050916837.1, EKN6259816.1, EKN3396085.1, HDL7423033.1, EKN4822249.1, WP_050880005.1, WP_050875182.1, HDL7335482.1, WP_050163371.1, EKN3734297.1, HDL6886350.1, HEB4799167.1, WP_263700684.1, EKN3341279.1, HDL7478311.1, EKN3957448.1, WP_050327958.1, HDL7339638.1, HDL6946689.1, WP_019079471.1, HDV7159892.1, HDL7467111.1, WP_172654802.1, WP_050870895.1, EKN3469507.1, WP_019083153.1, WP_076706633.1, HDW8038129.1, CQG99659.1, WP_050945964.1, HDL6872782.1, WP_046694976.1, WP_263699488.1, WP_263697703.1, EKN3724091.1, HDL7089513.1, CQH55963.1, HDL7666636.1, EKN5102379.1, WP_046050899.1, HDL6959493.1, EKN5159301.1, HDL8096476.1, EKN3683754.1, EKN3973834.1, WP_219650266.1, EKN6365751.1, HDL8258876.1, EKN4119566.1, HDM8089399.1, WP_263696663.1, EKN6178815.1, ELI8097017.1, HDL7909664.1, WP_219643580.1, WP_050149278.1, CNH43347.1, HDL8000813.1, WP_020283191.1, HDL8788876.1, HDL7394360.1, HDL7984656.1, WP_005164817.1, HDL8028561.1, CBY27149.1, HDL8032249.1, HDL8024436.1, HDL8004719.1, HDL6519422.1, HDL8048091.1, WP_016266185.1, CCV61752.1, WP_049526748.1, WP_145535945.1, WP_050114521.1, WP_309477957.1, WP_289816262.1, WP_050291733.1, WP_145589360.1, WP_038633022.1, WP_265526189.1, WP_145537514.1, WP_050095454.1, WP_057634251.1, WP_050289682.1, WP_145531133.1, WP_145586506.1, WP_100286507.1, WP_219655343.1, WP_049557371.1, WP_087795913.1, WP_145510696.1, WP_271304184.1, WP_004390823.1, WP_145556851.1, WP_050120114.1, WP_219648204.1, WP_159678331.1, or the like, or a variant thereof, and specifically, may be a protein consisting of the amino acid sequence or the variant thereof. As in the first monomer, a variant of the amino acid sequence may include a variant peptide having different sequences by deletion, insertion, or substitution of amino acids, or a combination thereof, within a range that does not affect the function of the amino acid sequence, or may be in the form of a protein fragment having the same function. Amino acid modifications at the protein and peptide level that do not change the activity of the amino acid sequence are known in the art, and in some cases, the amino acid sequence may be modified by phosphorylation, sulfation, acrylation, glycosylation, methylation, farnesylation, or the like. Therefore, a variant of the amino acid sequence may have an amino acid sequence that is "substantially" identical to the amino acid sequences described above. The peptide having the substantially identical amino acid sequence may be an amino acid sequence having 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, or 99.5% or more homology with each of the amino acid sequences, but is not limited thereto, and any protein having an amino acid sequence having 70% or more homology with each of the amino acid sequences and having an activity of binding to a lipid membrane is included within the scope of the present invention.

Specifically, in the present invention, the subunit may contain, but is not limited to, a first monomer having any one amino acid sequence selected from the group consisting of SEQ ID NO: 1, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 19, SEQ ID NO: 20, SEQ ID NO: 21, SEQ ID NO: 22, SEQ ID NO: 23, SEQ ID NO: 24, SEQ ID NO: 30, SEQ ID NO: 31, SEQ ID NO: 32, SEQ ID NO: 33, and SEQ ID NO: 34; a second monomer having any one amino acid sequence selected from the group consisting of SEQ ID NO: 2, SEQ ID NO: 18, SEQ ID NO: 25, SEQ ID NO: 26, SEQ ID NO: 27, SEQ ID NO: 28, and SEQ ID NO: 29.

In another aspect, the YaxAB nanopore of the present invention includes a first opening, a middle area, and a second opening, and has a funnel shape, wherein an outer diameter of a lumen of the first opening may be 5 nm or more, the second opening may include a constriction, a diameter of the constriction may be 0.5 nm or more, an outer diameter of a lumen of the second opening may be 1 nm or more, and a depth of the lumen may be 5 nm or more.

Specifically, the YaxAB nanopore includes the first opening, the middle area, and the second opening. The first opening is a portion that is not inserted into the membrane and exposed and serves as an inlet, the outer diameter of the lumen located in the first opening may be 5 nm or more, for example, about 5 to 30 nm, 6 to 25 nm, 7 to 20 nm, 8 to 18 nm, or 10 to 17 nm, and specifically, in the case of the YaxAB $C_8$-$C_{10}$ nanopores, the outer diameter of the lumen of the first opening may be about 10 to 17 nm. The second opening is a portion that is inserted into the membrane and serves as an outlet, the outer diameter of the lumen of the second opening may be 1 nm or more, for example, about 1 to 20 nm, 1.1 to 15 nm, 1.2 to 10 nm, 1.3 to 5 nm, or 1.4 to 3 nm, and specifically, in the case of the YaxAB $C_8$-$C_{10}$ nanopores, a diameter of the lumen located at the second opening may be about 1.9 to 3.1 nm. The middle area is a portion connecting the first opening and the second opening, and the middle area may have a shape in which a diameter of the lumen gradually decreases as it goes from the first opening to the second opening. The depth of the lumen extending from the first opening to the second opening through the middle area in the YaxAB nanopore may be 5 nm or more, for example, about 5 to 20 nm, 6 to 18 nm, 7 to 15 nm, or 10 to 13 nm. Therefore, the YaxAB nanopore may have a funnel shape with a wide inlet and a relatively much narrower outlet. In addition, the lumen of the nanopore may have a funnel shape or a coffee dripper shape with a deep depth and a narrowing width.

The second opening includes a constriction. The constriction is a region in the lumen where the width is narrowed, and a diameter of the constriction may be 0.5 nm or more, for example, about 0.5 to 18 nm, 1 to 15 nm, 1.5 to 10 nm, 1.7 to 5 nm, or 1.9 to 3.1 nm, and specifically, in the case of YaxAB nanopores $C_8$-$C_{10}$, the diameter may be about 1.9 to 3.1 nm.

The constriction may be electronegative or electropositive. When the constriction is electronegative, a surface charge of the lumen of the constriction has a characteristic of having a net negative charge. Specifically, in the YaxAB nanopore of the present invention, the subunits are arranged so that the second monomer faces the lumen, and when the surface charge of the second monomer has a negative charge, the constriction may have a negative surface charge at pH 7.5. Therefore, the electronegative constriction may impart selectivity for a positively charged substance and/or cation in a direction from the first opening to the second opening or in a direction from the second opening to the first opening. In this case, the YaxAB nanopore may have a cation selectivity that is 1.1 times or more, 1.4 times or more, 1.7 times or more, or 2.3 times or more greater than an anion selectivity. In a specific embodiment of the present invention, as a result of calculating the ion selectivity of the YaxAB nanopore of the present invention, it was confirmed that the YaxAB nanopore had a cation selectivity that is 1.4 times or more greater than an anion selectivity under a condition of pH 7.5 (see FIG. 9).

When the constriction is electropositive, the surface charge of the lumen of the constriction has a characteristic of having a net positive charge. Specifically, in the YaxAB nanopore of the present invention, the subunits are arranged so that the second monomer faces the lumen, and when the surface charge of the second monomer has a positive charge, the constriction may have a positive surface charge at pH 7.5. Therefore, the electropositive constriction may impart selectivity for a negatively charged substance and/or anion in a direction from the first opening to the second opening or in a direction from the second opening to the first opening. In this case, the YaxAB nanopore may have an anion selectivity that is 1.1 times or more, 1.4 times or more, 1.7 times or more, or 2.3 times or more greater than a cation selectivity. Therefore, the YaxAB nanopore of the present invention may have an ion selectivity of 1.1 times or more.

Meanwhile, the first monomer may be expressed from a nucleic acid sequence or a variant thereof encoding a protein having an amino acid sequence of any one of SEQ ID NO: 7, SEQ ID NO: 24, or SEQ ID NO: 34, or an amino acid sequence of NCBI accession NO. WP_005169901.1 (SEQ ID NO: 3), WP_011816273.1 (SEQ ID NO: 35), HDL8508204.1, HDL8476175.1, HDL7646675.1, PNM17258.1, WP_050336257.1, HDL8287337.1, HDL7822654.1, HDL7748605.1, HDL6737510.1, HEB4799346.1, WP_221871123.1, EKN3734298.1, EKN3569010.1, HDL7089514.1, WP_050160085.1, WP_221876226.1, EKN4747040.1, WP_050916836.1, HDM8435746.1, HDL8238855.1, WP_019079472.1, EKN3341278.1, WP_050163370.1, HDL7317648.1, HDM8081034.1, WP_050870897.1, EKN3737775.1, HDL7467110.1, HDL7471348.1, WP_046694975.1, WP_301211860.1, HDL7726911.1, WP_268215747.1, EKN5089609.1, WP_057620566.1, EKN6360258.1, WP_019083154.1, EKN5159302.1, WP_050131624.1, ELI8015017.1, EKN4802793.1, EKN6165952.1, ELI8293390.1, ELI8097018.1, WP_050157801.1, EKN4708697.1, EKN5102380.1, EKN5913011.1, WP_050164534.1, WP_242365000.1, HDL6759266.1, WP_050327956.1, EKN3969341.1, HDL8127360.1, EKN3724092.1, WP_050334096.1, HDL7761807.1, HDL6872781.1, EKN3573207.1, EKN3562735.1, EKN5949803.1, HDL6624424.1, WP_219649234.1, EKN3957447.1, EKN3611203.1, EKN5162868.1, WP_172654801.1, HDL6886351.1, WP_263797395.1, EKN3989601.1, HDL7855057.1, HDW8038128.1, EKN3870380.1, WP_057631219.1, EKN5074149.1, WP_219650267.1, HDL6522072.1, EKN4058214.1, EKN4068486.1, HDL6961480.1, WP_075338710.1, EKN5112876.1, HDL8368473.1, HDL7423034.1, HDM8447363.1, EKN6363360.1, EKN3564743.1, HDL7684594.1, HDL7735658.1, HDL7339639.1, HDL7966642.1, CNH41000.1, HDL7909665.1, WP_219648645.1, WP_219643575.1, CQD54886.1, CNG18579.1, EKN4119565.1, WP_145592082.1, WP_038639531.1, AJJ34365.1, WP_050114520.1, WP_271298628.1, WP_004390824.1, WP_050095452.1, WP_145562021.1, WP_159678334.1, WP_309477958.1, WP_145510697.1, WP_219646505.1, WP_145556850.1, WP_145537515.1, WP_219655358.1, CNG40075.1, EKN3837153.1, EKN3315583.1, WP_301178346.1, HDL8028676.1, HDL8052082.1, HDL7585724.1, HDL8104119.1, WP_005164820.1, WP_050127938.1, WP_271306546.1, CBX71227.1, KGA73448.1, HDL8279144.1, HDL8024634.1, WP_115240285.1, WP_254461835.1, HDL8320198.1, WP_050124432.1, WP_050326508.1, HDL8083732.1, EKN3713082.1, HDL8750155.1, CNK45388.1, WP_221864008.1, SUP62773.1, or the like. For example, the first monomer may be expressed from a nucleic acid sequence encoding a protein having an amino acid sequence of SEQ ID NO: 7, a protein having an amino acid sequence of SEQ ID NO: 24, a protein having an amino acid sequence of SEQ ID NO: 1, a protein having an amino acid sequence of SEQ ID NO: 5, a protein having an amino acid sequence of SEQ ID NO: 6, a protein having an amino acid sequence of SEQ ID NO: 8, a protein having an amino acid sequence of SEQ ID NO: 9, a protein having an amino acid sequence of SEQ ID NO: 10, a protein having an amino acid sequence of SEQ ID NO: 11, a protein having an amino acid sequence of SEQ ID NO: 12, a protein having an amino acid sequence of SEQ ID NO: 13, a protein having an amino acid sequence of SEQ ID NO: 14, a protein having an amino acid sequence of SEQ ID NO: 15, a protein having an amino acid sequence of SEQ ID NO: 16, a protein having an amino acid sequence of SEQ ID NO: 17, a protein having an amino acid sequence of SEQ ID NO: 19, a protein having an amino acid sequence of SEQ ID NO: 20, a protein having an amino acid sequence of SEQ ID NO: 21, a protein having an amino acid sequence of SEQ ID NO: 22, a protein having an amino acid sequence of SEQ ID NO: 23, a protein having an amino acid sequence of SEQ ID NO: 30, a protein having an amino acid sequence of SEQ ID NO: 31, a protein having an amino acid sequence of SEQ ID NO: 32, a protein having an amino acid sequence of SEQ ID NO: 33, or a protein having an amino acid sequence of SEQ ID NO: 34. In addition, the nucleic acid sequence encoding the protein as described above may be a codon optimized for expression in a suitable host, such as *E. coli,* or chromosomal DNA encoding a wild-type first monomer extracted from a YaxAB nanopore producing organism, such as *Yersinia enterocolitic,* may be extracted from a nanopore producing organism, such as *Yersinia enterocolitic.* The extracted nucleic acid sequence or synthesized nucleic acid sequence may be amplified using PCR containing a specific primer, and the amplified sequence may be used to cause site-directed mutagenesis, thereby producing a mutant. A suitable method of the site-directed mutagenesis is known in the related art, and for example, a method using a polymerase chain reaction may be used. A variant of the nucleic acid sequence may be a nucleic acid sequence showing at least 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, or 99.5% or more homology with a nucleic acid encoding a protein having the amino acid sequence of SEQ ID NO: 7, SEQ ID NO: 24, or SEQ ID NO: 34, but is not limited thereto, and from this, any nucleic acid sequence may be included without limitation as long as it encodes a protein having the amino acid sequence of SEQ ID NO: 7, SEQ ID NO: 24, or SEQ ID NO: 34.

In addition, the first monomer may be expressed from a host cell into which a nucleic acid sequence encoding a protein is introduced, the protein having an amino acid sequence of any one of SEQ ID NO: 7, SEQ ID NO: 24, or SEQ ID NO: 34, or an amino acid sequence of NCBI accession NO. WP_005169901.1 (SEQ ID NO: 3), WP_011816273.1 (SEQ ID NO: 35), HDL8508204.1, HDL8476175.1, HDL7646675.1, PNM17258.1, WP_050336257.1, HDL8287337.1, HDL7822654.1, HDL7748605.1, HDL6737510.1, HEB4799346.1, WP_221871123.1, EKN3734298.1, EKN3569010.1, HDL7089514.1, WP_050160085.1, WP_221876226.1, EKN4747040.1, WP_050916836.1, HDM8435746.1, HDL8238855.1, WP_019079472.1, EKN3341278.1, WP_050163370.1, HDL7317648.1, HDM8081034.1, WP_050870897.1, EKN3737775.1, HDL7467110.1, HDL7471348.1, WP_046694975.1, WP_301211860.1, HDL7726911.1, WP_268215747.1, EKN5089609.1, WP_057620566.1, EKN6360258.1, WP_019083154.1, EKN5159302.1, WP_050131624.1, ELI8015017.1, EKN4802793.1, EKN6165952.1, ELI8293390.1, ELI8097018.1, WP_050157801.1, EKN4708697.1, EKN5102380.1, EKN5913011.1, WP_050164534.1, WP_242365000.1, HDL6759266.1, WP_050327956.1, EKN3969341.1, HDL8127360.1, EKN3724092.1, WP_050334096.1, HDL7761807.1, HDL6872781.1, EKN3573207.1, EKN3562735.1, EKN5949803.1, HDL6624424.1, WP_219649234.1, EKN3957447.1, EKN3611203.1, EKN5162868.1, WP_172654801.1, HDL6886351.1, WP_263797395.1, EKN3989601.1, HDL7855057.1, HDW8038128.1, EKN3870380.1, WP_057631219.1, EKN5074149.1, WP_219650267.1, HDL6522072.1, EKN4058214.1, EKN4068486.1, HDL6961480.1, WP_075338710.1, EKN5112876.1, HDL8368473.1, HDL7423034.1, HDM8447363.1, EKN6363360.1, EKN3564743.1, HDL7684594.1, HDL7735658.1, HDL7339639.1, HDL7966642.1, CNH41000.1, HDL7909665.1, WP_219648645.1, WP_219643575.1, CQD54886.1, CNG18579.1, EKN4119565.1, WP_145592082.1, WP_038639531.1, AJJ34365.1, WP_050114520.1, WP_271298628.1, WP_004390824.1, WP_050095452.1, WP_145562021.1, WP_159678334.1, WP_309477958.1, WP_145510697.1, WP_219646505.1, WP_145556850.1, WP_145537515.1, WP_219655358.1, CNG40075.1, EKN3837153.1, EKN3315583.1, WP_301178346.1, HDL8028676.1, HDL8052082.1, HDL7585724.1, HDL8104119.1, WP_005164820.1, WP_050127938.1, WP_271306546.1, CBX71227.1, KGA73448.1, HDL8279144.1, HDL8024634.1, WP_115240285.1, WP_254461835.1, HDL8320198.1, WP_050124432.1, WP_050326508.1, HDL8083732.1, EKN3713082.1, HDL8750155.1, CNK45388.1, WP_221864008.1, SUP62773.1, or the like. For example, the first monomer may be expressed from a vector having a nucleic acid sequence encoding a protein having the amino acid sequence of SEQ ID NO: 7, SEQ ID NO: 24, or SEQ ID NO: 34 and a host cell containing the vector. The vector may be obtained by incorporating a nucleic acid sequence encoding a protein having the amino acid sequence of SEQ ID NO: 7, SEQ ID NO: 24, or SEQ ID NO: 34 into a recombinant vector, such as a cloning vector or an expression vector. A nucleic acid sequence encoding a protein having the amino acid sequence of SEQ ID NO: 7, SEQ ID NO: 24, or SEQ ID NO: 34 may be operably linked to a promoter contained in the vector. The vector may be used to replicate and express the nucleic acid sequence in a host cell. Therefore, the first monomer may be obtained by introducing the nucleic acid sequence into a recombinant vector, introducing the vector into a compatible host cell, growing the host cell under conditions which induce replication of the vector, and recovering the vector from the host cell. As a host cell suitable for cloning or expressing the nucleic acid sequence, any host cell known in the related art may be used.

Meanwhile, the second monomer may be expressed from a nucleic acid sequence or a variant thereof encoding a protein having an amino acid sequence of SEQ ID NO: 18 or SEQ ID NO: 25, or an amino acid sequence of NCBI accession NO. WP_011816274.1 (SEQ ID NO: 4), WP_050336255.1 (SEQ ID NO: 36), WP_005169898.1, PNM17259.1, WP_050137918.1, HDL7748604.1, HDL6508072.1, WP_050137583.1, WP_050322893.1, HDL7822655.1, HDL7646676.1, WP_050321988.1, WP_221871120.1, HDL6961479.1, WP_050298175.1, CNG06681.1, EKN5919601.1, EKN5074150.1, WP_075338711.1, HDL7057906.1, WP_219656641.1, WP_050157802.1, ELI8015018.1, EKN4068485.1, HDL7533782.1, WP_057631218.1, WP_050916837.1, EKN6259816.1, EKN3396085.1, HDL7423033.1, EKN4822249.1, WP_050880005.1, WP_050875182.1, HDL7335482.1, WP_050163371.1, EKN3734297.1, HDL6886350.1, HEB4799167.1, WP_263700684.1, EKN3341279.1, HDL7478311.1, EKN3957448.1, WP_050327958.1, HDL7339638.1, HDL6946689.1, WP_019079471.1, HDV7159892.1, HDL7467111.1, WP_172654802.1, WP_050870895.1, EKN3469507.1, WP_019083153.1, WP_076706633.1, HDW8038129.1, CQG99659.1, WP_050945964.1, HDL6872782.1, WP_046694976.1, WP_263699488.1, WP_263697703.1, EKN3724091.1, HDL7089513.1, CQH55963.1, HDL7666636.1, EKN5102379.1, WP_046050899.1, HDL6959493.1, EKN5159301.1, HDL8096476.1, EKN3683754.1, EKN3973834.1, WP_219650266.1, EKN6365751.1, HDL8258876.1, EKN4119566.1, HDM8089399.1, WP_263696663.1, EKN6178815.1, ELI8097017.1, HDL7909664.1, WP_219643580.1, WP_050149278.1, CNH43347.1, HDL8000813.1, WP_020283191.1, HDL8788876.1, HDL7394360.1, HDL7984656.1, WP_005164817.1, HDL8028561.1, CBY27149.1, HDL8032249.1, HDL8024436.1, HDL8004719.1, HDL6519422.1, HDL8048091.1, WP_016266185.1, CCV61752.1, WP_049526748.1, WP_145535945.1, WP_050114521.1, WP_309477957.1, WP_289816262.1, WP_050291733.1, WP_145589360.1, WP_038633022.1, WP_265526189.1, WP_145537514.1, WP_050095454.1, WP_057634251.1, WP_050289682.1, WP_145531133.1, WP_145586506.1, WP_100286507.1, WP_219655343.1, WP_049557371.1, WP_087795913.1, WP_145510696.1, WP_271304184.1, WP_004390823.1, WP_145556851.1, WP_050120114.1, WP_219648204.1, WP_159678331.1, or the like. The second monomer may be expressed from a nucleic acid sequence encoding a protein having an amino acid sequence of SEQ ID NO: 18, a protein having an amino acid sequence of SEQ ID NO: 25, a protein having an amino acid sequence of SEQ ID NO: 2, a protein having an amino acid sequence of SEQ ID NO: 26, a protein having an amino acid sequence of SEQ ID NO: 27, a protein having an amino acid sequence of SEQ ID NO: 28, or a protein having an amino acid sequence of SEQ ID NO: 29. In addition, the nucleic acid sequence encoding the protein as described above may be a codon optimized for expression in a suitable host, such as *E. coli*, or chromosomal DNA encoding a wild-type second monomer extracted from a YaxAB nanopore producing organism, such as *Yersinia enterocolitic*, may be extracted from a nanopore producing organism, such as *Yersinia enterocolitic*. The extracted nucleic acid sequence or synthesized nucleic acid sequence may be amplified using PCR containing a specific primer, and the amplified sequence may be used to cause site-directed mutagenesis, thereby producing a mutant. A suitable method of the site-directed mutagenesis is known in the related art, and for example, a method using a polymerase chain reaction may be used. A variant of the nucleic acid sequence may be a nucleic acid sequence showing at least 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, or 99.5% or more homology with a nucleic acid encoding a protein having the amino acid sequence of SEQ ID NO: 18 or SEQ ID NO: 25, but is not limited thereto, and from this, any nucleic acid sequence may be included without limitation as long as it encodes a protein having the amino acid sequence of SEQ ID NO: 18 or SEQ ID NO: 25. In addition, the second monomer may be expressed using a vector and a host cell containing the vector as described above in the description of the first monomer.

In addition, the second monomer may be expressed from a host cell into which a nucleic acid sequence encoding a protein is introduced, the protein having an amino acid sequence of SEQ ID NO: 18 or SEQ ID NO: 25, or an amino acid sequence of NCBI accession NO. WP_011816274.1 (SEQ ID NO: 4), WP_050336255.1 (SEQ ID NO: 36), WP_005169898.1, PNM17259.1, WP_050137918.1, HDL7748604.1, HDL6508072.1, WP_050137583.1, WP_050322893.1, HDL7822655.1, HDL7646676.1, WP_050321988.1, WP_221871120.1, HDL6961479.1, WP_050298175.1, CNG06681.1, EKN5919601.1, EKN5074150.1, WP_075338711.1, HDL7057906.1, WP_219656641.1, WP_050157802.1, ELI8015018.1, EKN4068485.1, HDL7533782.1, WP_057631218.1, WP_050916837.1, EKN6259816.1, EKN3396085.1, HDL7423033.1, EKN4822249.1, WP_050880005.1, WP_050875182.1, HDL7335482.1, WP_050163371.1, EKN3734297.1, HDL6886350.1, HEB4799167.1, WP_263700684.1, EKN3341279.1, HDL7478311.1, EKN3957448.1, WP_050327958.1, HDL7339638.1, HDL6946689.1, WP_019079471.1, HDV7159892.1, HDL7467111.1, WP_172654802.1, WP_050870895.1, EKN3469507.1, WP_019083153.1, WP_076706633.1, HDW8038129.1, CQG99659.1, WP_050945964.1, HDL6872782.1, WP_046694976.1, WP_263699488.1, WP_263697703.1, EKN3724091.1, HDL7089513.1, CQH55963.1, HDL7666636.1, EKN5102379.1, WP_046050899.1, HDL6959493.1, EKN5159301.1, HDL8096476.1, EKN3683754.1, EKN3973834.1, WP_219650266.1, EKN6365751.1, HDL8258876.1, EKN4119566.1, HDM8089399.1, WP_263696663.1, EKN6178815.1, ELI8097017.1, HDL7909664.1, WP_219643580.1, WP_050149278.1, CNH43347.1, HDL8000813.1, WP_020283191.1, HDL8788876.1, HDL7394360.1, HDL7984656.1, WP_005164817.1, HDL8028561.1, CBY27149.1, HDL8032249.1, HDL8024436.1, HDL8004719.1, HDL6519422.1, HDL8048091.1, WP_016266185.1, CCV61752.1, WP_049526748.1, WP_145535945.1, WP_050114521.1, WP_309477957.1, WP_289816262.1, WP_050291733.1, WP_145589360.1, WP_038633022.1, WP_265526189.1, WP_145537514.1, WP_050095454.1, WP_057634251.1, WP_050289682.1, WP_145531133.1, WP_145586506.1, WP_100286507.1, WP_219655343.1, WP_049557371.1, WP_087795913.1, WP_145510696.1, WP_271304184.1, WP_004390823.1, WP_145556851.1, WP_050120114.1, WP_219648204.1, WP_159678331.1, or the like. For example, the first monomer may be expressed from a vector having a nucleic acid sequence encoding a protein having the amino acid sequence of SEQ ID NO: 18 or SEQ ID NO: 25 and a host cell containing the vector. The vector may be obtained by incorporating a nucleic acid sequence encoding a protein having the amino acid sequence of SEQ ID NO: 18 or SEQ ID NO: 25 into a recombinant vector, such as a cloning vector or an expression vector. A nucleic acid sequence encoding a protein having the amino acid sequence of SEQ ID NO: 18 or SEQ ID NO: 25 may be operably linked to a promoter contained in the vector. The vector may be used to replicate and express the nucleic acid sequence in a host cell. Therefore, the second monomer may be obtained by introducing the nucleic acid sequence into a recombinant vector, introducing the vector into a compatible host cell, growing the host cell under conditions which induce replication of the vector, and recovering the vector from the host cell. As a host cell suitable for cloning or expressing the nucleic acid sequence, any host cell known in the related art may be used.

Meanwhile, the first monomer and the second monomer may be bound to each other to form a heterodimer. The heterodimer is a YaxAB nanopore subunit, and the subunits may assemble to form a YaxAB nanopore. Therefore, the subunit is a basic unit (protomer) constituting the YaxAB nanopore of the present invention.

In addition, the first monomer and the second monomer may improve the nanopore forming ability by adjusting an amino acid sequence. The term "nanopore forming ability" as used in the present invention means oligomerization of the subunits, in which the subunits containing the first monomer and the second monomer are repeatedly connected to each other to form pores with a nanometer (nm, $10^{-9}$) size. Therefore, an excellent nanopore forming ability means that the subunits stably form pores or that the subunits may form nanopores even when present at a low concentration.

In a specific embodiment of the present invention, in a wild-type YaxAB nanopore of the present invention, no oligomer band appears under low protein concentration conditions, whereas in a modified YaxAB_dN nanopore composed of YaxAB subunits containing a first monomer of SEQ ID NO: 1 (YaxA_(45-410)) and a second monomer of SEQ ID NO: 2 (YaxB_(12-343)), oligomer bands clearly appeared even under low protein concentration conditions; thus, it was confirmed that the nanopore was formed in a more stable state than the wild-type YaxAB (see FIG. 30).

In addition, in a specific embodiment of the present invention, as a result of producing various truncated sequences and analyzing the oligomerization tendency in order to confirm the minimum amino acid sequence capable of forming the YaxAB nanopore of the present invention, in the case of a nanopore subunit containing a first monomer having an amino acid sequence consisting of SEQ ID NO: 7 or SEQ ID NO: 24 and a second monomer having an amino acid sequence consisting of SEQ ID NO: 18 or SEQ ID NO: 25, YaxAB oligomers were stably formed, and the nanopore-containing membrane of the present invention was successfully implemented by the oligomers; thus, it was confirmed that an analyte protein (Bcl-xL) was successfully captured using the nanopore system (see FIGS. 35 and 36).

In another specific embodiment of the present invention, it was confirmed that in the case of a nanopore subunit containing a first monomer containing at least one of the amino acid sequences consisting of SEQ ID NO: 5, SEQ ID NO: 9, SEQ ID NO: 10, SEQ ID NO: 11, SEQ ID NO: 12, SEQ ID NO: 32, SEQ ID NO: 33, or SEQ ID NO: 34 and a second monomer having the amino acid sequence consisting of SEQ ID NO: 2, YaxAB oligomers were stably formed (see FIG. 32).

The YaxAB nanopore of the present invention may be formed by assembling 2 or more, for example, 3 or more, 5 or more, or 7 or more subunits, and specifically, may be formed by assembling 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more subunits, and specifically, 4 to 20 subunits. Depending on the number of subunits, the diameter of the lumen of the YaxAB nanopore may vary, and specifically, as the number of subunits increases, the diameter of the lumen of the YaxAB nanopore of the present invention may increase.

In the YaxAB nanopore of the present invention, the number of subunits constituting the nanopore may be easily adjusted by adjusting the length of the amino acid sequence of each of the first monomer and the second monomer. In other words, as the amino acid sequences of the first monomer and the second monomer change, the number of subunits forming the nanopore may also change. In a specific embodiment of the present invention, it was confirmed that there was a difference in the types of gradient blue native polyacrylamide gel electrophoresis bands that appeared when a nanopore was formed with a wild-type YaxAB subunit and a modified YaxAB_dN subunit, confirming that the number of subunits constituting the nanopore may be adjusted by changing the amino acid sequences of the subunits (see FIG. 30). Through this, a novel YaxAB-C$_8$ pore that was unable to be formed in wild-type YaxAB was formed.

In addition, in a specific embodiment of the present invention, as a result of analyzing gradient blue native polyacrylamide gel electrophoresis that appeared when a nanopore was formed using the modified Yax-

AB_dN subunit, it was confirmed that, although the bands corresponding to YaxAB-C$_8$, YaxAB-C$_9$, and YaxAB-C$_{10}$ (C$_8$, C$_9$, and C$_{10}$ bands) mainly appeared, the bands located above the C$_{10}$ band (C$_{11}$, C$_{12}$, C$_{13}$, C$_{14}$, and C$_{15}$ bands) also appeared in a well separated state, confirming that the number of subunits constituting the nanopore may be adjusted by changing the amino acid sequences of the subunits (see FIG. 8). Through this, novel nanopores with various sizes that were unable to be formed with the wild-type YaxAB sequence were formed. Therefore, the modified YaxAB_dN nanopore of the present invention not only ensures nanopores with various sizes by one-time purification, but also selectively ensures nanopores with a desired size at the same time, and thus has a significant advantage over a conventional biological nanopore having a fixed pore size.

In addition, the purification efficiency of the first monomer and the second monomer may be increased by adjusting the lengths of the amino acid sequences of the first monomer and the second monomer. In particular, in a case in which the first monomer consists of the amino acid sequence of SEQ ID NO: 1, the protein purification efficiency may be 2 times or more, 3 times or more, or 4 times or more greater than that of the wild-type YaxA protein. In addition, in a case in which the second monomer consists of the amino acid sequence of SEQ ID NO: 2, the protein purification efficiency may be 1.1 times or more greater. The protein purification process may be performed by a known conventional protein purification method. When the purification efficiency of each of the monomers is high, a nanopore may be formed in a more stable state than the wild-type YaxAB subunit.

In a specific embodiment of the present invention, the first monomer having the amino acid sequence of SEQ ID NO: 1 of the present invention was confirmed to have a purified protein yield that is 4.7 times greater than that of wild-type YaxA, and the second monomer having the amino acid sequence of SEQ ID NO: 2 was confirmed to have a purified protein yield that is 1.1 times greater than that of wild-type YaxB, thereby confirming superior purification efficiency under the same protein purification conditions (see FIG. 29).

## 2. Nanopore-containing membrane

In addition, another aspect of the present invention provides a nanopore-containing membrane including a membrane layer; and a YaxAB nanopore inserted into the membrane layer.

The membrane may be included without limitation as long as it serves as a support to which the nanopore may be fixed and may divide a space or chamber in which the nanopore exists into two compartments. The membrane serves to block a fluid or a substance contained in the fluid from passing through without additional means. The membrane layer may include a phospholipid and an amphiphile.

The phospholipid is an amphipathic molecule containing at least one phosphorus group and serves as a support constituting the membrane. A phospholipid membrane refers to a structure in which a hydrophobic head of the phospholipid points in one direction and a hydrophilic tail points in the opposite direction. The phospholipid may be 1,2-diphytanoyl-sn-glycero-3-phosphocholine (DPhPC), 1,2-dipalmitoyl-sn-glycero-3-phosphocholine (DPPC), 1,2-dipalmitoyl-sn-glycero-3-phosphorylethanolamine (DPPE), 1,2-dipalmitoyl-sn-glycero-3-phosphorylglycerol (DPPG), 1,2-dipalmitoyl-sn-glycero-3-phospho-L-serine (DPPS), 1,2-dihexanoyl-sn-glycero-3-phosphocholine (DHPC), 1,2-dilauroyl-sn-glycero-3-phosphocholine (DLPC), 1,2-dimyristoyl-sn-glycero-3-phosphocholine (DMPC), 1,2-dimyristoyl-sn-glycero-3-phosphorylethanolamine (DMPE), 1,2-dimyristoyl-sn-glycero-3-phosphorylglycerol (DMPG), 1,2-dimyristoyl-sn-glycero-3-phospho-L-serine (DMPS), 1,2-dioleoyl-sn-glycero-3-phospho-L-serine (DOPS), 1,2-dioleoyl-sn-glycero-3-phosphoethanolamine (DOPE), 1,2-dioleoyl-sn-glycero-3-phosphoglycerol (DOPG), 1,2-distearoyl-sn-glycero-3-phosphocholine (DSPC), 1-palmitoyl-2-oleoyl-SN-glycero-3-phosphocholine (POPC), mycolic acid, and the like.

The amphiphile is a substance having both watersoluble and fat-soluble properties, and refers to a substance that stabilizes the membrane by maintaining the fluidity of the membrane containing the phospholipid at a constant level. When the amphiphile is included, the stability of the membrane may be increased, the number of nanopores formed may be affected, and the nanopore life may be adjusted by changing a nanopore disjoining force by also affecting the elasticity of the membrane.

The amphiphile may be, for example, campesterol, sitosterol, stigmasterol, cholesterol, ergosterol, cardiolipin, sphingomyelin, 1-palmitoyl-2-cholesterylhemisuccinoyl-sn-glycero-3-phosphocholine (PChemsPC), 1-oleoyl-2-cholesterylhemisuccinoyl-sn-glycero-3-phosphocholine (OChemsPC), 1-palmitoyl-2-cholesterylcarbonoyl-sn-glycero-3-phosphocholine (PChcPC), 1,2-dicholesterylhemisuccinoyl-sn-glycero-3-phosphocholine (DChemsPC), 10,12-pentacosadiynoic acid (PCDA), 10,12-tricosadiynoic acid (TCDA), 5,7-hexadecadiynoic acid (HDDA), 9,12-octadecadiynoic acid (ODDA), and the like, but is not limited thereto, and any amphiphile that increases the stability of the membrane may be included without limitation.

The membrane may include the phospholipid and the amphiphile in ratios of 50 to 95 mol% and 5 to 50 mol%, in ratios of 55 to 95 mol% and 5 to 45 mol%, or in ratios of 60 to 95 mol% and 5 to 40 mol%, respectively. In the above ratio ranges, as the ratio of the amphiphile included in the membrane increases, the elasticity of the lipid membrane may decrease, and accordingly, the disjoining force for forming nanopores may decrease, thereby increasing the life of the nanopores and thus increasing the formation frequency of the nanopores. When the phospholipid and the amphiphile in the membrane are out of the above ratios, the YaxAB nanopore of the present invention may not be stably inserted into the membrane, and the membrane may rupture, or when a voltage is applied, a constant electrical conductivity may not be measured, and an ionic current according to Ohm's law may not flow.

In the present invention, the YaxAB nanopore is as described above in **"YaxAB nanopore subunit and YaxAB nanopore containing the same"** section, and therefore, the above description is cited and not described repeatedly.

The YaxAB nanopore may be inserted into the membrane by a known method. For example, the nanopore may be inserted into the membrane through changes in ionic current. In the nanopore-containing membrane, the stability of the YaxAB nanopore of the present invention is improved. The YaxAB nanopore of the present invention may be efficiently inserted without rupturing the membrane, such that the frequency of nanopore formation may be high and the number of stably inserted nanopores may be increased. In addition, in a case in which the nanopore-containing membrane is used, a stable electrical conductivity value and current flow according to Ohm's law may be exhibited through the YaxAB nanopore even when a voltage is applied.

In a specific embodiment of the present invention, it was confirmed that the YaxAB nanopore of the present invention was stably inserted into a DPhPC-sterol composite membrane compared to an artificial membrane composed of only DPhPC (see FIG. 6).

## 3. Nanopore system

In addition, still another aspect of the present invention provides a nanopore system using the YaxAB nanopore.

The nanopore system of the present invention includes a chamber; and a YaxAB nanopore-containing membrane.

The chamber refers to a structure having an internal space formed that may accommodate a certain volume of fluid. The space within the chamber is a fluid-filled compartment, and the fluid may be an electrolyte solution. In addition, the chamber may include an electrode capable of generating an electrical potential difference across the membrane to facilitate electrolyte flow through the nanopores between the chambers. The internal space of the chamber is divided into two compartments, for example, first and second compartments or cis and trans compartments, by the nanopore-containing membrane. In a case in which an analyte is added to the compartment where the first opening faces, the first opening of the YaxAB nanopore is a cis opening and the second opening is a trans opening.

The YaxAB nanopore and the nanopore-containing membrane are based on what have been described above and will not be described repeatedly.

The term "nanopore system" as used in the present invention refers to a novel system that may detect or analyze changes in physicochemical characteristics, including changes in fine structure and dynamics of biomolecules at a single molecular level, and may be used as a sensor system or the like. In the nanopore system, a nanopore is typically inserted into an insulating membrane and serves as only a conduit for allowing an ionic current to flow between two compartments divided by the insulating membrane. The nanopore system operates on the principle that the length, size, structure, dynamics, charge, and shape of biomolecules are determined by detecting a minute fluctuation in the ionic current when charged biomolecules are translocated through the nanopore or are trapped in the lumen of the nanopore in the presence of an external electric field.

The nanopore system may include a liquid medium, and the liquid medium may be an aqueous solution containing a salt. The salt may cause an ionic current to flow as it is translocated through the nanopore between the two compartments divided by the insulating membrane. In a case in which the salt is an ion, the salt may be a metal salt, a halide salt such as an alkali metal chloride salt, or the like. Specifically, the salt may be a cation such as $NH^{4+}$, $K^+$, $Na^+$, $Li^+$, $Mg^{2+}$, $Ca^{2+}$, or $Gdm^+$ and an anion such as $F^-$, $SO_4^{2-}$, $HPO_4^{2-}$, $C_2H_3O^{2-}$, $Cl^-$, $Br^-$, $NO^{3-}$, $ClO^{3-}$, $I^-$, $ClO^{4-}$, or $SCN^-$, and may be, for example, sodium chloride (NaCl), potassium chloride (KCl), lithium chloride (LiCl), calcium chloride ($CaCl_2$), cesium chloride (CsCl), guanidinium chloride (GdmCl), potassium ferrocyanide, or potassium ferricyanide. The salt may be an organic salt, for example, tetramethyl ammonium chloride, trimethylphenyl ammonium chloride, phenyltrimethyl ammonium chloride, or 1-ethyl-3-methyl imidazolium chloride. However, the salt is not limited thereto, and any salt having a charge may be included without limitation.

In addition, a concentration of the salt may be 10 mM to 5 M, 20 mM to 4.8 M, 30 mM to 4.5 M, 40 mM to 4.2 M, or 50 mM to 4 M. In addition, the salts present in the two compartments may be present in asymmetric concentrations.

In addition, the two compartments within the nanopore system may include an electrode. By applying one or more electrical potentials to the electrode, a flow of ions may be caused from one compartment to the other compartment across the nanopore-containing membrane.

The nanopore system of the present invention may operate by inducing electroosmotic force or electrophoretic force to allow an analyte to be translocated through or be captured through the pores in a state where a gradient is formed according to a difference in salt concentration in two compartments divided by the nanopore-containing membrane, a voltage is applied, or both. The nanopore system may operate by inducing either electroosmosis or electrophoresis, may operate by inducing both electrophoresis and electroosmosis, or may operate by a balance between electrophoresis and electroosmosis.

The term "electroosmosis" as used in the present invention refers to a phenomenon in which, when electrodes are attached to both sides of a porous membrane such as the nanopore-containing membrane and a direct current voltage is applied, a liquid moves toward one pole through the nanopores included in the membrane.

The term "electrophoresis" as used in the present invention refers to a phenomenon in which molecules move according to their charge and size in an electric field.

In the nanopore system, when a working electrode is positioned in the compartment having the cis opening of the YaxAB nanopore, a potential difference (voltage) between the two compartments may be -200 mV, -180 mV, -160 mV, -140 mV, - 120 mV, -100 mV, -90 mV, -85 mV, -80 mV, -75 mV, -70 mV, -65 mV, -60 mV, -55 mV, -50 mV, -45 mV, -40 mV, -35 mV, -30 mV, - 25 mV, -20 mV, -15 mV, -10 mV, -5 mV, 0 mV, +5 mV, +10 mV, +15 mV, +20 mV, +25 mV, +30 mV, +35 mV, +40 mV, +45 mV, +50 mV, +55 mV, +60 mV, +90 mV, +100 mV, +120 mV, +130 mV, +140 mV, +200 mV, +300 mV, or +400 mV to 500 mV. Conversely, when the working electrode is positioned in the compartment having the trans opening of the YaxAB nanopore, a potential difference (voltage) between the two compartments may be +200 mV, +180 mV, +160 mV, +140 mV, +120 mV, +100 mV, +90 mV, +85 mV, +80 mV, +75 mV, +70 mV, +65 mV, +60 mV, +55 mV, +50 mV, +45 mV, +40 mV, +35 mV, +30 mV, +25 mV, +20 mV, +15 mV, +10 mV, +5 mV, 0 mV, -5 mV, -10 mV, -15 mV, -20 mV, -25 mV, -30 mV, -35 mV, -40 mV, -45 mV, -50 mV, -55 mV, -60 mV, -90 mV, -100 mV, -120 mV, -130 mV, -140 mV, -200 mV, -300 mV, or -400 mV to -500 mV. The applied potential refers to a potential of the working electrode, and when a positive applied potential is applied to the compartment where the cis opening of the YaxAB nanopore is located, a base current in an open state that flows stably may be observed. That is, when a voltage in a range of -100 mV to +200 mV or -200 mV to +500 mV is applied to the YaxAB nanopore in a case in which the working electrode is located at the cis opening of the pore in the two compartments divided by the nanopore-containing membrane in the nanopore system, or when a voltage in a range of +100 to -200 mV or +200 mV to -500 mV is applied to the YaxAB nanopore in a case in which the working electrode is located at the trans opening of the pore, the YaxAB nanopore is stably maintained in an open state, which allows charged ions to move across the nanopore between the two compartments so as to cause an ionic current to flow.

The analyte is operated to be translocated through the nanopore of the nanopore system of the present invention, be trapped into the lumen, or allow the nanopore to be gated. For example, when a voltage is applied to the nanopore system to form an electric field, the analyte may be translocated, be captured, or be gated. In this case, the flow of the ionic current flowing through the nanopore is blocked by the analyte, and the resulting minute fluctuations are represented as current signals, which may be used to detect the analyte or analyze its characteristics. The characteristics of the analyte may be electrical characteristics, chemical characteristics, or physical characteristics of the analyte, and specifically, may be at least one selected from the group consisting of a surface charge of the analyte, an isoelectric point of the analyte, stability of the analyte, an isomer, a length, a size, a molecular weight, a structure, dynamics, strength and orientation of a dipole moment, orientation, flexibility, conformational heterogeneity, a shape, and an amount of the analyte, and changes thereof.

Since the YaxAB nanopore has a wide funnel shape at the inlet (first opening), analytes with various sizes and molecular weights may be detected. The analyte may be an inorganic particle containing a peptide, a protein, a lipid, a carbohydrate, an inorganic substance, a small molecule compound, a nucleic acid, or a nanomaterial, and for example, the analyte may be a receptor, an antibody, or an aptamer. When the analyte is a protein, a molecular weight of the analyte may be 1 to 1,200 kDa, 1 to 1,000 kDa, or 1 to 800 kDa. In a specific embodiment of the present invention, it was confirmed that a protein having a molecular weight of 1 to 670 kDa was detected by the nanopore system of the present invention (see FIGS. 10, 18, and 19). The nanopore system may capture an analyte into the lumen of the nanopore regardless of the surface charge of the analyte because the lumen of the YaxAB nanopore has an electronegative surface charge, and such a characteristic may induce an electroosmotic force greater than an electrophoretic force.

In addition, since the YaxAB nanopore has a funnel shape, an ionic current density increases as it approaches the constriction where a diameter becomes narrower in the lumen of the nanopore, and therefore, an ionic current blockade by the analyte may be maximized, and at the same time, as the electroosmosis force and the electrophoresis force are balanced, the analyte may remain in the nanopore lumen while exhibiting its own movement; thus, a higher resolution signal than that in a conventional nanopore system may appear.

In a specific embodiment of the present invention, when a positive voltage was applied to the nanopore system of the present invention through molecular dynamics simulation, it was confirmed that the cation and water molecules predominantly flowed from the first opening of the YaxAB nanopore toward the second opening, confirming that electroosmosis was induced (see FIG. 4). In addition, the YaxAB nanopore may detect proteins with various surface charges of isoelectric points of 2 to 12, 3 to 11, and 4 to 10 by utilizing the electroosmosis force as well as the electrophoresis force. In a specific embodiment of the present invention, it was confirmed that proteins having a wide range of surface charges (neutral, negative, and positive) with an isoelectric point of 4.1 to 9.6 were also captured into the lumen, confirming that detection was possible regardless of the overall charge of the proteins (see FIGS. 10, 18, and 19).

In another specific embodiment of the present invention, the nanopore system of the present invention was observed to exhibit a greater change in ionic current blockade than that caused by a conventional nanopore system and to exhibit a distinctly different electrical signal pattern depending on the type of analyte, confirming that the nanopore system of the present invention had excellent resolution (see FIGS. 11 and 14).

In addition, in another specific embodiment of the present invention, as illustrated in FIG. 10, it was confirmed that a detection range was 1 to 77 kDa in the YaxAB-$C_8$ nanopore system of the present invention; a detection range was 158 kDa or less in the YaxAB-$C_{11}$ system; and a detection range was 669 kDa or less in the YaxAB-$C_{12}$ system, confirming that the nanopore system had a significantly wider analyte detection range than the detectable analyte ranges of the conventional nanopore systems, which are 25 kDa or less (FraC nanopore); 25 to 42 kDa (ClyA nanopore), and 64.5 kDa or less (PlyAB nanopore). That is, the YaxAB nanopore system of the present invention not only produces pores with various sizes by one-time purification process, but also allows for adjustment of the pore size. Therefore, it was confirmed that the YaxAB nanopore system of the present invention had an excellent analyte detection range compared to the conventional nanopores.

## 4. Utilization of nanopore system

In addition, still another aspect of the present invention provides a single molecule analysis method for a single analyte or a plurality of analytes, the single molecule analysis method including placing at least one analyte in one compartment of the nanopore system; and measuring changes in electrical signal in the two compartments before and after the placing of the analyte.

The analyte may be an inorganic particle containing a peptide, a protein, a lipid, a carbohydrate, an inorganic substance, a small molecule compound, a nucleic acid, or a nanomaterial, and for example, the analyte may be a receptor, an antibody, or an aptamer. More specifically, the analyte may be a biomolecule, and specifically, may be a single compound such as a nucleic acid, an amino acid, or a monosaccharide, may be a polymer such as a polynucleotide, a peptide, a protein, a polysaccharide, a carbohydrate, or a lipid, or may be a molecule such as a natural or synthetic organic substance, an inorganic substance, or a complex thereof. For example, the nucleic acid may be single-stranded DNA (ssDNA), double-stranded DNA (dsDNA), single-stranded RNA (ssRNA), double-stranded RNA (dsRNA), a double-stranded DNA/RNA hybrid, or a combination thereof, and the protein may be a linear protein whose structure is denatured using a surfactant such as SDS and a peptide, but is not limited thereto.

The single molecule analysis method for a single analyte or a plurality of analytes includes placing at least one analyte in one compartment divided by the nanopore-containing membrane, which may be, for example, placing the analyte in one of the compartments, particularly in one compartment where the first opening of the nanopore faces. In addition, the treatment of the analyte may be performed in a situation where the analyte is induced to move to another compartment through the nanopore, for example, in a situation where a gradient is formed according to a difference in salt concentration in the two compartments divided by the membrane, a voltage is applied, or both of these are provided.

In addition, the treatment of the analyte includes measuring changes in electrical signals in the two compartments before and after the placing of the analyte.

The "electrical signal" means a signal generated when ions and/or charged small molecules pass from one compartment divided by the membrane to another compartment, for example, from a first compartment to a second compartment, and specifically, may be, but is not limited to, a current pattern of an ionic current, a base current in an open state (open pore current: $I_o$, $I_{open}$), a magnitude of a current drop ($\Delta I$), a current blockade, an event duration, a frequency per unit time of a nanopore signal pattern, a current noise ($I_N$), and the like. The frequency per unit time of the nanopore signal pattern may include, but is not limited to, an event frequency and an inter-event interval. The base current in the open state may mean a current in a state where only a nanopore exists. The magnitude of the current drop ($\Delta I$) may mean a magnitude of a decrease in the amount of passage of an electrolyte, such as an ion and/or a charged small molecule substance, due to the analyte being trapped inside the nanopore or the analyte being translocated through the nanopore. The current blockade may be a ratio ($\Delta I/I_o$) of $\Delta I$, which is the magnitude of the current drop due to the analyte, to $I_o$, which is the base current when the nanopore is in the open state (open pore current). The event duration may be the time that the event lasts when the analyte event is manifested by the magnitude of the current drop ($\Delta I$). The current noise may include a power spectral density (PSD) or an ionic current standard deviation (SD), and the power spectral density is a representation of noise power generated by the flow of the ionic current on a frequency spectrum, which represents a distribution of power density according to the signal frequency. The standard deviation represents a standard deviation value of the current blockade signal itself that is generated as the ionic current drops. In addition, the current noise ($I_N$) represents a square root of an integral value of the power spectrum of the ionic current or a standard deviation value of the ionic current when the current drops, which may mean the quantification of the electrical noise characteristics of the nanopore, and may include the current noise amplified by amplifying a difference in the current noise through signal filtering in a low-oscillation section. In addition, the electrical signal may include a density contour plot, a two-dimensional scatter plot, or a violin plot that two-dimensionally represents the current blockade ($\Delta I/I_0$) and the current noise ($I_N$). The analysis method for a single analyte or a plurality of analytes includes qualitative and quantitative analysis of the analyte. For example, the analysis method may be an analysis of the presence or absence of an analyte, the type of analyte, a structure of an analyte, dynamics and its transformation, and the amount of analyte.

In an embodiment of the present invention, in a case in which the nanopore system was used, it was confirmed that analysis of a single molecule analyte was possible as distinctly different ionic current change patterns between the analytes with various sizes and various surface charges appeared (see FIG. 10).

In another embodiment of the present invention, as the nanopore system was used to translocate EGF, which is a single molecule compound that is small in size and negatively charged at pH 7.5, or p53TAD1 peptide, which is an intrinsically disordered protein (IDP) having a small molecular weight of 1.8 kDa, and p53TAD protein, which is an intrinsically disordered protein having a molecular weight of 8.3 kDa, using the electrophoresis force, it was confirmed that detection and analysis of the intrinsically disordered protein and the peptide analytes were possible through the nanopore system (see FIGS. 26 and 27).

In still another embodiment of the present invention, as a result of independently adding Lambda DNA, single-stranded nucleic acid (50 mer), and double-stranded nucleic acid (23 bp) to the nanopore system and observing whether or not the nucleic acids were translocated, the ionic current blockade was confirmed before and after the addition of each nucleic acid; thus, it was confirmed that nucleic acids may be detected and analyzed through the nanopore system of the present invention by tanslocating the nucleic acids by the nanopore system using the electrophoresis force as well as the electroosmosis force (see FIG. 28).

In another embodiment of the present invention, protein profiling and drug identification according to target protein affinity for Bcl-2 family proteins (Bcl-xL, Bcl-w, Bcl-2, and Mcl-1) were performed using the nanopore system. As a result, it was observed that the nanopore current blockade signals for Bcl-2 family proteins Bcl-xL, Bcl-w, Bcl-2, and Mcl-1 all appeared in unique patterns that were able to be distinguished, a unique current blockade signal derived from each protein was observed even when equal amounts of Bcl-2 family proteins were mixed, and when A1155463, which is a small molecule drug targeting Bcl-xL, was added at an equal ratio, the unique current blockade signals of the proteins appeared in the case of Bcl-w, Bcl-2, Mcl-1, and mTOR, whereas the unique current blockade signal disappeared and a new current blockade signal appeared only in the case of the target protein Bcl-xL; thus, it was confirmed that profiling of Bcl-2 family proteins was possible through the nanopore system, and identification of drugs with selectivity with respect to target proteins was also possible (see FIG. 20).

The analysis method for a single analyte or a plurality of analytes may be used to measure and analyze the presence or absence, type, behavior, charge, type, size, structure, dynamics, orientation, flexibility, structural heterogeneity, or amount of the single analyte or the plurality of analytes. The analysis method may be used for biosensors for various analytes such as proteins and nucleic acids, detection of analytes at a single molecular level and analysis of structure and dynamics, analysis of biomolecular interactions, and sequence analysis of nucleic acids and proteins. In addition, the analysis method for a single analyte or a plurality of analytes may be utilized for new drug screening, disease diagnosis, protein identification, and proteomic analysis including protein post-translational modification. In addition, the analysis method for a single analyte or a plurality of analytes may be utilized for detection, identification, quantitative analysis, and screening of biomolecules through detection of characteristic electrical signals and fingerprinting of target substances in the presence of a plurality of analytes.

As an example, in a case in which the analyte is a nucleic acid, since the ionic current change pattern is different depending on the type of nucleotide when the nucleic acid is translocated through the nanopore, the nucleic acids may be identified by analyzing the pattern, which may be utilized for nucleic acid sequence analysis. Furthermore, the pattern is analyzed, such that the nucleotides constituting the nucleic acid may be identified, thereby allowing the nucleic acid sequence to be analyzed accurately and quickly. The nucleotides constituting the nucleic acid include a base, a sugar, and at least one phosphate group, and may be adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (UDP), uridine triphosphate (UTP), cytidine monophosphate (CMP), cytidine diphosphate (CDP), cytidine triphosphate (CTP), cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP), or deoxycytidine triphosphate (dCTP).

Still another aspect of the present invention provides a method for analyzing an interaction between an analyte and a ligand or screening a ligand for an analyte, the method including: performing a treatment with at least one analyte and at least one ligand candidate substance for the analyte in one compartment or two compartments of the nanopore system; and measuring changes in electrical signal in the two compartments before and after the treatment with the candidate substance.

The method for analyzing an interaction between an analyte and a ligand or screening a ligand for an analyte includes treating at least one analyte with at least one ligand candidate substance for the analyte in one compartment or two compartments of the nanopore system.

The analyte means a subject to be targeted for detection, interaction analysis, and screening of a ligand through an interaction analysis of the ligand or a screening method for a ligand for the analyte. The analyte may be a peptide, a polypeptide, a protein, an inorganic substance, a small molecule compound, or a nucleic acid, but is not limited thereto. In particular, the small molecule compound refers to an organic compound having a small molecular weight, for example, 1,000 daltons or less, in the fields of molecular biology and pharmacology. The small molecule compound often acts as a regulator or a drug in a biological process, and is about $10^{-9}$ m, which is small in size, and in the field of pharmacology, a small molecule refers to a molecule or a drug that binds to a biopolymer such as a protein or a nucleic acid and regulates the function of the biopolymer. The small molecule compound has various biological functions as a cell signaling substance. The small molecule compound may be naturally occurring (secondary metabolite) or artificially synthesized. The nucleic acid may be single-stranded DNA (ssDNA), double-stranded DNA (dsDNA), single-stranded RNA (ssRNA), double-stranded RNA (dsRNA), a double-stranded DNA/RNA hybrid, or a combination of the nucleic acids.

The ligand candidate substance means a substance expected to interact with the analyte. In particular, when the ligand interacts with the analyte in a free state, an oscillation movement pattern, a rotational motion pattern, or a nanopore translocation pattern of the analyte when captured in the lumen of the nanopore may be changed for physical or chemical reasons. For example, when the ligand interacts with the analyte in a free state to form a complex, a ratio of the analyte present in a free state decreases. Therefore, not only is the electrical signal exhibited by the analyte in a free state significantly decreased, but a new electrical signal exhibited by the complex of the analyte and the ligand is significantly increased, such that the complex of the analyte and the ligand may be directly detected.

The method for analyzing an interaction between an analyte and a ligand or screening a ligand for an analyte of the present invention may be utilized as a drug screening method. Specifically, in a case in which the analyte is a target of a drug and the ligand candidate substance is a drug candidate substance, analysis or screening of the interaction between the target of the drug and the drug candidate substance is possible, and accordingly, the drug candidate substance may be effectively screened.

In an embodiment of the present invention, the nanopore system was used to analyze an interaction between a target protein and a peptide binding to the target protein, thereby performing screening of a peptide drug. As a result of reacting Bcl-xL protein and three types of Bak-BH3 mutant peptides (Bak_BH3_D84A, Bak_BH3_I85A, and Bak_BH3 L78A) that bind to Bcl-xL protein and wild-type Bak_BH3 peptide, and observing and analyzing the current blockade signals derived from Bcl-xL protein and a Bcl-xL+peptide drug complex that appear at this time, it was confirmed that the scatter plot results obtained by measuring and analyzing the Bcl-xL protein and four types of mixed peptides were consistent with the scatter plot tendencies observed in the individual experiments, and the signal frequency of the Bcl-xL-peptide complex was consistent with the order of binding affinity between Bcl-xL protein and each peptide. Therefore, it was confirmed that the YaxAB nanopore of the present invention may perform binding affinity-based screening for a peptide drug that binds to a protein and may effectively select a peptide drug with a high binding affinity for the target protein even in a peptide mixed solution, and thus, may be usefully utilized as a drug screening platform (see FIG. 19). The description of the electrical signal is as described above in the analysis method for an analyte, and therefore, the above description is cited and not described repeatedly.

The method for analyzing an interaction between an analyte and a ligand or screening a ligand for an analyte may include performing a simultaneous treatment with at least one analyte and at least one ligand candidate substance for the analyte in one compartment or two compartments of the nanopore system.

In the performing of the simultaneous treatment with the at least one analyte and the at least one ligand candidate substance for the analyte in the one compartment or two compartments of the nanopore system, at least one analyte may be first placed in the one compartment or two compartments, and then, a plurality of ligands of the analyte, for example, 2 or more, 4 or more, 8 or more, 10 or more, 20 or more, 50 or more, or 100 or more ligands, may be treated simultaneously in the same compartment.

The method for analyzing an interaction between an analyte and a ligand or screening a ligand for an analyte of the present invention includes measuring changes in electrical signal of the analyte before and after the treatment with the candidate substance. The electrical signal exhibited by the nanopore oscillation pattern or rotational motion pattern of the analyte captured in the nanopore lumen in a free state or the nanopore translocation pattern of the analyte is measured, such that it is possible to determine whether the analyte interacts with the candidate substance, for example, whether the analyte and the candidate substance are bound to form a complex.

In addition, the method for analyzing an interaction between an analyte and a ligand or screening a ligand for an analyte may further include determining the candidate substance as a ligand interacting with the analyte in a case in which the change is significant. The case in which the change is significant may include a case in which a new signal that did not appear before or after the treatment with the ligand candidate substance appears.

In an embodiment of the present invention, using the YaxAB nanopore of the present invention, in a case in which Bak-BH3, which is a peptide that binds to Bcl-xL protein, or a small molecule compound (ABT-737) that binds to a protein was treated, it was confirmed that a distinct change in the pattern of ionic current was observed compared to a negative control (see FIG. 11), and even in a case in which Bax-BH3 peptide or Quercetin small molecule compound, which is a weak binder having a low binding affinity for Bcl-xL protein, was treated, it was also confirmed that changes in the pattern of ionic current was observed (see FIGS. 12 and 13).

In another embodiment of the present invention, as a result of measuring a current blockade signal generated when a glucose/galactose binding protein (GBP) that undergoes allosteric motion when binding to glucose or galactose, and galactose, which is a small molecule compound that targets GBP, are bound, using the YaxAB nanopore of the present invention, it was confirmed that a gating frequency occurring in the GBP-derived current blockade signal significantly increased when galactose was added, confirming that the GBP-galactose interaction depending on the presence or absence of galactose was effectively analyzed (see FIG. 14).

In still another embodiment of the present invention, when the target protein Bcl-xL was individually treated with non-binders (LCL-161 and GDC-0152) and binders (ABT-737 and A-1331852), using the YaxAB nanopore system, it was confirmed that the current noise ($I_N$) signals of the non-binders (LCL-161 and GDC-0152) did not change, and only the current noise ($I_N$) signals of the binders (ABT-737 and A-1331852) were specifically increased. Through this, it was confirmed that an interaction of a small molecule compound binding to Bcl-xL protein may be specifically detected through current noise ($I_N$) signal analysis of the YaxAB nanopore (see FIG. 17A).

In still another embodiment of the present invention, it was confirmed that single molecule fingerprinting analysis of protein-drug interactions may be performed at high resolution through a 2D density contour plot that expresses a ratio of the complex with small molecule compounds (LCL-161, GDC-0152, ABT-737, and A-1331852) that bind to the target protein Bcl-xL in real time as a two-dimensional representation of the current blockade ($\Delta I/I_o$) and current noise ($I_N$). Through this, it was confirmed that the signal of the Bcl-xL+small molecule compound complex changed depending on the type of small molecule compound binding to Bcl-xL, and thus, it was confirmed that various small molecule compounds binding to the Bcl-xL protein were clearly distinguished (see FIG. 17B), confirming that interactions with various ligands for the analyte Bcl-xL protein may be analyzed.

In another specific embodiment of the present invention, it was confirmed that, when the Bcl-xL+Bak-BH3 complex was simultaneously treated with four types of small molecule compounds (LCL-161, GDC-0152, ABT-737, and A-1331852) that bind to Bcl-xL, the ratio of the Bcl-xL+ABT-737 complex gradually decreased over time, while the ratio of the Bcl-xL+A-1331852 complex continuously increased, confirming that the binding activity between an analyte and a plurality of small molecule compounds may be simultaneously analyzed in real time using the YaxAB nanopore system of the present invention (see FIG. 17C).

In still another specific embodiment of the present invention, when a ligand (Bak-BH3) that binds to a target protein Bcl-xL and small molecule compounds (ABT-737 and A-1331852) were treated, it was confirmed that a ratio of an individual complex of the ligand and small molecule compounds that competitively bind to Bcl-xL was able to be analyzed in real time through a 2D density contour plot and a violin plot of current blockade ($\Delta I/I_o$)-current noise ($I_N$) with high resolution at a single molecule fingerprinting level. Through this, it was confirmed that after the addition of ABT-737 to the Bcl-xL+Bak-BH3 complex, the signal of the Bcl-xL+Bak-BH3 complex decreased and the signal of the Bcl-xL+ABT-737 complex increased in real time, it was confirmed that after the addition of A-1331852 to the Bcl-xL+ABT-737 complex, the signal of the Bcl-xL+ABT-737 complex decreased and the signal of the Bcl-xL+A-1331852 complex increased in real time, and through this, it was confirmed that ABT-737 and A-1331852 competitively bind to the same binding site as Bak-BH3 in the Bcl-xL protein (see FIG. 18).

A ratio of molecular weights of the analyte and the ligand may be 1:1 or more, 1:2 or more, 1:5 or more, 1:10 or more, 1:20 or more, 1:20 or more, 1:50 or more, 1:150 or more, 1:170 or more, 1:190 or more, 1:250 or more, 1:500 or more, or 1:1,000 or more. In a specific embodiment of the present invention, it was confirmed that since the nanopore system of the present invention was able to clearly distinguish the patterns of ionic currents of an analyte and an analyte-ligand complex even though the ratio of the molecular weights of the analyte and the ligand differed by 16 times or 194 times, it was possible to sensitively analyze whether or not there was an interaction between the analyte and the ligand (see FIGS. 38 and 39).

A concentration ratio of the analyte and the ligand may be 1:2 or more, 1:4 or more, 1:10 or more, 1:50 or more, 1:60 or more, 1:80 or more, 1:90 or more, 1:100 or more, 1:120 or more, 1:150 or more, 1:200 or more, 1:300 or more, 1:500 or more, or 1:1,000 or more. In a specific embodiment of the present invention, even though the ratio of the ligand binding to the analyte was 1:2 or 1:100, which was a large difference in concentration, the difference in the pattern of ionic current was clearly distinguished from the case in which the analyte was captured alone; thus, it was confirmed that it was possible to sensitively analyze whether or not there was an interaction between the analyte and the ligand (see FIGS. 40 and 41).

In addition, the method for analyzing an interaction between an analyte and a ligand or screening a ligand for an analyte may be a qualitative analysis and/or quantitative analysis of the interaction between the analyte and the ligand. For example, the method may be an analysis of whether a ligand candidate substance binds to an analyte, an analysis of the type and amount of the ligand, and a real-time analysis of them. It may be seen that the degree or frequency of interaction between the analyte and the ligand becomes stronger or higher depending on the change in the electrical signal. In addition, the degree or frequency of interaction between the proteins as described above may be quantified, for example, by a binding affinity (binding dissociation constant ($K_D$)).

In a specific embodiment of the present invention, the YaxAB nanopore was used to perform a current noise dose-dependent quantitative analysis on the target protein Bcl-xL and the ligand Bak-BH3 peptide or the small molecule drugs ABT-737 and A-1331852, and the binding affinity ($K_D$) for Bcl-xL binding was calculated (see FIG. 15).

The method for analyzing an interaction between an analyte and a ligand or screening a ligand for an analyte may be used for protein-protein interaction, protein-ligand interaction research, and drug screening, and may be used for new drug discovery and design through drug screening of a protein-protein interaction inhibitor or promoter, a small molecule compound, or the like, and protein binding site mapping because it may analyze the competitive binding or structure-activity relationship (SAR) between the analyte and the ligand candidate substance when a plurality of candidate substances are treated simultaneously.

Still another aspect of the present invention provides a method for analyzing or screening an interaction inhibitor or promoter between biomolecules, the method including: reacting a plurality of biomolecules capable of interacting in one compartment of the nanopore system; performing a treatment with a candidate substance of the interaction inhibitor or promoter in the one compartment or two compartments; and measuring changes in electrical signal in the two compartments before and after the treatment with the candidate substance.

The method for analyzing or screening an interaction inhibitor or promoter of the present invention includes treating biomolecules in a free state in one of the two compartments of the nanopore system. In an embodiment of the present invention, biomolecules may be first placed in one of the compartments to allow a reaction to proceed, and then, the candidate substance may be treated in the reactants or the opposite compartment. In other words, the candidate substance may be treated after the biomolecules in a free state are placed in one of the compartments. In addition, the biomolecules in a free state and the candidate substance may be simultaneously treated in one of the compartments. In addition, the treatment with the candidate substance may be performed in a situation where the biomolecules are induced to move to another compartment through the YaxAB nanopores contained in the nanopore system, for example, a gradient is formed according to the difference in salt concentration in the two compartments divided by the separation membrane, a voltage is applied, or both of these are provided.

In addition, the biomolecules may be used without limitation as long as they may interact with each other, for example, may be bound to each other to form a complex, and may be, for example, inorganic particles formed of peptides, proteins, lipids, carbohydrates, small molecule compounds, nucleic acids, or nanomaterials. For example, the biomolecules may be receptors, antigens, antibodies, or aptamers.

The ligand candidate substance may be any type that is expected to inhibit or promote the interaction between the biomolecules, for example, a single compound such as a nucleic acid, an amino acid, or a monosaccharide, or a polymer such as a polynucleotide, a peptide, protein, or a polysaccharide, and means a molecule such as a natural or synthetic organic substance, an inorganic substance, or a complex thereof. For example, the ligand candidate substances may be receptors, antigens, antibodies, or aptamers.

Among the ligand candidate substances, a ligand candidate substance that inhibits the interaction between the biomolecules, particularly, a ligand candidate substance that competitively binds to some of the biomolecules and inhibits the interaction between the biomolecules, is called an "inhibitor", and the inhibitor may reduce the interaction between the biomolecules through such competitive binding. In other words, the inhibitor may bind to some of the biomolecules and increase a ratio of the biomolecules present in a free state where they do not interact with each other, and may also increase a ratio of a complex in which the inhibitor interacts with some of the biomolecules. As described above, when the ratio of some of the biomolecules present in a state where they do not interact with each other increases, the electrical signal resulting from the interaction between the biomolecules decreases, and a new electrical signal pattern exhibited by the complex in which the inhibitor interacts with some of the biomolecules may appear, such that an inhibitor that inhibits the interaction between the biomolecules may be directly detected or analyzed.

In addition, among the ligand candidate substances, a ligand candidate substance that promotes the interaction between the biomolecules is called a "promoter", and the promoter includes a molecular glue and a protein degradation agent (PROTAC), and may induce or increase the interaction between the biomolecules. For example, the promoter is a molecular glue and may bind to the biomolecules to induce or increase the interaction between the biomolecules. As described above, when the ratio of the biomolecules present in the state where the biomolecules interact with each other increases, the electrical signal resulting from the interaction between the biomolecules may increase, and a new electrical signal pattern exhibited by a complex in which the biomolecules and the promoter interact may appear, such that the promoter that promotes the interaction between the biomolecules may be directly detected or analyzed.

Therefore, the method for analyzing or screening an interaction inhibitor or promoter between biomolecules may further include determining the candidate substance as an inhibitor that inhibits the interaction between the biomolecules or a promoter that promotes the interaction in a case in which the change in electrical signal is significant.

The case in which the change in electrical signal is significant may include a case in which a new signal that did not appear before or after a treatment with a candidate substance for an interaction inhibitor or promoter, or a case in which an electrical signal resulting from an interaction between biomolecules increases or decreases. In particular, the case in which the change in electrical signal may include a case in which a pattern appearing in a two-dimensional density contour plot of current blockade ($\Delta I/I_o$) and current noise ($I_N$) changes.

The method for analyzing or screening an interaction inhibitor or promoter between biomolecules may be used for new drug discovery and design through drug screening of a protein-protein interaction inhibitor or promoter and protein binding site mapping.

In a specific embodiment of the present invention, activity of a protein-protein inhibitor targeting the Bcl-xL+Bak-BH3 complex was analyzed using the YaxAB nanopore system. In a case in which a treatment was performed with a small molecule compound (ABT-737) that binds to the Bcl-xL protein, the signal of the Bcl-xL+Bak-BH3 complex was confirmed to decrease and the signal of the Bcl-xL+ABT-737 complex was confirmed to increase, confirming that the binding activity of the inhibitor for an interaction between an analyte and a ligand may be analyzed in real time (see FIG. 16).

In another embodiment of the present invention, activity of an inhibitor for an interaction between BRD4-BD1 target protein and acetylated peptide (H4_1-12 K5acK8ac) was analyzed using the YaxAB nanopore system. As a result, in a case in which the BRD4-BD1 target protein and the acetylated peptide (H4_1-12 K5acK8ac) were reacted with each of four types of small molecule compounds (GSK778, Y06026, XMD8-92, and PLX51107) that bind to the BRD4-BD1 protein, the unique current blockade signals of the target protein-small molecule compound complexes were confirmed, the signals derived from the four types of the target protein-small molecule compound complexes were significantly different from the signals of the target protein-acetylated peptide complexes, and differences also appeared between the target protein-small molecule compound complexes; thus, it was confirmed that the activity of the interaction inhibitor that inhibits target protein-acetylated peptide interaction may be analyzed, and further, small molecule compounds that bind to target proteins may be distinguished (see FIG. 22).

In still another embodiment of the present invention, the YaxAB nanopore system was used to analyze the promotion of the protein-protein interactions by the molecular glue. In a case in which mTOR protein, FKBP12 protein, and rapamycin, which acts as a molecular glue, were added together, a new current blockade signal appeared along with current blockade signals corresponding to mTOR protein and FKBP12 protein, it was confirmed that such a signal was a current blockade signal by a ternary complex in which mTOR, FKBP12, and the molecular glue were all bound, rather than a binary complex formed by binding of mTOR or FKBP12 protein and the molecular glue; thus, it was confirmed that it was possible to distinguish between the protein and the ternary complex, and through this, it was possible to analyze the promotion of the protein-protein interaction by the molecular glue (see FIG. 21).

The analysis may be a qualitative or quantitative analysis of the inhibitor for the interaction between the analyte and the ligand. For example, the analysis may be, but is not limited to, the presence or absence of the interaction inhibitor, the amount of the inhibitor, whether there is competitive binding with the ligand for the analyte, identification of the binding site of the inhibitor to the target substance (binding site mapping), or a binding affinity of the analyte and the inhibitor (binding dissociation constant, $K_D$).

In addition, the analysis may be a qualitative or quantitative analysis of an interaction promoter of at least one analyte and at least one ligand. For example, the analysis may be, but is not limited to, the presence or absence of the interaction promoter, the amount of the promoter, the presence or absence of a binary complex, or the presence or absence of a ternary complex.

Still another aspect of the present invention provides a method for identifying and quantitatively analyzing an analyte in a sample, the method including: placing a sample in one compartment of the nanopore system; and comparing changes in characteristic electrical signal caused by a plurality of analytes in the sample with a database of electrical signals for each substance.

In addition, still another aspect of the present invention provides a method for providing information for diagnosing a biomarker-associated disease, the method including: placing a sample in one compartment of the nanopore system; and measuring changes in characteristic electrical signal caused by a biomarker for a specific disease among changes in electrical signals induced by the sample.

The sample means a sample that may contain the analyte or biomarker without limitation, and may be, for example, one or more selected from the group consisting of a cell sample, a tissue sample, a blood sample, a urine sample, a saliva sample, a lymph sample, a cerebrospinal fluid sample, an amniotic fluid sample, a pleural fluid sample, a pericardial fluid sample, an ascites sample, an aqueous humor sample, a bone marrow sample, a semen sample, a biopsy sample, a cancer sample, a tumor sample, a forensic sample, an archaeological sample, a paleontological sample, an infection sample, a production sample, a plant sample, a microbial sample, a virus sample, a soil sample, a marine sample, and a freshwater sample.

The tissue sample may be derived from one or more tissues selected from the group consisting of epididymis, eye, muscle, skin, tendon, vein, artery, blood, heart, spleen, lymph nodes, bone, bone marrow, lung, bronchial tubes, tracheae, intestine, small intestine, large intestine, colon, rectum, salivary glands, tongue, bladder, appendix, liver, pancreas, brain, stomach, skin, kidney, ureters, urinary bladder, urethra, sex glands, testis, ovary, uterus, fallopian tubes, thymus, pituitary gland, thyroid, adrenal glands, and parathyroid glands. In addition, the tissue sample may be derived from any of a variety of organs of a human or another organism.

The cell sample may be derived from an animal cell, a plant cell, a fungal cell, a bacterial cell, or a protoplast cell, specifically, may be derived from an animal cell, and more specifically, may be derived from a human cell.

For example, the cell sample may be derived from one or more cells selected from the group consisting of a germ cell (egg cell, sperm, or the like), an ovarian epithelial cell, an ovarian fibroblast, an immune cell, a B cell, a T cell, a natural killer cell, a dendritic cell, a cancer cell, a eukaryotic cell, a stem cell, a blood cell, a muscle cell, an adipocyte, a skin cell, a nerve cell, a bone cell, a pancreatic cell, an endothelial cell, a pancreatic epithelial cell, a pancreatic alpha cell, a pancreatic beta cell, a pancreatic endothelial cell, a bone marrow lymphoblast, a bone marrow B lymphoblast, a bone marrow macrophage, a bone marrow erythroid cell, a bone marrow dendritic cell, a bone marrow adipocyte, a bone marrow osteocyte, a bone marrow chondrocyte, a preosteoblast, a bone marrow megakaryoblast, a brain B lymphocyte, a brain glial cell, a neuron, a brain astrocyte, a neuroectodermal cell, a brain macrophage, a brain microglial cell, a brain epithelial cell, a cortical neuron, a brain fibroblast, a breast epithelial cell, a colon epithelial cell, a colon B lymphocyte, a breast myoepithelial cell, a breast fibroblast, a colon enterocyte, a cervical epithelial cell, a ductal epithelial cell, a tongue epithelial cell, a tonsillar dendritic cell, a tonsillar B lymphocyte, a peripheral blood lymphoblast, a peripheral blood T lymphoblast, a peripheral blood skin T lymphocyte, a peripheral blood natural killer cell, a peripheral blood B lymphoblast, a peripheral blood monocyte, a peripheral blood myeloblast, a peripheral blood mononuclear cell, a peripheral blood preosteoblast, a peripheral blood macrophage, a peripheral blood basophil, a liver endothelial cell, a liver mastocyte, a liver epithelial cell, a liver B lymphocyte, a spleen endothelial cell, a spleen epithelial cell, a spleen B lymphocyte, a liver cell, a liver fibroblast, a lung epithelial cell, a bronchial epithelial cell, a lung fibroblast, a lung B lymphocyte, a lung Schwann cell, a lung squamous cell, a lung macrophage, a lung osteoblast, a neuroendocrine cell, an alveolar cell, a gastric epithelial cell, a gastric fibroblast, a stem cell, a fetal cell, a tumor cell, a suspicious cancer cell, a cancer cell, and a cell that has undergone a gene editing procedure.

In the method for identifying and quantitatively analyzing an analyte in a sample of the present invention, the analyte means a subject for analyzing the presence or absence and amount in the sample through the method, and the analyte may be, but is not limited to, a peptide, a polypeptide, a protein, an inorganic substance, a small molecule compound, or a nucleic acid.

In the method for identifying and quantitatively analyzing an analyte in a sample of the present invention, a sample is placed in one compartment of the nanopore system, and changes in characteristic electrical signals caused by a plurality of analytes in the sample are measured, and the measured changes are compared with a database of electrical signals for each substance, such that identification and quantitative analysis of the analyte in the sample may be performed. The database of electrical signals for each substance may be constructed by measuring characteristic electrical signals for each substance using the nanopore system of the present invention.

The method for identifying and quantitatively analyzing an analyte in a sample of the present invention may accurately measure the presence or absence and amount of an analyte by detecting changes in characteristic electrical signal of a specific analyte to be identified among the electrical signal changes of the entire sample, even when numerous types of analytes are included in various samples, and may thus be effectively used for analyte identification and quantitative analysis, and further, drug screening and the like.

In addition, in the present invention, the "biomarker" means an anatomical, physiological, biochemical, or molecular parameter associated with the presence of a specific physiological state or progression. The biomarker may include an organic biomolecule such as a polypeptide, protein, or nucleic acid (for example, mRNA or the like), lipid, glycolipid, glycoprotein, or sugar (for example, monosaccharide, disaccharide, oligosaccharide, or the like) that shows an increase or decrease within an individual or within a specific sample derived from an individual.

The method for providing information for diagnosing a biomarker-associated disease of the present invention may accurately measure the presence or absence and amount of the biomarker in the sample by placing a sample in one compartment of the nanopore system and measuring changes in characteristic electrical signal caused by a biomarker known to be associated with a specific disease among changes in electrical signals induced by the sample, and may thus be effectively used for diagnosis of a biomarker-associated disease and prediction of prognosis of a disease.

The disease is not limited as long as it is a disease for which an association with a specific biomarker is identified, and may be an infectious or non-infectious disease. The infectious disease is a disease caused by microorganisms such as bacteria and viruses, and may be, for example, sepsis, septic shock, severe acute respiratory syndrome coronavirus (SARS-CoV) infection, Middle East respiratory syndrome (MERS), salmonellosis, food poisoning, typhoid fever, paratyphoid fever, systemic inflammatory response syndrome (SIRS), multiple organ dysfunction syndrome (MODS), pneumonia, pulmonary tuberculosis, tuberculosis, colds, influenza, respiratory tract infection, rhinitis, nasopharyngitis, otitis media, bronchitis, lymphadenitis, parotitis, lymphadenitis, cheilitis, stomatitis, arthritis, myositis, dermatitis, vasculitis, gingivitis, periodontitis, keratitis, conjunctivitis, wound infection, peritonitis, hepatitis, osteomyelitis, cellulitis, meningitis, encephalitis, brain abscess, encephalomyelitis, meningitis, osteomyelitis, nephritis, carditis, endocarditis, enteritis, gastritis, esophagitis, duodenitis, colitis, urethritis, cystitis, vaginitis, cervicitis, salpingitis, erythema infectiosum, bacillary dysentery, abscess and ulcer, bacteremia, diarrhea, dysentery, gastroenteritis, gastrointestinal tract inflammation, urogenital abscess, an infection of open wound or wound, purulent inflammation, abscess, boil, pyoderma, impetigo, folliculitis, cellulitis, postoperative wound infection, skin scalded syndrome, skin burn syndrome, thrombotic thrombocytopenia, hemolytic uremic syndrome, renal failure, pyelonephritis, glomerulonephritis, nervous system abscess, otitis media, sinusitis, pharyngitis, tonsillitis, mastoiditis, phlegmon, odontogenic infection, dacryocystitis, pleurisy, abdominal abscess, liver abscess, cholecystitis, splenic abscess, pericarditis, myocarditis, placentitis, amniotic sac, mastitis, mastadenitis, puerperal fever, toxic shock syndrome, Lyme disease, gas gangrene, atherosclerosis, Mycobacterium avium complex (MAC), enterohemorrhagic *Escherichia coli* (EHEC) infection, enteropathogenic *Escherichia coli* (EPEC) infection, enteroinvasive *Escherichia coli* (EIEC) infection, methicillin-resistant *Staphylococcus aureus* (MRSA) infection, vancomycin-resistant *Staphylococcus aureus* (VRSA) infection, or listerosis.

The non-infectious disease may be cancer, autoimmune disease, non-infectious inflammatory disease, neurodegenerative disease, heart disease, stroke, diabetes, metabolic disease, chronic kidney disease, or chronic respiratory disease. The cancer may be non-small cell lung cancer, small cell lung cancer, renal cell cancer, kidney cancer, liver cancer, bone cancer, skin cancer, colon cancer, rectal cancer, ovarian cancer, breast cancer, pancreatic cancer, stomach cancer, bladder cancer, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycosis fungoides, Merkel cell carcinoma, classical Hodgkin lymphoma (CHL), primary mediastinal large B-cell lymphoma, T-cell/histiocyte-rich B-cell lymphoma, Epstein-Barr virus (EBV)-positive and -negative posttransplantation lymphoproliferative disease (PTLD), EBV-associated diffuse large B-cell lymphoma (DLBCL), plasmablastic lymphoma, extranodal NK/T-cell lymphoma, nasopharyngeal carcinoma, human herpes virus 8 (HHV8)-associated primary effusion lymphoma, other hematological malignancies including Hodgkin lymphoma, primary central nervous system (CNS) lymphoma, spinal tumor, brainstem glioma, or the like. The autoimmune disease may be lupus, systemic lupus erythematosus, Sjogren's syndrome, arthritis, rheumatoid arthritis, asthma, COPD, pelvic inflammatory disease, Alzheimer's disease, inflammatory bowel disease, Crohn's disease, ulcerative colitis, Peyronie's disease, coeliac disease, gallbladder disease, pilonidal disease, peritonitis, psoriasis, psoriatic arthritis, vasculitis, surgical adhesions, stroke, type 1 diabetes, Lyme disease, meningoencephalitis, autoimmune uveitis, multiple sclerosis, Guillain-Barr syndrome, atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, other autoimmune diseases, pancreatitis, trauma (surgery), graft-versus-host disease, transplant rejection, heart disease including ischemic diseases such as myocardial infarction and atherosclerosis, intravascular coagulation, bone resorption, osteoporosis, osteoarthritis, periodontitis and hypochlorhydria, infertility related to lack of fetal-maternal tolerance, leukoplakia, myasthenia gravis, systemic sclerosis, or the like. The neurodegenerative disease may be cognitive impairment, brain tumor, Alzheimer's disease, dementia, stroke, spinal cord injury, amyotrophic lateral sclerosis, Parkinson's disease, Huntington's disease, multiple sclerosis, glioblastoma, melanoma, pain, memory decline, or the like.

In a specific embodiment of the present invention, it was confirmed whether selective detection and quantitative analysis of a specific biomarker protein present in a serum sample is possible. It was confirmed whether unique characteristic electric signals of Bcl-xL at concentrations of 10 nM and 20 nM appeared in the presence of fetal bovine serum (FBS) similar to human serum, and when a voltage of 80 mV was applied in the presence of FBS from which some proteins were removed (depleted FBS), a unique characteristic electric signal of 10 nM Bcl-xL protein was observed in addition to various nanopore electric signals measured in the depleted FBS; thus, a signal distribution consistent with the unique electric signal of Bcl-xL protein among various scattered signals in the depleted FBS was confirmed. In addition, as a result of performing electrical measurements under 20 nM Bcl-xL protein conditions in the presence of depleted FBS, unique electrical signals of Bcl-xL were more measured compared to 10 nM Bcl-xL protein conditions, and this was confirmed in the two-dimensional scatter plot results that the Bcl-xL protein signal distribution was clearly densely distributed in a concentration-dependent manner; thus, it was confirmed that the nanopore system of the present invention may accurately measure the presence or absence and amount of an analyte protein in a mixed sample containing numerous types of substances (see FIG. 23).

Hereinafter, the present invention will be described in detail with reference to examples.

However, the following examples are intended to illustrate the present invention specifically, and the present invention is not limited by the following examples.

[Example 1]

**Production of YaxAB nanopore sensor**

**[1-1] Preparation of sample**

Compounds used in a nanopore measurement buffer were purchased from Sigma-Aldrich (St. Louise, USA), lipids and surfactants were purchased from Avanti Polar Lipids (Alabaster, AL, USA), and Bak-BH3 peptide and Bax-BH3 peptide were purchased from Peptron Inc. (Daejeon, Korea). Small molecule compounds ABT-737 (Cayman chemical), LCL-161 (Selleckchem), GDC-0152 (Selleckchem), A-1331852 (Selleckchem), and Quercetin (Sigma-Aldrich) were prepared by dissolving in dimethyl sulfoxide-$d_6$ (Sigma-Aldrich).

**[1-2] Purification of first monomer (YaxA) and second monomer (YaxB)**

A first monomer (YaxA) and a second monomer (YaxB) used in the experiments were synthesized by Cosmogenetech. As for the first monomer (YaxA), an amino acid sequence of SEQ ID NO: 1 (YaxA_(45-410)) and a wild-type amino acid sequence of SEQ ID NO: 3 (YaxA_FL) were synthesized and purified, and as for the second monomer (YaxB), an amino acid sequence of SEQ ID NO: 2 (YaxB_(12-343)) and a wild-type amino acid sequence of SEQ ID NO: 4 (YaxB_FL) was synthesized and purified.

Specifically, YaxA and YaxB were inserted into a pET15b vector containing a thrombin cleavage site with N-terminal 6xHis-SUMO and 6xHis tag, respectively. YaxA was expressed in *E. coli* BL21 (DE3) pLysS cells grown overnight at 18°C in 2xYT medium after induction with 0.5 mM IPTG. YaxB was also expressed under the same conditions as those of YaxA. In order to purify the expressed YaxA protein, the cell pellets were resuspended in lysis buffer (50 mM Tris pH 8.0, 300 mM NaCl, 1 mM PMSF, 1 g/ml DNase I, and 0.2 mg/ml lysozyme) and lysed using a microfluidizer. The YaxA protein was dissolved in 3% DDM at 4°C for 2 hours. The purified lysate was loaded onto a 5 mL Ni-NTA affinity column equilibrated with buffer A (50 mM Tris pH 8.0, 300 mM NaCl, 0.05% Cymal-6, and 10 mM imidazole), and the proteins bound to Ni-NTA resin were eluted with a linear gradient of 10 mM to 1 M imidazole. Dialysis was performed at 4°C against buffer B (50 mM Tris pH 8.0, and 300 mM NaCl) in the presence of thrombin protease. YaxA was further purified in a second Ni-NTA step to remove the cleaved tag and protease. In the case of the YaxB protein, cell pellets were resuspended in lysis buffer (50 mM Tris pH 8.0, 300 mM NaCl, 1 mM PMSF, 1 g/mL DNase I, and 0.2 mg/mL lysozyme) and lysed using an ultrasonicator. The clarified lysate was loaded onto a 5 mL Ni-NTA affinity column equilibrated with buffer C (50 mM Tris pH 8.0, 300 mM NaCl, and 10 mM imidazole), and the bound protein was eluted with a linear gradient of 10 mM to 1 M imidazole. Dialysis was performed at 4°C against buffer E (20 mM HEPES pH 7.0, and 25 mM NaCl) in the presence of thrombin protease. Next, the sample was loaded onto a 5 mL HiTrap Q column equilibrated in buffer E and eluted with a linear salt gradient of 0 to 1 M NaCl. The peak fractions were further purified using Superdex 75 16/600 gel filtration column (GE Healthcare) using buffer F (25 mM HEPES pH 7.0, and 150 mM NaCl).

**[1-3] Preparation of YaxAB nanopore**

A modified YaxAB_dN nanopore was prepared using the first monomer of SEQ ID NO: 1 (YaxA_(45-410)) and the second monomer of SEQ ID NO: 2 (YaxB_(12-343)) purified in Example 1-2. In the same manner, a wild-type YaxAB nanopore containing a wild-type first monomer (SEQ ID NO: 3, YaxA_FL) and a wild-type second monomer (SEQ ID NO: 4, YaxB_FL) was prepared.

Specifically, 6 mg of each monomer was incubated together at 25°C for 30 minutes to allow complex formation. Cymal-6 (1.5% w/v) was added to the mixture of YaxA and YaxB, and the mixture was incubated at 4°C for 30 minutes and then injected onto a Superose 6 column running in the gel filtration buffer (25 mM HEPES pH 7.0, 150 mM NaCl, and 0.05% w/v Cymal-6). The peak fractions were separated into different sized YaxAB oligomer proteins with different numbers of YaxAB nanopore subunits by 4 to 16% blue native polyacrylamide gel electrophoresis (BN-PAGE, Thermo Fisher Scientific Korea, Invitrogen) (see FIG. 1). The bands corresponding to the YaxAB oligomer proteins were cut out from the gel and soaked in the gel filtration buffer, and the YaxAB oligomer protein was extracted from each band at 4°C. A wild-type YaxAB nanopore was extracted in the same manner, and a supernatant containing the modified YaxAB_dN nanopore or the wild-type YaxAB nanopore was used in the experiments.

**[1-4] Production of nanopore system and nanopore experimental data measurement method**

A nanopore sensor was produced using a YaxAB-$C_8$ nanopore containing the first monomer of SEQ ID NO: 1 (YaxA_(45-410)) and the second monomer of SEQ ID NO: 2 (YaxB_(12-343)) prepared in Example 1-3.

Specifically, two compartments were assembled by positioning a Teflon film having 100 $\mu$m pores between two Teflon chambers, and each compartment was filled with 0.8 mL of a solution containing 10 mM Tris-HCl (pH 7.5), 1 mM EDTA, and 1 M KCl. As the composition of the solution, several types of buffers, such as HEPES, phosphoate buffer, PBS, sodium acetate, and etc., as well as Tris-HCl may be used. The nanopores were pre-painted with lipid bilayers prior to assembly.

For electrical measurements, among the Ag/AgCl electrodes, a ground electrode was connected to a trans side, and a working electrode was connected to a cis side. Purified YaxAB-$C_8$ to YaxAB-$C_{13}$ were added to the cis compartment so that a single YaxAB nanopore was inserted into the lipid membrane, and a nanopore current signal was measured by applying a voltage of +100 mV to the cis compartment. After insertion of the YaxAB nanopore, 10 to 1,000 nM of various proteins (Bcl-xL, Hsp33, FKBP12, holo-transferrin, MDM2, BSA, Aldolase, Ferritin, and Thyroglobulin) or Bcl-2 family proteins were added, and then, A single or multiple different peptide ligands (Bak-BH3, Bak-BH3-I83A, Bak-BH3-D84A, and Bak-BH3-L78A), or a single or multiple different small molecule compounds (ABT-737, LCL-161, GDC-0152, A-1331852, Quercetin, A1155463, FK506, and Oxaliplatin) were added to the cis compartment. Electrical signals were recorded at a bias voltage of 40 to 120 mV using a patch clamp amplifier (Axopatch 200B, Molecular Devices Inc., Sunnyvale, CA, USA), and the collected data were analyzed using the single-channel search function of pClamp 11 software (Molecular Devices Inc.). Current signals recorded at a sampling rate of 100 kHz were filtered with a built-in 8-pole low-pass Bessel filter at 10 kHz. Sampling rates and filters may be used under various conditions for signal optimization. All nanopore experiments were conducted at 25°C.

[Example 2]

**YaxAB nanopore formation and electrical characteristic analysis**

**[2-1] Formation of YaxAB nanopore**

In order to confirm whether the YaxAB nanopore protein produced in Example 1-3 may be used as a nanopore sensor, the structure of the modified YaxAB_dN nanopore was analyzed by Negative-stain EM and Cryo-EM. As a result, as illustrated in FIGS. 2A to 2C, the modified YaxAB_dN nanopore was confirmed to be an alpha helical pore complex assembled with 8 to 10 subunits.

Based on the EM data, the modeling structure of FIG. 2D and the three-dimensional structure of YaxAB-$C_8$ as illustrated in FIG. 2E were derived. As a result, the YaxAB nanopore was confirmed to have a distinct symmetrical structure and have a wide first opening, a narrow second opening, and a middle area connecting the first opening and second opening. In addition, it was confirmed that a lumen of the nanopore had a constriction where a diameter became narrower. In particular, it was confirmed that the YaxAB-$C_8$ nanopore had a funnel structure with a wide inlet and a narrow outlet, in which a depth of the lumen was 17 nm, a diameter of a lumen of the wide first opening was 10 nm, an outer diameter of a lumen of the second opening was 2.7 nm, and a diameter of the constriction of the lumen located at the second opening was 1.9 nm, and the lumen of the nanopore had a wavy shape due to alpha helices in the shape of a coffee dripper with a constriction. Based on this, the structure of the YaxAB nanopore is illustrated in FIG. 3.

**[2-2] Molecular dynamics simulation analysis**

Prior to the experiments, the characteristics of the YaxAB nanopore were analyzed using molecular dynamics (MD) simulations. A simulation was carried out using a setup in which YaxAB-$C_8$ was inserted into a composite membrane comprising 1,2-diphytanoyl-sn-glycero-3-phosphocholine (DPhPC) and sterol, having a size of 25 $\times$ 25 nm. The molecular dynamics simulations were performed under a temperature condition of 303 K with a 1 M KCl solution and no surface tension through GROMACS 2020.2 package.

In order to measure the free energy of Bcl-xL protein through the YaxAB-$C_8$ nanopore at 100 mV, Bcl-xL was added at a distance of z = 10 nm from a nanopore-containing membrane, and then, MD simulations were performed by adjusting the distance (z) from the membrane in a range of 2 nm to 13 nm at intervals of 1 nm. The Bcl-xL protein was freely moved in x and y directions, and the free energy was calculated by integrating the average force at each set z value.

As a result, as shown in FIG. 4A, a local distribution of water and ion molecules inside the nanopore was confirmed, and it was confirmed that a flow velocity of potassium ions and water became faster as it approached the constriction of the nanopore when a positive voltage was applied. In the constriction of the lumen, a flow velocity of potassium ions (0.16 nm$^{-2}$ns$^{-1}$) was faster than a flow velocity of chloride ions (0.04 nm$^{-2}$ns$^{-1}$), which means that when a positive voltage is applied, the electroosmotic force (EOF) acts in the cis-to-trans direction.

In addition, as a result of measuring the free energy by MD simulations, as shown in FIG. 5, when the Bcl-xL protein was located at a distance of z = 4 and 10 nm, the free energy showed a double minimum. This was predicted to oscillate between several residence sites (R1 to R3) while inducing various current blockade levels (L1 to L3) due to the transient residence of the Bcl-xL protein at the free energy minimum. The minimum free energy value of the Bcl-xL observed at a distance of z = 4 nm means that the protein is preferentially located at this location once trapped within the YaxAB nanopore. MD simulations predicted that the flow of potassium ions and water would be significantly reduced in the constriction when Bcl-xL protein was trapped at z = 4 nm compared to the empty YaxAB nanopore (FIG. 4B). Therefore, due to the weakened EOF, the Bcl-xL protein will move toward the cis side (z = 10 nm). MD simulations showed that the flow of potassium ions and water was larger when the Bcl-xL protein was captured at a distance of z = 10 nm than when the Bcl-xL protein was captured at a distance of z = 4 nm (FIG. 4C). This suggests that the restored EOF causes the Bcl-xL protein to move back to the trans side (z = 4 nm).

## [2-3] Improvement to increase stability of nanopore-containing membrane

It was confirmed that there was a problem that the YaxAB nanopore was not inserted well into the DPhPC membrane, which is an artificial biomembrane material that has been widely used in the related art, or even when inserted, the YaxAB nanopore had low stability, and thus, the membrane easily ruptured (see FIG. 6A).

In order to complement this, a DPhPC-sterol composite membrane was constructed by combining 5 to 50 mol% of sterol molecules with DPhPC, and in the case of such a composite membrane, not only were all of the $C_6$, $C_7$, $C_8$, $C_9$, $C_{10}$, $C_{11}$, $C_{12}$, and $C_{13}$ nanopores, which are the YaxAB nanopores of the present invention, successfully inserted, but also, as shown in Table 1, it was confirmed that a certain amount of current flowed significantly stably following Ohm's law even after being inserted into the membrane (see FIGS. 6B and 7).

[Table 1]

| YaxAB nanopore | Experimental value (pA) |
|---|---|
| $C_4$ | 103.9 $\pm$ 2.26 |
| $C_5$ | 189.618 $\pm$ 5.37 |
| $C_6$ | 297.70 $\pm$ 7.85 |
| $C_7$ | 414.07 $\pm$ 2.69 |
| $C_8$ | 506.06 $\pm$ 6.35 |
| $C_9$ | 776.04 $\pm$ 17.37 |
| $C_{10}$ | 1086.52 $\pm$ 16.50 |

(continued)

| YaxAB nanopore | Experimental value (pA) |
|---|---|
| $C_{11}$ | 1459.18 $\pm$ 24.62 |
| $C_{12}$ | 1846.61 |
| $C_{13}$ | 2435.60 |

In addition, the conductivity results measured when a voltage of 100 mV was applied to the cis compartment were matched with each of the modified YaxAB_dN nanopores. A constant conductivity was observed as 1.0 $\pm$ 0.1 nS for YaxAB-$C_4$; 1.8 $\pm$ 0.1 nS for YaxAB-$C_5$; 3.0 $\pm$ 0.1 nS for YaxAB-$C_6$; 4.1 $\pm$ 0.1 nS for YaxAB-$C_7$; 5.0 $\pm$ 0.1 nS for YaxAB-$C_8$; 7.8 $\pm$ 0.2 nS for YaxAB-$C_9$; 10.9 $\pm$ 0.2 nS for YaxAB-$C_{10}$; 14.6 $\pm$ 0.2 nS for YaxAB-$C_{11}$; 18.5 nS for YaxAB-$C_{12}$; and 24.4 nS for YaxAB-$C_{13}$ (see FIGS. 7 and 8). The experimental values were confirmed to be theoretically consistent with the current values derived from the structures of YaxAB nanopores (5.4 nS for YaxAB-$C_8$; 8.2 nS for YaxAB-$C_9$; and 11.7 nS for Yax-AB-$C_{10}$), and through this, it was confirmed that the YaxAB nanopores of the present invention followed Ohm's law and were present significantly stably on the DPhPC-sterol composite membrane. A nanopore sensor was produced using a DPhPC-sterol composite membrane into which YaxAB-$C_8$ (hereinafter, referred to as a YaxAB nanopore) among them was inserted, and then, an experiment was performed.

## [2-4] Measurement of cation selectivity of YaxAB nanopore

In order to measure the movement of ions passing through the YaxAB nanopore of the present invention, ionic currents were measured by applying various voltages.

An ion selectivity ($P_K^+/P_{Cl}^-$), representing the relative permeability ratio of potassium ions to chloride ions, was calculated using the Goldman-Hodgkin-Katz equation of the following Equation 1.

<Equation 1>

$$\frac{P_{K^+}}{P_{Cl^-}} = \frac{[\alpha_{Cl^-}]_{cis} - [\alpha_{Cl^-}]_{trans}e^{V_rF/RT}}{[\alpha_{K^+}]_{cis}e^{V_rF/RT} - [\alpha_{K^+}]_{trans}}$$

Here, $[\alpha_{K+/Cl}^-]_{cis/trans}$ represents activity of potassium ions in a cis chamber or activity of chloride ions in a trans chamber, R represents an ideal gas constant, F represents a Faraday constant, and Vr represents a reversal potential measured under asymmetric salt conditions.

The reversal potential (Vr) of the YaxAB nanopore was measured using KCl solutions at different concentrations in 10 mM Tris HCl buffer at pH 7.5 in the compartments on both sides of the nanopore (trans/cis:

2M KCl/0.5M KCl). Specifically, 800 μL of a 1 M KCl solution was added to both the cis and trans compartments, and then, the YaxAB nanopore was inserted into the membrane. After adjusting the offset and balancing electrodes, 400 μL of the 1 M KCl solution was removed from both compartments, 400 μL of 3 M KCl was added to the trans compartment, and an equal volume of salt-free buffer was added to the cis compartment to induce a salt gradient. For each ion activity, the cation selectivity was calculated by multiplying a molar concentration of the given ion by an average ion activity coefficient (2 M KCl: 0.573, 0.5 M KCl: 0.649).

As a result, as illustrated in FIG. 9, it was confirmed that all three types of the modified YaxAB_dN nanopores ($C_8$, $C_9$, and $C_{10}$ nanopores) had asymmetric I-V curves. It was confirmed that the cation selectivity ($P_K^+/P_{Cl}^-$) was 2.34 for YaxAB-$C_8$; 1.72 for YaxAB-$C_9$; and 1.47 for YaxAB-$C_{10}$, and such a high cation selectivity of the YaxAB nanopore of the present invention may induce an electroosmotic force that acts predominantly in the opposite direction to the electrophoretic force. In a case in which a positive voltage is applied under a condition of pH 7.5, the amino acids forming the inner wall of the pore are negatively charged, which may induce strong electroosmosis by cations that predominantly flow from the cis direction to the trans direction along the inner wall of the pore (due to the excellent cation selectivity).

As such, since the YaxAB nanopore of the present invention may induce electroosmosis, an analyte may be captured inside the nanopore regardless of the overall charge, and since the diameter of the YaxAB nanopore may be controlled by adjusting the number of heterodimers composed of portions of the monomers YaxA and YaxB, in addition to having a wide inlet, even a large analyte may be captured inside the nanopore; thus, there is an advantage in that a subject to be analyzed is not limited by the charge or size of the analyte. Furthermore, since the YaxAB nanopore has a funnel structure in which the inlet is wide but the outlet is relatively much narrower, a current blockade by the analyte may change more drastically when the analyte is captured inside the nanopore compared to conventional cylindrical nanopores, and as a result, the YaxAB nanopore has the advantage of being able to detect even small changes in the analyte much more sensitively by maximizing the difference in current blockade by the analyte.

[Example 3]

## Detection of analyte at single molecular level using YaxAB nanopore sensor

In order to confirm whether the YaxAB nanopore sensor of the present invention may detect proteins at a single molecular level, an experiment was performed using the YaxAB-$C_8$ nanopore sensor produced in Example 1-4.

First, in order to confirm the principle of change in ionic current by an analyte in the YaxAB nanopore of the present invention, Bcl-xL protein (3.4 x 5.1 nm) having an overall negative charge, which is larger than 2 nm and cannot pass through the YaxAB nanopore of the present invention, was selected as a representative analyte. The Bcl-xL protein thus selected was placed in the cis compartment, and (+) and (-) 100 mV voltages were applied to the cis compartment and the trans compartment, respectively.

In the case of the Bcl-xL protein, since the inside of the pore was negatively charged at pH 7.5, the Bcl-xL protein was not able to flow into the inside of the nanopore by the electrophoretic force (EPF) caused by the voltage applied to the both compartments. However, the Bcl-xL protein flowed into the pore by the electroosmotic force (EOF) induced by the YaxAB nanopore of the present invention (see Capture (I) in FIG. 5A), and at this time, a current blockade of 38.4 ± 4.7% compared to the open pore current ($I_{open}$) was observed (see L1 in FIG. 5A). After trapping within the pore, the Bcl-xL protein underwent oscillation movement due to the balance between the electroosmotic force and the electrophoretic force (see Trap (II) in FIG. 5A), and the oscillation movement induced a current blockade in a range of 68.4 ± 3.6% to 79.0 ± 1.8% of the open pore current ($I_{open}$) in the ionic current (see L2 and L3 in FIG. 5A). In addition, when the Bcl-xL protein, which underwent oscillation movement as above, escaped from the nanopore (see Escape (III) in FIG. 5), current blockade was no longer induced and the ionic current restored to the level of the open pore current ($I_{open}$) in the open state.

Finally, it was confirmed that the Bcl-xL protein captured in the YaxAB nanopore exhibited three types of ionic current patterns (L1, L2, and L3), and in particular, the current blockade between L1 and L2/L3 was 30.1 to 41.0%, which was greater than those of conventional nanopores (for example, ClyA, PlyAB, MspA, NeoTrap, and the like).

The current blockade levels of the conventional ClyA nanopores were confirmed to be 1.3% for SBP1, 1.7% for SBP2, 8.3% for BtuF, 3.4% for GBP, 4.8% for SiaP, 2.5% for TbpA, 6.2% for LBP, 3.6% for SpuD, and 4.1% for MBP (Zernia et al., "Current Blockades of Proteins inside Nanopores for Real-Time Metabolome Analysis", ACS Nano, 14(2): 2296-2307 (2020)), and the current blockade levels of the conventional PlyAB nanopores were confirmed to be 23.2% for HbA and 21.5% for HbS (Huang et al., "Detection of single amino acid differences in haemoglobin from blood samples using a nanopore", Analytical Chemistry (2021)).

In addition, although the specific current blockade level was not separately presented for the conventional MspA nanopore, it was confirmed to be within 15% for lysozyme and within 18% for holo-myoglobin (S. Huang et al., "Machine Learning Assisted Simultaneous Structural Profiling of Differently Charged Proteins in a Mycobacterium smegmatis Porin A (MspA) Electroosmotic Trap", *JACS* (2022)).

In addition, although the specific current blockade level was not separately presented for the NeoTrap nanopore, it was confirmed that the current blockade level was within 20% for Hsp90 and Avidin, and thus, it was confirmed that the YaxAB nanopore of the present invention had a relatively high resolution (Schmid et al., "Nanopore electro-osmotic trap for the label-free study of single proteins and their conformations", *Nat. Nanotechnol.* (2021)).

Meanwhile, the current blockade of holo-myoglobin (within 18%) in MspA nanopore or HbA (within 23.2%) in PlyAB nanopore, which has a relatively high current blockade level among the conventional nanopores, was significantly lower than the current blockade of Bcl-xL (30.1 to 41.0%) in the YaxAB nanopore of the present invention. As a result of comparing the analytical resolution of the YaxAB nanopore and the conventional nanopores ClyA and MspA for the same analyte, in the case of the YaxAB nanopore of the present invention, a variation between the current levels in the GBP-derived signal was approximately 14.3%, which was significantly higher than a variation of 3.4% in the GBP-derived signal analyzed in the ClyA nanopore, which was 4.2 times or more greater (see FIGS. 14A and 14B), and in addition, in the case of the YaxAB nanopore of the present invention, variations between the current levels of the lysozyme and holo-myoglobin-derived signals were about 14.0% and 47.3%, respectively, which were about 1.3 times and 2.6 times greater than variations of lysozyme- and holo-myoglobin-derived signals analyzed by the MspA nanopore, which were 10.4% and 17.9%, respectively; thus, The excellent analytical performance of the YaxAB nanopore of the present invention was confirmed. (see FIGS. 14C and 14D).

In addition, using the YaxAB nanopore of the present invention, the signal characteristics of lysozyme were compared with those obtained using the MspA nanopore, based on three analytical parameters. As a result, in the YaxAB nanopore of the present invention, a value of the current blockade ($\Delta I/I_o$) of about $65.5 \pm 0.3\%$, a standard deviation (SD) of the ionic current of $15.9 \pm 0.6$ pA, and a trapping time in a range of 100 to 10,000 ms were derived from lysozyme protein, which were 1.9 times greater in $\Delta I/I_o$, 1.7 times greater in SD, and 100 times greater in analyte trapping time compared to $\Delta I/I_o$ of 33.9%, SD of 9 to 10 pA level, and a trapping time of 1 to 100 ms of the lysozyme protein signal derived from the conventional MspA nanopore, respectively; thus, when compared with the conventional MspA nanopore for the same analyte, it was confirmed that the analytical performance of the YaxAB nanopore of the present invention was excellent (see FIG. 14E).

Furthermore, in order to confirm a range of the protein that may be analyzed using the YaxAB nanopore of the present invention, the patterns of ionic currents were analyzed using Hsp33 protein (52.4 kDa) and BSA protein (66.5 kDa), which are larger in size and are more negatively charged than Bcl-xL protein (20.8 kDa), holo-Transferrin protein (77.1 kDa), which is large in size but has a relatively small negative charge, Aldolase protein (158 kDa), which is large in size but has a positive charge, Ferritin protein (440 kDa) and Thyrogobulin protein (669 kDa), which have similar surface charge characteristics to those of Bcl-xL but are larger in size, FKBP12 protein (13.0 kDa), which is electrically neutral and may be translocated through the YaxAB nanopore of the present invention because it is smaller than the Bcl-xL protein, and electrically positively charged MDM2 protein (12.3 kDa).

As a result, it was confirmed that the respective nine proteins exhibited distinctly different ionic current patterns (see FIG. 10), from which it was confirmed that single-molecule analysis of proteins of various sizes and charges is feasible.

[Example 4]

## Analysis of analyte-ligand interactions using the YaxAB nanopore sensor

### [4-1] Analysis of protein-protein or protein-small molecule compound interaction

First, it was confirmed whether a protein-protein or protein-small molecule compound interaction may be analyzed using the YaxAB nanopore of the present invention.

To the cis compartment of the sensor produced in Example 1-4, i) Bcl-xL protein was added alone at a concentration of 100 nanomolar (nM), ii) Bcl-xL protein and Bak-BH3 protein known to bind to Bcl-xL protein were added together at a molar ratio of 1:2, and iii) Bcl-xL protein and ABT-737, which is a small molecule compound known to bind to Bcl-xL protein and be a competitive inhibitor for Bak-BH3, were added together at a molar ratio of 1:2:5, and (+) and (-) voltages were applied to the cis compartment and the trans compartment, respectively.

As a result, it was confirmed that changes in pattern in which Bcl-xL protein induced the current blockade effect alone occurred due to the addition of Bak-BH3 protein or ABT-737, and different current blockade patterns appeared for these two substances; thus, it was confirmed that it was possible to distinguish between the protein-protein complex (Bcl-xL+Bak-BH3) and the protein-small molecule compound complex (Bcl-xL+ABT-737) (see FIGS. 11A and 11B).

In addition, as illustrated in FIGS. 11C to 11F, in addition to the current blockade analysis, current noise ($I_N$)-based analysis was also used to distinguish free Bcl-xL, Bcl-xL/small molecule compound complex, and Bcl-xL/peptide complex using the YaxAB nanopore. In particular, it was confirmed that the ionic current patterns of the analytes in the three cases as described above were able to be distinguished more clearly through signal filtering in a low oscillation range (100 Hz or lower).

Next, it was confirmed whether an interaction between a protein and a molecule with weak binding affinity may be analyzed using the YaxAB nanopore of the present invention.

To the cis compartment of the sensor produced in Example 1-4, i) Bcl-xL protein was added alone at a concentration of 1 micromolar ($\mu$M), and ii) Bcl-xL protein and Bax-BH3 peptide and Quercetin small molecule compound known to bind to Bcl-xL protein were added at molar ratios of 1:100 and 1:20, respectively. Thereafter, (+) and (-) voltages were applied to the cis compartment and the trans compartment, respectively.

As a result, the addition of Bax-BH3 peptide or Quercetin caused intermittent changes in the current blockade patterns induced by Bcl-xL protein alone, and these two substances also showed current blockade patterns which were different from each other; thus, it was confirmed that the interaction between Bcl-xL and the binders with low binding affinity can be analyzed (see FIGS. 12 and 13).

In addition, as illustrated in FIGS. 12 and 13, it was confirmed that the YaxAB nanopore was capable of distinguishing between free Bcl-xL and Bcl-xL complexes with either peptides or small-molecule compounds, using current noise ($I_N$)-based analysis in addition to current blockade analysis.

## [4-2] Analysis of protein structural change induced by ligand binding

Based on the results of the protein-small molecule compound interaction analysis, the YaxAB nanopore of the present invention was used to measure a current blockade signal generated when a glucose/galactose binding protein (GBP), which is composed of two domains and undergoes allosteric motion when binding to glucose or galactose, binds to galactose, which is a small molecule compound targeting GBP (see FIG. 14).

Specifically, 50 nM GBP was added to the cis compartment of the YaxAB nanopore and a (+) voltage of 80 mV was applied to the same compartment to confirm the current blockade signal generated by the capture of GBP protein. Current blockades corresponding to current levels 1 and 2 were observed, along with characteristic gating behavior. At this time, when galactose, which is a small molecule compound targeting GBP, was added to the trans compartment, it was confirmed that a gating frequency occurring in the GBP-derived current blockade signal significantly increased. In addition, in the case of GBP- and GBP-glucose-derived signals (G. Maglia et al., "Direct electrical quantification of glucose and asparagine from bodily fluids using nanopores", Nat. Commun.

(2018)) and GBP-galactose-derived signals (Chi et al., "Probing the Neuraminidase Activity of Influenza Virus Using a Cytolysin A Protein Nanopore", Anal. Chem. (2020)) in the conventional ClyA nanopore, a variation between current levels was confirmed to be approximately 3.4%, but in the case of the YaxAB nanopore of the present invention, a variation between current levels in the GBP and GBP-galactose derived signals was approximately 14.3%, which was significantly higher than that of the ClyA nanopore by 4.2 times or greater.

Through the above results, the unique electrical signals of GBP and the GBP-galactose complex, generated depending on the presence or absence of galactose were analyzed using the YaxAB nanopore of the present invention. As a result, it was possible to effectively analyze structural changes in the GBP protein due to galactose binding, and it was confirmed that the analysis resolution of the YaxAB nanopore of the present invention was significantly superior to that of the conventional ClyA nanopore.

## [4-3] Analysis of binding affinity of protein or small molecule compound for protein

Furthermore, based on the current noise ($I_N$) analysis measured in Example 3-2, a dose-dependent quantitative analysis was performed on the interaction between Bcl-xL protein and Bak-BH3 peptide or ABT-737 compound, and the binding affinity ($K_D$) for Bcl-xL protein was calculated.

Specifically, as a result of treating 100 nM Bcl-xL protein with Bak-BH3 protein at each of concentrations of 10, 30, 50, 100, 200, and 500 nM, it was confirmed that ratios of complex formation between the Bcl-xL protein and the Bak-BH3 protein at the different concentrations were 21.6%, 35.8%, 42.3%, 63.6%, 85.3%, and 95.9%, respectively, and from this, the binding affinity ($K_D$) of the Bak-BH3 protein for the Bcl-xL protein was analyzed to be 65.78 $\pm$ 10.32 nM (see FIG. 15A).

In addition, as a result of treating 100 nM Bcl-xL protein with ABT-737 compound at each of concentrations of 10, 50, 70, 100, 200, and 1,000 nM in the similar manner as described above, it was confirmed that ratios of complex formation between the Bcl-xL protein and the ABT-737 compound at the different concentrations were 17.4%, 58.2%, 71.1%, 87.9%, 98.0%, and 99.6%, respectively, and from this, the binding affinity ($K_D$) of the ABT-737 compound for the Bcl-xL protein was analyzed to be 37.97 $\pm$ 7.91 nM (see FIG. 15B).

In addition, as a result of treating 100 nM Bcl-xL protein with A-1331852 compound at each of concentrations of 10, 30, 50, 70, 100, 200, and 1,000 nM in the similar manner as described above, it was confirmed that ratios of complex formation between the Bcl-xL protein and the A-1331852 compound at the different concentrations were 34.5%, 65.7%, 78.1%, 98.2%, 99.5%, 100%, and 100%, respectively, and from this, the binding affinity ($K_D$) of the A-1331852 compound for the Bcl-xL protein was calculated to be 19.16 $\pm$ 4.47 nM (see FIG. 15C).

Through this, it was found that the YaxAB nanopore system of the present invention may analyze intensity of an interaction between a drug and a biomolecule such as a protein, and may thus be effectively used for drug screening.

**[4-4] Analysis of activity of protein-protein interaction inhibitor**

In addition, Bcl-xL protein at a concentration of 100 nM was reacted with 200 nM Bak-BH3 protein, and then, ABT-737, which is a competitive inhibitor of the Bak-BH3 protein, was treated at concentrations of 100, 500, and 1,000 nM. As a result, it was confirmed that as the concentration of the added ABT-737 compound increased, the ratio of occurrence of ionic current pattern of the complex of the Bcl-xL protein and the Bak-BH3 protein (Bcl-xL+Bak-BH3) gradually decreased, and the ratio of occurrence of ionic current pattern of the complex of the Bcl-xL protein and the ABT-737 compound (Bcl-xL+ABT-737) gradually increased (see FIG. 16).

From the above results, it was confirmed that the protein-drug complex may be directly detected with high resolution using the YaxAB nanopore of the present invention. This represents a significant improvement over conventional methods, which detect drug binding indirectly by measuring signal reduction of the the protein-protein complex, enabling direct and real-time detection and analysis of the protein-drug complex.

Next, it was confirmed whether it was possible to analyze an interaction between a target protein and a post-translational modification (PTM) peptide and a protein-protein interaction inhibitor using the YaxAB nanopore sensor.

First, an interaction between an acetylated peptide and a protein or a protein-small molecule compound was confirmed at a single molecular level using the YaxAB-$C_8$ nanopore sensor of the present invention. BRD4, which is a bromodomain and extra-terminal domain (BET) family protein, was used as the target protein. BRD4 is a chromatin binding protein associated with cancer and autoimmune diseases that acts as a scaffold for transcription factors at a promoter and a super enhancer. The BRD4 is divided into bromodomain 1 (BD1) and bromodomain 2 (BD2), BD1 has a more extended loop than BD2, and BD1 may specifically recognize and bind to an acetylated portion of an H4 portion of a histone protein. Considering the binding characteristics, the binding to the target protein BRD4-BD1 according to the presence or absence of PTM was confirmed using an H4 peptide consisting of 12 amino acids (H4_1-12) and the peptide acetylated at residues 5 and 8 (H4_1-12 K5acK8ac) in the histone protein (see FIGS. 22A and 22B).

Specifically, both compartments of the YaxAB-$C_8$ nanopore sensor were filled with a solution containing 10 mM Tris (pH 7.5), 1 mM EDTA, and 1 M LiCl. Then, 2 $\mu$M BRD4-BD1 protein was added to the cis compartment, a (+) voltage was applied, and a nanopore current blockade signal was observed. At this time, a unique nanopore current blockade signal expressed as a BRD4-BD1 protein-derived L1-L2 level, which is generated by the oscillation movement of BRD4-BD1 protein inside the nanopore, was observed. In addition, when the target protein and the acetylated peptide (H4_1-12 K5acK8ac) were added to the cis compartment at a molar ratio of 1:20 and the nanopore current blockade signal was measured, it was confirmed that an L3 level appeared newly in the BRD4-BD1 protein-derived L1-L2 level (see FIG. 22C).

Furthermore, the change in nanopore current blockade signal caused by the interaction between the BRD4-BD1 protein and the acetylated peptide (H4_1-12 K5acK8ac) was able to be expressed as the current noise ($I_N$) value through Power Spectrum Density (PSD) analysis, and based on this, a concentration-dependent quantitative analysis was performed to calculate a binding affinity ($K_D$) of the acetylated peptide (H4_1-12 K5acK8ac) for the BRD4-BD1 protein. Specifically, as a result of treating 2 $\mu$M of BRD4-BD1 protein with the H4_1-12 K5acK8ac peptide at concentrations of 2, 4, 6, 10, 20, and 40 $\mu$M, the binding affinity ($K_D$) of the acetylated peptide (H4_1-12 K5acK8ac) for the BRD4-BD1 protein was calculated to be $3.269 \pm 0.756 \ \mu$M (see FIG. 22D).

Through the above results, it was confirmed that the YaxAB nanopore system of the present invention was able to qualitatively and quantitatively analyze the interaction between the target protein and the ligand, which is determined depending on their post-translational modification (PTM) status.

Next, the activity of the inhibitor of the interaction between the BRD4-BD1 target protein and the acetylated peptide (H4_1-12 K5acK8ac) was confirmed in the YaxAB nanopore system of the present invention. Specifically, each of four types of small molecule compounds (GSK778, Y06026, XMD8-92, and PLX51107) binding to the BRD4-BD1 protein were reacted with the BRD4-BD1 protein, and then, the nanopore current blockade signals were measured, and as a result, characteristic nanopore current blockade patterns were observed for each protein-small molecule compound complex (see FIG. 22E).

Based on the above results, the BRD4-BD1 target protein and the acetylated peptide (H4_1-12 K5acK8ac) were reacted with each of the small molecule compounds, and then, nanopore current blockade signals were measured. As a result, the unique current blockade signals of the target protein-small molecule compound complexes were confirmed, and it was confirmed that the signals derived from the four types of target protein-small molecule compound complexes were significantly different from the signals of the target protein-acetylated peptide complexes, and there was a difference in the target protein-small molecule compound complexes (see FIG. 22F).

Through the above results, it is possible to not only analyze the target protein-acetylated peptide interaction using the YaxAB nanopore system of the present invention but also to analyze the activity of the interaction inhibitor that inhibits the target protein-acetylated peptide interaction, and furthermore, it is possible to distinguish small molecule compounds that bind to a target protein; thus, an interaction between the target protein and the acetylated peptide and screening of an interaction inhibitor therefor may be effectively performed using the YaxAB nanopore system of the present invention.

## [4-5] Screening of protein-drug interaction and single molecule fingerprinting analysis

It was confirmed how precisely the protein-small molecule compound interaction may be analyzed using the YaxAB nanopore of the present invention.

Two types of small molecule compounds that strongly bind to Bcl-xL protein (ABT-737 and A-1331852) and two types of small molecule compounds that do not bind to Bcl-xL protein (LCL-161 and GDC-0152) were selected, and Bcl-xL protein was treated with each of the selected small molecule compounds. As a result, as illustrated in FIG. 17A, when treated with ABT-737 and A-1331852 compounds, which are strong binders, the pattern of ionic current changed significantly, whereas when treated with LCL-161 and GDC-0152 compounds, which are non-binders, there was no significant difference from a negative control. In particular, as illustrated in FIG. 17B, it was confirmed that the event distribution of the mixture of Bcl-xL protein and the non-binders was identical to that of free Bcl-xL in the two-dimensional density contour plot of current blockade ($\Delta I/I_o$)-current noise ($I_N$). In contrast, strong binder-Bcl-xL protein complexes were clearly distinguished from the negative control by two separate peaks, similar to single-molecule fingerprinting.

In addition, in order to investigate whether the YaxAB nanopore of the present invention may be used to screen a drug from a mixture of various compounds, the protein-drug interaction (PDI) was monitored in real time using the current record of the YaxAB nanopore.

Specifically, as illustrated in FIG. 17C, after 35 minutes and 70 minutes of a simultaneous treatment of Bcl-xL with a mixture of compounds containing the non-binders (LCL-161 and GDC-0152) and the strong binders (ABT-737 and A-1331852), the distribution of the free Bcl-xL protein, which is a negative control, was significantly reduced (2.5% and 0.7%). In contrast, the event distributions of Bcl-xL/strong binder complexes appeared gradually, and after 70 minutes, two distinct event distributions corresponding to Bcl-xL+A-1331852 and Bcl-xL+ABT-737 complexes were clearly observed. At the same time point, a ratio of the Bcl-xL+ABT-737 complex gradually decreased (10.7% and 6.3%), whereas a ratio of the Bcl-xL+A-1331852 complex continuously increased (86.8% and 93.1%). From these experimental results, it was confirmed that the YaxAB nanopore of the present invention was able to screen the binding of drugs to the target protein at a single molecular level with a high resolution.

From this, the YaxAB nanopore may sensitively distinguish different small molecule compounds bound to the same protein with an excellent resolution, which may provide a concept and example for single molecule fingerprinting of protein-drug interactions using a biological nanopore sensor. From these results, it can be seen that a system using the YaxAB nanopore of the present invention may detect and analyze small molecule compounds with lower cost, faster, and higher sensitivity than a conventional biophysical analysis method such as ITC, NMR, or SPR.

## [4-6] Structure-activity relationship (SAR) analysis and binding site mapping

Meanwhile, it was examined whether the binding site where the protein-small molecule compound interaction occurs may be analyzed using the YaxAB nanopore of the present invention. To this end, Bcl-xL protein at a concentration of 100 nM was pre-reacted with 200 nM Bak-BH3 protein, and then, ABT-737, which is a competitive inhibitor of Bak-BH3 protein, was treated at a concentration of 200 nM.

As a result, it was observed that as the reaction time increased, the signal of the complex of the Bcl-xL protein and the Bak-BH3 protein (Bcl-xL+Bak-BH3) gradually decreased (87.6%, 34.0%, and 2.7%), and the signal of the complex of the Bcl-xL protein and the ABT-737 compound (Bcl-xL+ABT-737) increased (0%, 60.3%, and 94.9%) (FIG. 18B). In the 2D density contour plot of the current blockade ($\Delta I/I_o$)-current noise ($I_N$), the distribution of the Bcl-xL+Bak-BH3 complex gradually shifted to the newly formed distribution position of the Bcl-xL+ABT-737 complex over time, and after 40 minutes of the addition of ABT-737, only the Bcl-xL+ABT-737 complex remained, which means that ABT-737 completely replaced the Bak-BH3 peptide bound to Bcl-xL (see FIGS. 18B to 18D). From these results, it was confirmed that ABT-737 detached the previously bound Bak-BH3 and was bound to its site.

In addition, Bcl-xL protein at a concentration of 100 nM was reacted with 200 nM ABT-737 compound, and then, A-1331852 compound was treated at a concentration of 200 nM. As a result, it was observed that as the reaction time increased, the signal of the complex of the Bcl-xL protein and the ABT-737 compound (Bcl-xL+ABT-737) gradually decreased (93.0%, 52.5%, and 26.6%), and the signal of the complex of the Bcl-xL protein and the A-1331852 compound (Bcl-xL+A-1331852) increased (6.0%, 46.6%, and 72.5%) (see FIGS. 18E to 18G). From these results, it was confirmed that A-1331852 competed with the previously bound ABT-737 for binding to the same site.

From this, it can be seen that the YaxAB nanopore of the present invention may be used to screen drug binding activity for proteins at a single molecular level, and further, to analyze structure-activity relationship (SAR).

## [4-7] Screening of peptide drug that binds to target protein

An interaction between a target protein and a peptide was analyzed using the YaxAB nanopore sensor of the present invention, and through this, peptide drug screening was performed.

First, a solution containing 5 mM HEPES (pH 7.5), 1 mM EDTA, and 1 M KCl was placed in each compartment of the YaxAB nanopore sensor. The Bcl-xL protein was then reacted with each of the three Bak-BH3 mutant peptides (Bak_BH3_D84A, Bak_BH3_I85A, and Bak_BH3_L78A), which are known to bind to Bcl-xL, at a molar ratio of 1:15. The resulting mixture were added to the cis compartment, followed by the applications of a (+) voltage. At this time, current blockade signals derived from the Bcl-xL protein and the Bcl-xL+peptide drug complex were observed and analyzed to represent a 2D scatter plot of current noise (SD)-current blockade ($\Delta I/I_o$) (see FIGS. 19A and 19B).

Based on this, a solution obtained by mixing Bcl-xL protein and three types of mutant peptides (Bak_BH3_D84A, Bak_BH3_I85A, and Bak_BH3_L78A) and wild-type Bak_BH3 was reacted at a molar ratio of 1:15, and then, a current blockade signal was measured. As a result, unique current blockade signals that appeared when each peptide was bound were observed, and it was confirmed that the scatter plot results measured and analyzed by adding Bcl-xL protein and four mixed peptides were consistent with the scatter plot tendencies that appeared during individual experiments (see FIG. 19C).

In addition, the signal frequencies of the Bcl-xL-peptide complexes were 51% for wild-type Bak_BH3, 39% for Bak_BH3_D84A, 7% for Bak_BH3_I85A, and 3% for Bak_BH3_L78A, which were consistent with the binding affinity order between Bcl-xL protein and each peptide (wild-type Bak_BH3 0.34 $\mu$M; Bak_BH3_D84A 0.14 $\mu$M; Bak_BH3_I85A 93 $\mu$M; and Bak_BH3_L78A 270 $\mu$M).

Through the above results, it was confirmed that the YaxAB nanopore of the present invention may perform binding affinity-based screening for a peptide drug that binds to a protein, may effectively select a peptide drug having a high binding affinity for a target protein even in a peptide mixed solution, and may thus be usefully utilized as a drug screening platform.

## [4-8] Drug binding selectivity profiling for plurality of proteins

An experiment was performed to confirm whether protein binding selectivity profiling of a drug for Bcl-2 family proteins (Bcl-xL, Bcl-w, Bcl-2, and Mcl-1) is possible using the YaxAB nanopore sensor of the present invention.

First, a solution containing 10 mM Tris-HCl (pH 8.0), 1 mM EDTA, and 1 M KCl was placed in each compartment of the YaxAB nanopore sensor. Then, 50 nM of each of Bcl-2 family proteins (Bcl-xL, Bcl-w, Bcl-2, and Mcl-1), and mTOR protein as a negative control, were added to the cis compartment, and then a (+) voltage was applied.

At this time, it was observed that the nanopore current blockade signals for the respective proteins appeared as unique patterns that were all able to be distinguished, and through this, when a 2D scatter plot of current noise (SD)-current blockade ($\Delta I/I_o$) was represented, the signal distribution results derived from the respective proteins were observed (see FIGS. 20A to 20E).

Furthermore, equal concentrations (50 nM each) of Bcl-2 family proteins (Bcl-xL, Bcl-w, Bcl-2, and Mcl-1) and mTOR protein, which served as a negative control, were mixed to prepare a sample solution. The prepared solution was added to the cis compartment of the YaxAB nanopore system. Thereafter, a (+) voltage was applied. As a result, unique current blockade signals individually derived from Bcl-xL, Bcl-w, Bcl-2, Mcl-1, and mTOR proteins were observed. In addition, as a result of analyzing the nanopore current blockade signals derived from the mixed solution, the protein signal distributions corresponding to four types of Bcl-2 family proteins and one type of negative control were also observed on the 2D scatter plot (see FIG. 20F).

As a result of adding A1155463, which is a small molecule drug targeting Bcl-xL, into the mixture solution at a 1:1 molar ratio (50 nM), and subsequently analyzing the nanopore current blockade signals, it was observed that, in the case of Bcl-w, Bcl-2, Mcl-1, and mTOR, unique current blockade signals of proteins appeared, whereas only in the case of the target protein Bcl-xL, the unique current blockade signal of protein disappeared and a new current blockade signal appeared. As a result of analyzing the measured signals, distinct signal distributions for Bcl-w, Bcl-2, Mcl-1, and mTOR proteins were observed on the 2D scatter plot. In contrast, the signal distribution for the target protein Bcl-xL disappeared, and instead, a new distribution appeared, which was expected to represent the Bcl-xL-A1155463 complex (see FIG. 20G) .

Through the above results, it was verified that it is possible to profile Bcl-2 family proteins and also to identify a drug having selectivity for the target protein using the YaxAB nanopore sensor.

[Example 5]

## Whether ternary complex analysis including analysis of promotion of protein-protein interaction by molecular glue is possible using YaxAB nanopore sensor

It was examined whether the promotion of the protein-protein interaction by a molecular glue may be analyzed using the YaxAB nanopore system of the present invention.

First, the mTOR-FRB domain and the FKBP12 protein do not interact with each other alone, and the protein-protein interaction between two is mediated by rapamycin, which functions as a molecular glue. Specifically, rapamycin first binds to the FKBP12 protein, and subsequently, the mTOR-FRB domain binds to the FKBP12-rapamycin complex to form a ternary complex (see FIG. 21A).

Using the above characteristics, a solution containing 10 mM Tris-HCl (pH 8.0), 1 mM EDTA, and 1 M KCl was placed in each compartment of the YaxAB nanopore system. Then, 160 nM mTOR-FRB domain protein (hereinafter, referred to as mTOR protein) or FKBP12 protein was added to the cis compartment, and a (+) voltage was applied. As a result, nanopore current blockade signals were observed (see FIGS. 21B and 21C).

As a result, unique current blockade signals appeared from the mTOR protein and FKBP12 protein, respectively, and as a result of scatter plot analysis, it was confirmed that the distributions of signals derived from the respective proteins were clearly distinguished from each other. In addition, 160 nM each of mTOR protein and FKBP12 protein were added to the cis compartment at a 1:1 molar ratio as another negative control. Upon applications of a (+) voltage, nanopore current block signals were observed, current blockade signals of mTOR protein alone and FKBP12 protein alone appeared in a mixed state, and as a result of scatter plot analysis, it was confirmed that a unique signal distribution derived from each protein was reproduced (see FIG. 21F).

Based on the experimental results, when mTOR protein, FKBP12 protein, and rapamycin, which acts as a molecular glue, were added to the cis compartment at a molar ratio of 1:1:10, a (+) voltage was applied, and characteristic current blockade signals were observed, a new current blockade signal was observed along with the current blockade signals corresponding to mTOR protein and FKBP12 protein (see FIG. 21G).

In addition, in order to confirm that the new signal is a current blockade signal attributable to a ternary complex in which mTOR, FKBP12, and the molecular glue are all bound, rather than a binary complex formed by binding of mTOR or FKBP12 protein and the molecular glue, mTOR protein or FKBP12 protein and rapamycin were added to the cis compartment of the YaxAB nanopore system at a 1:1 ratio, a (+) voltage was applied. Thereafter, current blockade signals were observed (FIGS. 21D and 21E).

As a result, since the current blockade signals derived from mTOR protein and FKBP12 protein did not significantly change depending on the presence or absence of rapamycin, it was confirmed that the new current blockade signal was a current blockade signal derived from a ternary complex in which mTOR protein, FKBP12 protein, and rapamycin were all bound.

Through the above results, it was found that it was possible to distinguish between the protein and the ternary complex using the YaxAB nanopore system of the present invention, and through this, it was possible to analyze the promotion of the protein-protein interaction by the molecular glue.

Next, in order to confirm whether a molecular glue present in a mixed solution of various small molecule compounds may be screened, negative small molecule compounds (Astemizole, Mibefradil, Terfenadine, Pranlukast, Capecitabine, Dimantine, Sulfanilamide, and Monobenzone) that do not bind to the mTOR-FRB domain were selected by nuclear magnetic resonance (NMR) spectroscopy, and it was examined whether the molecular glue was screened by observing a ternary complex signal according to the presence or absence of rapamycin, which is a molecular glue, in the negative small molecule compound mixed solution.

Specifically, when equal amounts of mTOR protein and FKBP12 protein were reacted with a mixed solution of eight types of negative small molecule compounds at a molar ratio of 1:1:10, and nanopore current blockade signals were observed, only unique current blockade signals of the mTOR protein and FKBP12 protein were observed (see FIGS. 21H). Meanwhile, when rapamycin, which is a molecular glue, was reacted at a molar ratio of 1:1:10:10 under the above experimental conditions, a current blockade signal corresponding to the ternary complex was observed along with the mTOR protein and FKBP12 protein (see FIG. 21I).

In summary of the above results, the YaxAB nanopore system of the present invention may distinguish among a single protein, a protein-small molecule compound binary complex, and a protein-small molecule compound-protein ternary complex. Furthermore, the YaxAB nanopore system may even screen for the presence or absence of a molecular glue in a mixed solution of various small molecule compounds, without interference from negative small molecule compounds. Therefore, it was confirmed that a molecular glue capable of promoting a protein-protein interaction may be screened using the YaxAB nanopore system of the present invention.

[Example 6]

**Whether selective detection and quantitative analysis of biomarker protein is possible using YaxAB nanopore**

It was examined whether selective detection and quantitative analysis of a specific biomarker protein in the presence of serum was possible using the YaxAB nanopore system of the present invention.

The Bcl-xL protein selected as an analyte is a protein overexpressed in various types of malignant tumors, is well known as a target of a cancer therapeutic agent, and can also be used as a biomarker for cancer diagnosis (Morales-Martinez et al., "Roles and Regulation of BCL-xL in Hematological Malignancies", Int. J.

Mol. Sci. (2022)).

First, after 800 μl of 10 mM Tris (pH 7.5), 1 mM EDTA, and 1 M KCl buffer was added to both compartments of the YaxAB nanopore system, 50 nM Bcl-xL protein was introduced into the cis compartment to confirm a unique electrical signal of Bcl-xL protein. A voltage of 80 mV was then applied, and a change in electrical signal was measured. When the results were statistically analyzed and a two-dimensional scatter plot of current noise (SD)-current blockade ($\Delta I/I_o$) was represented, the two-dimensional scatter plot results showing a uniform current pattern and a dense distribution were confirmed as illustrated in FIG. 23A.

Next, in order to utilize the YaxAB nanopore system of the present invention for cancer diagnosis, an experiment was performed to determine whether a trace amount of Bcl-xL protein present in a mixed solution was able to be detected. First, it was observed whether a unique characteristic electrical signal of Bcl-xL at concentrations of 10 nM and 20 nM was generated in the presence of Fetal Bovine Serum (FBS), which is similar to human serum. The FBS solution was used after removing serum proteins, such as albumin and immunoglobulin, using a depletion mini spin column. Thereafter, 5 μl of the FBS solution was added to the cis compartment of the YaxAB nanopore system in which 800 μl of 10 mM Tris pH 7.5, 1 mM EDTA, and 1 M KCl buffer was present in each of the both compartments, and a change in electrical signal that occurred after applying a voltage of 80 mV was measured.

As a result, various types of electrical signals were observed, and when these electrical signals were statistically analyzed and represented as a two-dimensional scatter plot, the distributed results were shown as illustrated in FIG. 23B. Specifically, when a voltage of 80 mV was applied to the cis compartment of the YaxAB nanopore system in the presence of FBS from which some proteins were removed (depleted FBS), a unique characteristic electrical signal of 10 nM Bcl-xL protein was observed in addition to various nanopore electrical signals measured in the depleted FBS. When the signals were analyzed and represented as a two-dimensional scatter plot, a signal distribution corresponding to the unique electrical signal of the Bcl-xL protein in the various scattered signals of depleted FBS was shown (see FIG. 23C).

Furthermore, as a result of performing electrical measurements under 20 nM Bcl-xL protein condition in the presence of depleted FBS, a greater number of unique electrical signals of Bcl-xL were measured compared to 10 nM Bcl-xL protein condition, and it was also shown in two-dimensional scatter plot results that the Bcl-xL protein signal distribution was clearly densely represented in a concentration-dependent manner (see FIG. 23D).

Accordingly, it was examined whether a trace amount of Bcl-xL protein present in a mixed solution may be detected, and this verified that the YaxAB nanopore system of the present invention may be used to specifically and highly sensitively detect and quantitatively analyze a trace amount of a biomarker protein in a mixed solution within a short period of time; thus, it was confirmed that the system has the possibility to be used for the diagnosis of various diseases including cancer and genetic diseases, and further for early diagnosis requiring the detection of trace amounts of biomarkers.

Meanwhile, as illustrated in FIG. 24, an experiment was performed to confirm whether mutant Bcl-xL proteins frequently observed in cancer patients may be detected using the YaxAB nanopore system of the present invention. Specifically, Bcl-xL_R100E,R103E mutant protein, in which arginine residues at positions 100 and 103 of the Bcl-xL protein, which may be used as a biomarker for cancer diagnosis, were substituted with (-) charged glutamic acid to change the overall net charge from -11.5e to -14.8e, and Bcl-xL_E31K,E36K mutant protein, in which glutamic acid residues at positions 31 and 36 were substituted with (+) charged lysine residues to change the overall net charge to -7.4e, were used to confirm whether changes in electrical signals were able to be measured even when amino acid residues of the protein were substituted due to mutations (see FIG. 24A).

Using the YaxAB nanopore system, each of Bcl-xL wild-type protein, Bcl-xL E31K,E36K mutant protein, and Bcl-xL R100E,R103E mutant protein was added in 10 mM Tris pH 7.5, 1 mM EDTA, and 1 M KCl buffer, and then, the nanopore electrical signals were measured. As a result, it was observed that the three types of unique nanopore electrical signals of the three types of proteins were distinct from each other (see FIG. 24B). This is because the net charge of the Bcl-xL protein affects the oscillation of the analyte protein inside the pore, which is caused by the balance between the electroosmotic force (EOF) of the YaxAB nanopore and the electrophoretic force (EPF) caused by the applied voltage.

In the case of the Bcl-xL_R100E,R103E mutant protein, the signal frequency of L1, which is the smallest current blockade level, increased. It was predicted that the Bcl-xL_R100E,R103E mutant had a strong repulsive force by EPF due to increased (-) charge compared to the wild-type Bcl-xL protein, and thus had a tendency to escape from the YaxAB nanopore without remaining in the YaxAB nanopore for a long time, and this tendency was revealed through electrical signal analysis. In addition, in the case of an increase in signal frequency of L3, which is the deepest current blockade level in Bcl-xL_E31K,E36K, since (+) charge increased compared to the wild-type Bcl-xL protein, the influence of EPF was less, and there was a tendency to locate deeper into the YaxAB nanopore, which was also shown through the result of the increase in the electrical signal frequency of L3.

As such, when the net charge of the protein changes, the oscillation movement of the protein within the nanopore is affected, and the generated electrical signal is also affected. Accordingly, since a significantly distinct change in the electrical signal was detectable even with only two point mutations in the protein, it was possible to accurately detect and distinguish not only Bcl-xL wild-type proteins but also trace amounts of mutant proteins using the YaxAB nanopore system of the present invention, which demonstrated the possibility of diagnosing various diseases such as cancer or genetic diseases requiring the detection of mutant proteins observed in cancer patients.

[Example 7]

**Whether electroosmosis or electrophoresis-based detection and analysis of analyte translocating through YaxAB nanopores is possible**

It was examined whether YaxAB nanopore of the present invention could be used to detect and analyze single-molecule proteins and nucleic acids translocating through the nanopore, driven by the electroosmosis or electrophoresis forces.

First, mTOR, which is small in size and weakly negatively charged at pH 7.5, was translocated through the nanopore by electroosmosis force due to the strong cation selectivity of the YaxAB nanopore, and the resulting ionic current blockade was measured. As a result, as illustrated in FIG. 25, it was observed that as the voltage applied to the cis and trans compartments increased, the dwell time of the single protein molecule within the nanopore also increased. However, above a certain threshold voltage, the dwell time significantly decreased. Accordingly, it was confirmed that if the analyte has a diameter comparable to that of the YaxAB nanopore, the analyte may be effectively detected and analyzed through direct translocation via electroosmotic force, rather than relying on a method involving oscillating inside the nanopore after electroosmotic introduction.

Next, EGF, which is a single molecule protein that is small in size and negatively charged at pH 7.5, was added to the trans compartment and translocated through the YaxAB nanopore of the present invention by the electrophoresis force, and the resulting ionic current blockade was measured. As a result, as illustrated in FIG. 26, it was observed that the dwell time it took for the single protein molecule to be translocated through the nanopore decreased as the voltage applied to the cis compartment and the trans compartment increased. In addition, $p53_{TAD1}$ peptide, which is an intrinsically disordered protein (IDP) with a small molecular weight of 1.8 kDa, was added to the trans compartment and translocated through the YaxAB nanopore of the present invention by electrophoresis force. As a result, a difference in ionic current blockade was observed before and after the addition of the $p53_{TAD1}$ peptide, and ionic current blockade was detected following its translocation, as illustrated in FIG. 27. In addition, even for $p53_{TAD}$ protein with a molecular weight of 8.3 kDa, an ionic current blockade was observed before and after its addition, as illustrated in FIG. 27. Through this, it was confirmed that intrinsically disordered protein and peptide analytes may be detected and analyzed by translocating the analyte through the nanopore via electrophoresis.

Meanwhile, as illustrated in FIG. 28, Lambda DNA, single-stranded nucleic acid (50 mer), and a double-stranded nucleic acid (23 bp) were independently added to the trans compartment of the YaxAB nanopore, and electrophoretic translocation of each nucleic acid was observed. As a result, an ionic current blockade was confirmed before and after the addition of each nucleic acid. Accordingly, it was confirmed that the YaxAB nanopore of the present invention enables detection and analysis of intrinsically disordered proteins and nucleic acids not only by the electroosmosis force but also by the electrophoresis force.

[Example 8]

**Analysis of purification efficiency of modified YaxAB nanopore**

In order to confirm whether the purification efficiency of the modified monomer is improved compared to the wild-type monomer in the YaxAB nanopore of the present invention, the purification efficiencies of the respective monomer proteins according to the length of the amino acid sequence were compared.

Specifically, the modified first monomer (YaxA_(45-410)) having the amino acid sequence of SEQ ID NO: 1, the modified second monomer (YaxB_(12-343)) having the amino acid sequence of SEQ ID NO: 2, the wild-type first monomer (YaxA_FL) having the amino acid sequence of SEQ ID NO: 3, and the wild-type second monomer (YaxB_FL) having the amino acid sequence of SEQ ID NO: 4 were purified using the same method as in Example 1-2. In the protein purification, 6 liters of *E. coli* culture medium was used for the purification of YaxA_FL, and 1 liter of *E. coli* culture medium was used for the purification of YaxB_FL. 2 liters of *E. coli* culture medium was used for the purification of YaxA_(45-410), and 1 liter of *E. coli* culture medium was used for the purification of YaxB_(12-343).

As shown in FIG. 29, the purified yields per liter of the respective monomer proteins were 12 mg for YaxA_FL, 66.2 mg for YaxB_FL, 56.1 mg for YaxA_(45-410), and 72.3 mg for YaxB_(12-343), and under the same purification conditions, the yield of the modified first monomer (YaxA_(45-410)) was 4.7 times greater than that of the first monomer (YaxA_FL) of the wild-type YaxAB. In addition, under the same purification conditions, the yield of the modified second monomer (YaxB_(12-343)) was 1.1 times greater than that of the wild-type second monomer (YaxB_FL). From the above results, it was found that, in the case of each modified monomer constituting the modified YaxAB_dN nanopore, the purified yield was higher than that of the wild-type monomer under the same purification conditions.

[Example 9]

**Analysis of oligomerization mechanism of modified YaxAB nanopore**

The monomer purified in Example 8 was subjected to oligomerization to form YaxAB nanopores (wild-type YaxAB and modified YaxAB_dN) in the same manner as that of Example 1-3, each nanopore was subjected to electrophoresis with a gradient blue polyacrylamide gel, and then, the bands of different oligomers of the wild-type nanopore and the modified nanopore were examined.

As a result, as illustrated in FIG. 30, when the first monomer and the second monomer at low concentrations were used, the bands of the wild-type YaxAB nanopore were not detected, whereas those of the modified YaxAB_dN nanopore were clearly distinguished into different oligomer types. In addition, when the first and second monomer were used at high concentrations, the modified YaxAB_dN and wild-type YaxAB nanopores exhibited distinct band patterns, indicating differences in oligomerization behavior. From the above results, it was confirmed that the first monomer (YaxA_(45-410)) having the amino acid sequence of SEQ ID NO: 1 and the second monomer (YaxB_(12-343)) having the amino acid sequence of SEQ ID NO: 2 not only had a higher yield, but also enabled more stable oligomerization under the same purification conditions compared to the wild-type YaxAB nanopore. Furthermore, it was confirmed that YaxAB-$C_8$ composed of eight subunits, which cannot be formed with a wild-type monomer, can be formed.

In addition, as illustrated in FIG. 31, as a result of showing the distribution of electrical conductivity of purified YaxAB nanopore by each band, in the wild-type YaxAB nanopore, $C_9$, $C_{10}$, and $C_{11}$ mainly appeared in each band, whereas in the modified YaxAB_dN nanopore, $C_8$, $C_9$, $C_{10}$, and $C_{11}$ mainly appeared in each band. As such, it was found that the oligomerization mechanism of the YaxAB nanopore may be adjusted by changing the amino acid sequence of each of the first monomer or the second monomer.

In addition, the oligomerization tendency with the second monomer YaxB_(12-343) was examined using YaxA_(X-411) whose sequence length was adjusted based on the sequence of the wild-type first monomer YaxA_FL.

Specifically, YaxA_(X-411) refers to the first monomer of YaxA FL(1-411), in which the N-terminal residues from position 1 to X were deleted. Variants of YaxA_(X-411), where X is 11, 21, 31, 41, 51, 61, 71, or 81 were produced, and oligomerized with YaxB_(12-343) using the same method as in Example 1-3.

As a result, as illustrated in FIGS. 32 and 33, it was confirmed that, when each of wild-type Yax-A_FL(1-411), YaxA_(11-411), YaxA_(21-411), YaxA_(31-411), YaxA_(41-411), YaxA_(51-411), and YaxA_(61-411) was reacted with YaxB_(12-343), Yax-

AB-$C_8$, YaxAB-$C_9$, and YaxAB-$C_{10}$ were formed. On the other hands, when YaxA_(71-411) and YaxA_(81-411) were reacted with YaxB_(12-343), YaxAB-$C_7$ oligomer was formed.

In addition, the oligomerization tendency with the second monomer YaxB_(12-343) was confirmed using YaxA_(X-410) with adjusted YaxA sequence length.

Specifically, YaxA_(X-410) refers to the first monomer, in which the N-terminal residues from position 1 to X were deleted and the C-terminal residue from position 1 was deleted. Variants of YaxA_(X-410), where X is 3, 5, 8, or 9 were produced and oligomerized with YaxB_(12-343) using the same method as in Example 1-3.

As a result, as illustrated in FIG. 33, it was confirmed, when each of the YaxA_(3-410), YaxA_(5-410), YaxA_(8-410), and YaxA_(9-410) was reacted with YaxB_(12-343), YaxAB oligomers were formed.

Furthermore, as illustrated in FIG. 34, it was confirmed that the YaxAB nanopore system, utilizing YaxAB oligomers formed by reacting each of the YaxA_(11-411), YaxA_(21-411), YaxA_(31-411), YaxA_(41-411), YaxA_(51-411), and YaxA_(3-410) with YaxB_(12-343), was able to detect Bcl-xL protein.

Thereafter, in order to confirm the minimum amino acid sequence that form a YaxAB nanopore, the C-terminal of the YaxA amino acid sequence was fixed, and a sequence from YaxA_(52-410) to YaxA_(60-410) was prepared, and the oligomerization tendency was analyzed. As a result, it was confirmed that YaxAB oligomers were formed when YaxA_(57-410) was reacted with YaxB_(12-343), but no oligomers were formed when YaxA_(58-410) was reacted with YaxB_(12-343). Furthermore, as a result of preparing a sequence from YaxA_(57-409) to YaxA_(57-391) and analyzing oligomerization tendency, it was confirmed that in the case of YaxA_(57-398), YaxAB oligomers were formed, and in the case of YaxA_(57-397), YaxAB oligomers were not formed.

In addition, as a result of analyzing the minimum amino acid sequence of YaxB_(12-343) that form a Yax-AB nanopore by the method described above, it was confirmed to be YaxB_(26-342). Furthermore, when YaxA_(57-398) was reacted with YaxB_(26-342), YaxAB oligomers were formed, the nanopore-containing membrane of the present invention was successfully implemented by the oligomer, and through this, it was confirmed that the Bcl-xL protein was successfully captured using the nanopore system.

In addition, contrary to the above method, as a result of fixing the N-terminal of the YaxA amino acid sequence, preparing a sequence from YaxA_(45-390) to YaxA_(45-369), and analyzing oligomerization tendency, it was confirmed that, in the case of YaxA_(45-383), YaxAB oligomers were formed, and in the case of YaxA_(45-382), YaxAB oligomers were not formed.

Furthermore, as a result of preparing a sequence from YaxA_(46-383) to YaxA (58-383) and analyzing oligomerization tendency, it was confirmed that in the case of YaxA_(52-383), YaxAB oligomers were formed, and in the case of YaxA_(53-383), YaxAB oligomers were not formed.

In addition, as a result of preparing a sequence from YaxB_(12-325) to YaxB_(12-314) by the above method and analyzing oligomerization tendency, it was confirmed that in the case of YaxB_(12-316), YaxAB oligomers were formed, and in the case of YaxB_(12-315), YaxAB oligomers were not formed. Furthermore, as a result of preparing a sequence from YaxB_(13-316) to YaxB_(18-316) and analyzing oligomerization tendency, it was confirmed that in the case of YaxB_(12-316), YaxAB oligomers were formed, and in the case of YaxB_(13-316), YaxAB oligomers were not formed. In addition, in a case in which YaxA_(52-383) was reacted with YaxB_(12-316), YaxAB oligomers were formed, the nanopore-containing membrane of the present invention was successfully implemented by the oligomer, and through this, it was confirmed that the Bcl-xL protein was successfully captured using the nanopore system.

Through this, as illustrated in FIGS. 32, 35, 36, and 37, it was confirmed that the minimum amino acid sequence of YaxA that form a YaxAB nanopore capable of constructing the nanopore-containing membrane of the present invention is YaxA_(57-398) having the amino acid sequence of SEQ ID NO: 7 and YaxA_(52-383) having the amino acid sequence of SEQ ID NO: 24, the minimum amino acid sequence of YaxB is YaxB_(26-342) having the amino acid sequence of SEQ ID NO: 18 and YaxB_(12-316) having the amino acid sequence of SEQ ID NO: 25, and all YaxABs obtained by a combination thereof form oligomers. Furthermore, it was confirmed that the nanopore-containing membrane of the present invention was successfully implemented in the case of the above oligomers formed by combination, and through this, the Bcl-xL protein was successfully captured using the nanopore system (see FIG. 36).

In addition, it was confirmed that in a case of a nanopore subunit containing a first monomer having at least one of the amino acid sequences consisting of SEQ ID NO: 5, SEQ ID NO: 9, SEQ ID NO: 10, SEQ ID NO: 11, SEQ ID NO: 12, SEQ ID NO: 32, SEQ ID NO: 33, or SEQ ID NO: 34 and a second monomer having an amino acid sequence consisting of SEQ ID NO: 2, YaxAB oligomers were stably formed (see FIG. 32).

Based on the above results, it was found that the minimum amino acid sequences of YaxA that form a YaxAB nanopore capable of constructing the nanopore-containing membrane of the present invention are YaxA_(57-398) having the amino acid sequence of SEQ ID NO: 7, YaxA_(52-383) having the amino acid sequence of SEQ ID NO: 24, and YaxA_(81-411) having the amino acid sequence of SEQ ID NO: 34. It was found that the minimum amino acid sequences of YaxB are YaxB_(26-342) having the amino acid sequence of SEQ ID NO: 18 and YaxB_(12-316) having the amino acid sequence of SEQ ID NO: 25, and based on the above examples, the minimum amino acid sequence that form the YaxAB nanopore of the present invention was identified (see FIGS. 32 to 36).

[Example 10]

**Confirmation of analytical precision of analyte-ligand interaction of YaxAB nanopore**

**[10-1] Whether precise interaction analysis is possible even when there is large difference in molecular weight between analyte and ligand**

The range of the protein-small molecule compound complex that may be analyzed using the YaxAB nanopore of the present invention was examined.

Specifically, FKBP12 protein (13.0 kDa) was added alone at 100 nM to the cis compartment of the sensor produced in Example 1-4, or ii) 100 nM FKBP12 protein and 1 uM FK506 were added at a molar ratio of 1:10, and (+) and (-) voltages were applied to the cis compartment and the trans compartment, respectively.

As a result, as illustrated in FIG. 38, it was observed that a change occurred in the current blockade pattern in the case in which FK506 was added together, unlike in the case in which FKBP12 protein was added alone, and thus, it was confirmed that the distinction between the protein and the protein-small molecule compound complex (FKBP12+FK506) was possible.

In addition, i) holo-transferrin protein (77.1 kDa) was added alone to the cis compartment in the same manner as above, or ii) oxaliplatin was added to holo-transferrin, and a voltage was applied. As a result, as illustrated in FIG. 39, a distinct change in the current blockade pattern was observed by the addition of oxaliplatin, and thus, it was confirmed that it was possible to distinguish between the case in which the protein was added alone and the protein-small molecule compound complex (holo-transferrin+oxaliplatin).

In the protein-small molecule compound complex as described above, the molecular weight ratio of FKBP12 and FK506 was 16:1, and the molecular weight ratio of holo-transferrin and oxaliplatin was 194:1. Thus, it was confirmed that the nanopore system of the present invention may sensitively distinguish the signals of the protein and the protein-small molecule compound complex, despite 16-fold to 194-fold difference in molecular weight ratio between the protein and the small molecule compound.

**[10-2] Whether precise interaction analysis is possible even when there is large difference in concentration between analyte and ligand or under high concentration drug condition**

In order to confirm whether the YaxAB nanopore system of the present invention may be usefully utilized for drug screening under conditions in which drugs at various concentrations are present, including high concentrations, protein-compound complex signals were compared under conditions in which target-specific binding compounds were present at various concentrations, and under conditions including non-binding compounds as negative controls.

Specifically, the interaction between Bcl-xL protein and the small molecule compound ABT-737 was analyzed in the same manner as that of Example 4-1, and it was confirmed that drug binding activity was able to be screened even under conditions in which ABT-737 was added at concentrations (binding ratios) of 200 nM (1:2) and 10 μM (1:100).

As a result, as illustrated in FIG. 40, the same results were obtained regardless of the concentration of the ABT-737 compound, and thus, it was confirmed that the YaxAB nanopore of the present invention may be used to screen a drug by analyzing drug binding activity for a protein even under conditions in which the drug is present at a high concentration (10 μM or more) or at a low concentration at a single molecule level.

In addition, in order to confirm whether non-specific signals were generated by non-target compounds, as illustrated in FIG. 41, four types of small molecule compounds (ABT-737, A-1331852, LCL-161, and GDC-0152) were added at a concentration of 200 nM or 1 μM in the YaxAB nanopore system of the present invention, and ion blocking current signals were measured for 30 minutes. In addition, four types of non-target compounds (LCL-161, GDC-0152, Birinapant, and Phentolamine) were added to the cis compartment at a concentration (concentration ratio) of 10 μM (1:100) in a state where Bcl-xL protein was trapped in the YaxAB-$C_8$ nanopore, and the ion blocking current signals were observed for 30 minutes.

As a result, as illustrated in FIG. 41A, it was confirmed that in a case in which only the compound was added, the ionic current flowing through the YaxAB-$C_8$ nanopore was not affected. In addition, as illustrated in FIGS. 41B and 41C, the capture signal obtained when Bcl-xL was added alone and the ionic current signal obtained when Bcl-xL was added together with a non-target compound were measured to be almost identical, and thus, it was confirmed that the YaxAB nanopore system of the present invention may specifically detect the signal of the captured analyte (Bcl-xL) even under conditions in which four types of non-target compounds at high concentration are added.

[Example 11]

**Confirmation whether nanopore system** of **present invention operates even in solution containing DMSO**

In addition, in order to confirm whether the YaxAB nanopore system of the present invention operates stably even under conditions in which DMSO is added, which is necessary for detecting a poorly soluble drugs, each of Bcl-xL protein and three types of ligands (Bak-BH3 peptide, ABT-737, and A-1331852) was added in the same manner as that of Example 4-1 under DMSO conditions at various concentrations (0, 0.01, 0.1, 0.5, 1, 2, 5, and 10%), and complex signals were observed.

As a result, as illustrated in FIG. 42, it was confirmed that the YaxAB nanopore system of the present invention enables stable and precise analysis by clearly distinguishing between the ionic current signals derived from free Bcl-xL and those derived from complexes formed independently between Bcl-xL and each of three different Bcl-xL-ligands, under DMSO conditions at a concentration of 5% or less.

**Claims**

1. A YaxAB nanopore comprising a first opening, a middle area, and a second opening, the YaxAB nanopore having a funnel shape,

   wherein an outer diameter of the first opening at a lumen is 5 nm or more,
   the second opening includes a constriction,
   a diameter of the constriction is 0.5 nm or more,
   an outer diameter of the second opening at the lumen is 1 nm or more, and
   a depth of the lumen is 5 nm or more.

2. A YaxAB nanopore comprising at least one subunit containing a first monomer comprising an amino acid sequence that is at least 70% identical to the amino acid sequence of any one of SEQ ID NO: 7, SEQ ID NO: 24, or SEQ ID NO: 34; and a second monomer comprising an amino acid sequence that is at least 70% identical to the amino acid sequence of SEQ ID NO: 18 or SEQ ID NO: 25.

3. The YaxAB nanopore of claim 1 or 2, wherein the YaxAB nanopore contains 4 to 20 subunits.

4. The YaxAB nanopore of claim 1 or 2, wherein the YaxAB nanopore exhibits a cation selectivity that is at least 1.1-fold greater than its anion selectivity, or an anion selectivity that is at least 1.1-fold greater than its cation selectivity.

5. The YaxAB nanopore of claim 1 or 2, wherein the subunit contains:

a first monomer having any one amino acid sequence selected from the group consisting of SEQ ID NO: 1, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 19, SEQ ID NO: 20, SEQ ID NO: 21, SEQ ID NO: 22, SEQ ID NO: 23, SEQ ID NO: 24, SEQ ID NO: 30, SEQ ID NO: 31, SEQ ID NO: 32, SEQ ID NO: 33, and SEQ ID NO: 34; and
a second monomer having any one amino acid sequence selected from the group consisting of SEQ ID NO: 2, SEQ ID NO: 18, SEQ ID NO: 25, SEQ ID NO: 26, SEQ ID NO: 27, SEQ ID NO: 28, and SEQ ID NO: 29.

6. A nanopore-containing membrane comprising:

a membrane layer; and
a YaxAB nanopore inserted into the membrane layer,
wherein the membrane layer includes at least one selected from the group consisting of 1,2-diphytanoyl-sn-glycero-3-phosphocholine (DPhPC), 1,2-dipalmitoyl-sn-glycero-3-phosphocholine (DPPC), 1,2-dipalmitoyl-sn-glycero-3-phosphorylethanolamine (DPPE), 1,2-dipalmitoyl-sn-glycero-3-phosphorylglycerol (DPPG), 1,2-dipalmitoyl-sn-glycero-3-phospho-L-serine (DPPS), 1,2-dihexanoyl-sn-glycero-3-phosphocholine (DHPC), 1,2-dilauroyl-sn-glycero-3-phosphocholine (DLPC), 1,2-dimyristoyl-sn-glycero-3-phosphocholine (DMPC), 1,2-dimyristoyl-sn-glycero-3-phosphorylethanolamine (DMPE), 1,2-dimyristoyl-sn-glycero-3-phosphorylglycerol (DMPG), 1,2-dimyristoyl-sn-glycero-3-phospho-L-serine (DMPS), 1,2-dioleoyl-sn-glycero-3-phospho-L-serine (DOPS), 1,2-dioleoyl-sn-glycero-3-phosphoethanolamine (DOPE), 1,2-dioleoyl-sn-glycero-3-phosphoglycerol (DOPG), 1,2-distearoyl-sn-glycero-3-phosphocholine (DSPC), 1-palmitoyl-2-oleoyl-SN-glycero-3-phosphocholine (POPC), and mycolic acid, and at least one selected from the group consisting of campesterol, sitosterol, stigmasterol, cholesterol, ergosterol, cardiolipin, sphingomyelin, 1-palmitoyl-2-cholesterylhemisuccinoyl-sn-glycero-3-phosphocholine (PChemsPC), 1-oleoyl-2-cholesterylhemisuccinoyl-sn-glycero-3-phosphocholine (OChemsPC), 1-palmitoyl-2-cholesterylcarbonoyl-sn-glycero-3-phosphocholine (PChcPC), 1,2-dicholesterylhemisuccinoyl-sn-glycero-3-phosphocholine (DChemsPC), 10,12-pentacosadiynoic acid (PCDA), 10,12-tricosadiynoic acid (TCDA), 5,7-hexadecadiynoic acid (HDDA), and 9,12-octadecadiynoic acid (ODDA).

7. The nanopore-containing membrane of claim 6, wherein the YaxAB nanopore contains at least one subunit containing:

a first monomer comprising an amino acid sequence that is at least 70% identical to the amino acid sequence of any one of SEQ ID NO: 7, SEQ ID NO: 24, SEQ ID NO: 34, or SEQ ID NO: 3; and
a second monomer comprising an amino acid sequence that is at least 70% identical to the amino acid sequence of any one of SEQ ID NO: 18, SEQ ID NO: 25, or SEQ ID NO: 4.

8. A nanopore system comprising:

a chamber; and
a YaxAB nanopore-containing membrane, wherein a space in the chamber is divided into two compartments by the nanopore-containing membrane.

9. The nanopore system of claim 8, wherein the nanopore system operates to allow an analyte to be translocated through, trapped in, or induce gating of a pore,

the translocation of an analyte, trapping of an analyte, or gating of the pore by an analyte is caused by electrophoresis or electroosmosis, characteristics of the analyte are analyzed by detecting oscillation or rotational motion of the analyte during trapping of the analyte inside the pore; clogging of the pore by the analyte; translocation of the analyte through the pore; or gating of the pore by the analyte, and
the characteristics of the analyte are at least one selected from the group consisting of a surface charge, an isoelectric point, stability, an isomer, a length, a size, a molecular weight, a structure, dynamics, strength and orientation of a dipole moment, orientation, flexibility, conformational heterogeneity, a shape, and an amount of the analyte, and changes thereof.

10. The nanopore system of claim 9, wherein the molecular weight of the analyte is 1 to 1,200 kDa.

11. The nanopore system of claim 8, wherein the nanopore system is used for at least one selected from the group consisting of analysis of an interaction between an analyte and a ligand, drug screening, nucleic acid sequencing, protein sequencing, peptide sequencing, protein identification, disease diagnosis, protein post-translational modification (PTM) analysis, proteomic analysis, and structural analysis of a biomolecule.

12. A single molecule analysis method for a single analyte or a plurality of analytes, the single molecule analysis method comprising:

    placing at least one analyte in one compartment of the nanopore system of claim 8; and measuring changes in electrical signal in the two compartments before and after the placing of the analyte.

13. The single molecule analysis method of claim 12, wherein the analyte is a peptide, a protein, a lipid, a carbohydrate, an inorganic substance, a small molecule compound, a nucleic acid, or an inorganic particle composed of nanomaterial,

    the electrical signal is a pattern of an ionic current, a base current in an open state ($I_o$), a magnitude of a current drop ($\Delta I$), a current blockade ($\Delta I/I_o$), an event duration, a frequency per unit time of a nanopore signal pattern, or a current noise ($I_N$), and the analysis is for detection or identification of the single analyte or the plurality of analytes.

14. A method for analyzing an interaction between an analyte and a ligand or screening a ligand for an analyte, the method comprising:

    performing a treatment with at least one analyte and at least one ligand candidate substance for the analyte in one compartment or two compartments of the nanopore system of claim 8; and measuring changes in electrical signal in the two compartments before and after the treatment with the candidate substance.

15. The method of claim 14, wherein the analysis is a quantitative analysis that calculates a binding affinity (dissociation constant, $K_D$) between the analyte and the ligand.

16. The method of claim 14, wherein the interaction is competitive binding between an analyte and a plurality of ligands, competitive binding between a plurality of analytes and a ligand of an analyte, ligand binding site mapping of an analyte, or a structure-activity relationship (SAR).

17. The method of claim 14, wherein the analyte is a peptide, a protein, a lipid, a carbohydrate, an inorganic substance, a small molecule compound, a nucleic acid, or an inorganic particle composed of nanomaterial, and the ligand is a peptide, a protein, a lipid, a carbohydrate, a small molecule compound, or a nucleic acid.

18. A method for analyzing or screening an interaction inhibitor or promoter between biomolecules, the method comprising:

    reacting a plurality of biomolecules capable of interacting in one compartment of the nanopore system of claim 8; performing a treatment with a candidate substance of the interaction inhibitor or promoter in the one compartment or two compartments; and measuring changes in electrical signal in the two compartments before and after the treatment with the candidate substance.

19. A method for identifying and quantitatively analyzing an analyte in a sample, the method comprising:

    placing a sample in one compartment of the nanopore system of claim 8; and comparing changes in characteristic electrical signal caused by a plurality of analytes in the sample with a database of electrical signals for each substance.

20. A method for providing information for diagnosing a biomarker-associated disease, the method comprising:

    placing a sample in one compartment of the nanopore system of claim 8; and measuring changes in characteristic electrical signal caused by a biomarker for a specific disease among changes in electrical signals induced by the sample.

FIG. 1]

[FIG. 2]

[FIG. 3]

[FIG. 4]

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

**A**

(+)

Electroosmotic flow
(EOF)

(-)

Electrophoretic flow
(EPF)

glucose/galactose

binding protein, GBP

(36.7 kDa, pI=6.15)

**B**

+ GBP

$I_o$

1 nS

3 s

+GBP
+ galactose

$I_o$

1 nS

3 s

**C**

Lysozyme

$I_o$

14.0%

2 s

**D**

holo-myoglobin

$I_o$

47.3%

2 s

**E**

Lysozyme (N=169)

65.6%

SD (pA)   Dwell time (ms)   Rel. freq. (%)

[FIG. 15]

a — $K_D = 65.78 \pm 10.32$ nM; Bound fraction (%) vs Conc. of Bak-BH3 (nM)

b — $K_D = 37.97 \pm 7.91$ nM; Bound fraction (%) vs Conc. of ABT-737 (nM)

c — $K_D = 19.16 \pm 4.47$ nM; Bound fraction (%) vs Conc. of A-1331852 (nM)

[FIG. 16]

[FIG. 17]

[FIG. 18]

[FIG. 19]

[FIG. 20]

[FIG. 21]

[FIG. 22]

[FIG. 23]

[FIG. 24]

**A**

Free Bcl-xL
PI 5.1, Net charge: -11.5e

Bcl-xL_E31K,E36K
PI 6.7, Net charge: -7.4e

Bcl-xL_R100E,R103E
PI 4.7, Net charge: -14.8e

**B**

Free Bcl-xL

Bcl-xL_E31K,E36K

Bcl-xL_R100E,R103E

**C**

Free Bcl-xL

Bcl-xL_E31K,E36K

Bcl-xL_R100E,R103E

60 mV

80 mV

100 mV

**A**

mTOR (12 kDa, pI 6.5)
Electroosmotic flow

(+)

1 M KCl pH 8.0

(−)

**B**

0 mV

60 mV

80 mV

100 mV

120 mV

140 mV

200 pA
0.5 s

**C**

140 mV
120 mV
100 mV
80 mV
60 mV

Conductance blockade (nS)

Log Dwell time (ms)

**D**

Conductance blockade (nS)

Applied potential (mV)

Translocation threshold

Dwell time (ms)

Applied potential (mV)

[FIG. 25]

[FIG. 26]

**A**

(+) 1 M KCl pH 8.0

(−)

Electrophoresis

**EGF human protein
(6.2 kDa, pI 4.5)**

**B**

40 mV

60 mV

80 mV

100 mV

120 mV

140 mV

500 pA

10 s

**C**

Conductance (nS)

Log Dwell Time (ms)

140 mV
120 mV
100 mV
80 mV
60 mV
40 mV

**D**

Conductance (nS)

Voltage (mV)

Mean of Dwell time (ms)

Voltage (mV)

**A**

(+) Electroosmotic flow (EOF)

(-) Electrophoretic flow (EPF)

p53$_{TAD1}$
(1.8 kDa, pI: 4.1)

100 mV
with YaxAB-$C_8$ (4.7 nS, 5.2 nS)

- p53$_{TAD1}$   $I_o$

+ p53$_{TAD1}$   $I_o$

$I_o$

$I_o$

2 nS
5 s

100 mV
with YaxAB-$C_{10}$ (11 nS)

- p53$_{TAD1}$   $I_o$

2 nS
5 s

+ p53$_{TAD1}$   $I_o$

$I_o$

2 nS
5 s

**B**

(+) Electroosmotic flow (EOF)

(-) Electrophoretic flow (EPF)

p53$_{TAD}$
(8.3 kDa, pI: 3.5)

100 mV
with YaxAB-$C_9$ (7.6 nS)

- p53$_{TAD}$   $I_o$

+ p53$_{TAD}$ at 60 mV   $I_o$

+ p53$_{TAD}$ at 100 mV   $I_o$

2 nS
5 s

[FIG. 27]

EP 4 610 652 A1

[FIG. 28]

A

(+)  Electroosmotic flow
     (EOF)

Electrophoretic flow
(EPF)

(-)  Nucleic acids

B

100 mV

with YaxAB-C$_9$ (8.0 nS)

Pore ———————— $I_o$

+ λ-DNA ———————— $I_o$

2 nS
     3 s

C

140 mV

with YaxAB-C$_9$ (7.4 nS)

Pore ———————— $I_o$

+ ssDNA ———————— $I_o$
(50 mer)

2 nS
     5 s

D

120 mV

with YaxAB-C$_9$ (8.3 nS)

Pore ———————— $I_o$

dsDNA ———————— $I_o$
(23 bp)

2 nS
     5 s

[FIG. 29]

[FIG. 30]

* YaxAB_FL is an oligomer formed by YaxA_FL (1-411) and YaxB_FL (1-344).

** YaxAB_dN is an oligomer formed by YaxA_(45-410) and YaxB_(12-343).

[FIG. 31]

[FIG. 32]

[FIG. 33]

**A**

**B**

[FIG. 34]

**A**

**B**

[FIG. 35]

YaxA_(45-410)  YaxA_(57-398)  YaxA_(52-383)
YaxB_(12-343)  YaxB_(26-342)  YaxB_(12-316)

$C_{10}$
$C_9$
$C_8$

YaxA_(45-410)  YaxA_(57-398)  YaxA_(52-383)
YaxB_(12-343)  YaxB_(12-316)  YaxB_(26-342)

$C_{10}$
$C_9$
$C_8$

[FIG. 36]

[FIG. 37]

[FIG. 38]

[FIG. 39]

**a** holo-transferrin

**b**

L1
25.09±0.01

L2
35.17±0.01

**c** holo-transferrin/oxaliplatin complex

**d**

L2
39.52±0.02

L1
25.55±0.04

[FIG. 40]

[FIG. 41]

**a**

YaxAB-$C_8$ free

0 min                    10 min

YaxAB-$C_8$+
LCL-161, GDC-0152, ABT-737,
and A-1331852 (200 nM)

0 min                    30 min

YaxAB-$C_8$+
LCL-161, GDC-0152, ABT-737,
and A-1331852 (1 μM)

0 min                    30 min

200 pA
2 s

200 pA
2 s

**b** Free Bcl-xL

100 pA
0.3 s

**c**

$\Delta I / I_0$ (%)

$I_N$ (pA)

0.89
0.8
0.71
0.63
0.54
0.45
0.36
0.28
0.19
0.1

**d** Bcl-xL + LCL-161, GDC-0152, Birinapant, and Phentoamine (1:100)

100 pA
0.3 s

**e**

30 min

$\Delta I / I_0$ (%)

$I_N$ (pA)

0.95
0.85
0.76
0.66
0.57
0.48
0.38
0.29
0.19
0.1

[FIG. 42]

# EP 4 610 652 A1

| INTERNATIONAL SEARCH REPORT | International application No. |
|---|---|
| | **PCT/KR2023/016681** |

**A. CLASSIFICATION OF SUBJECT MATTER**

**G01N 33/487**(2006.01)i; **C07K 14/00**(2006.01)i

According to International Patent Classification (IPC) or to both national classification and IPC

**B. FIELDS SEARCHED**

Minimum documentation searched (classification system followed by classification symbols)

G01N 33/487(2006.01); C12Q 1/6869(2018.01); G01N 24/10(2006.01); G01N 33/483(2006.01); G01N 33/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 나노포어(nanopore), 깔때기(funnel), 직경(diameter), 멤브레인(membrane), 단백질(protein), 분석(analysis)

**C. DOCUMENTS CONSIDERED TO BE RELEVANT**

| Category* | Citation of document, with indication, where appropriate, of the relevant passages | Relevant to claim No. |
|---|---|---|
| X | BRAUNING, B. et al. Structure and mechanism of the two-component α-helical po re-forming toxin YaxAB. Nature Communications. 2018, vol. 9, document no. 1806, pp. 1-14. See pages 3-5 and 10; and figures 2-4 and 7. | 1,3-4 |
| Y | | 6,8-20 |
| A | | 2,5,7 |
| Y | KR 10-2022-0131766 A (KOREA RESEARCH INSTITUTE OF BIOSCIENCE AND BIOTECHNOLOGY) 29 September 2022 (2022-09-29) See claims 1 and 17; paragraphs [0002], [0006], [0012], [0016]-[0019], [0026], [0037], [0041]-[0042], [0059] and [0068]-[0073]; and figure 1. | 6,8-20 |
| A | KR 10-2127111 B1 (UNIVERSITY-INDUSTRY COOPERATION GROUP OF KYUNG HEE UNIVERSITY) 26 June 2020 (2020-06-26) See entire document. | 1-20 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 February 2024** | **02 February 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

<table>
<tr><td colspan="2">International application No.</td></tr>
<tr><td colspan="2">**PCT/KR2023/016681**</td></tr>
</table>

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2017-0089471 A (KOREA RESEARCH INSTITUTE OF BIOSCIENCE AND BIOTECHNOLOGY et al.) 04 August 2017 (2017-08-04)<br>See entire document. | 1-20 |
| A | KR 10-2446129 B1 (GENVIDA TECHNOLOGY COMPANY LIMITED) 22 September 2022 (2022-09-22)<br>See entire document. | 1-20 |

Form PCT/ISA/210 (second sheet) (July 2022)

**INTERNATIONAL SEARCH REPORT**

International application No.

**PCT/KR2023/016681**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/KR2023/016681**

| Patent document cited in search report | | | Publication date (day/month/year) | Patent family member(s) | | | Publication date (day/month/year) |
|---|---|---|---|---|---|---|---|
| KR | 10-2022-0131766 | A | 29 September 2022 | None | | | |
| KR | 10-2127111 | B1 | 26 June 2020 | None | | | |
| KR | 10-2017-0089471 | A | 04 August 2017 | KR | 10-1838687 | B1 | 15 March 2018 |
| | | | | US | 11474093 | B2 | 18 October 2022 |
| | | | | US | 2019-0162713 | A1 | 30 May 2019 |
| | | | | WO | 2017-131270 | A1 | 03 August 2017 |
| KR | 10-2446129 | B1 | 22 September 2022 | CN | 110177618 | A | 27 August 2019 |
| | | | | CN | 110177618 | B | 14 December 2021 |
| | | | | EP | 3519097 | A1 | 07 August 2019 |
| | | | | JP | 2020-502538 | A | 23 January 2020 |
| | | | | JP | 2023-012466 | A | 25 January 2023 |
| | | | | JP | 7157747 | B2 | 20 October 2022 |
| | | | | KR | 10-2019-0062514 | A | 05 June 2019 |
| | | | | KR | 10-2022-0131352 | A | 27 September 2022 |
| | | | | US | 11565258 | B2 | 31 January 2023 |
| | | | | US | 2020-0038868 | A1 | 06 February 2020 |
| | | | | US | 2023-0094129 | A1 | 30 March 2023 |
| | | | | WO | 2018-067457 | A1 | 12 April 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220138724

**Non-patent literature cited in the description**

- **ZERNIA et al.** Current Blockades of Proteins inside Nanopores for Real-Time Metabolome Analysis. *ACS Nano*, 2020, vol. 14 (2), 2296-2307
- **HUANG et al.** Detection of single amino acid differences in haemoglobin from blood samples using a nanopore. *Analytical Chemistry*, 2021
- **G. MAGLIA et al.** Direct electrical quantification of glucose and asparagine from bodily fluids using nanopores. *Nat. Commun.*, 2018
- **CHI et al.** Probing the Neuraminidase Activity of Influenza Virus Using a Cytolysin A Protein Nanopore. *Anal. Chem.*, 2020
- **MORALES-MARTINEZ et al.** Roles and Regulation of BCL-xL in Hematological Malignancies. *Int. J. Mol. Sci.*, 2022